# EUROPEAN PATENT APPLICATION

(11) **EP 4 369 902 A1**
(43) Date of publication of application: **15.05.2024**
(21) Application number: 22838028.3
(22) Date of filing: 07.07.2022
(51) Int. Cl.: H10K 99/00

(54) **COMPOUND AND ORGANIC LIGHT-EMITTING DEVICE COMPRISING SAME**

(30) Priority: 07.07.2021 KR 20210089233
(71) Applicant: LG Chem, Ltd., Yeongdeungpo-gu Seoul 07336 (KR)
(72) Inventor: HA, Jae Seung, Daejeon 34122 (KR); KIM, Seonwoo, Daejeon 34122 (KR); KIM, Joo Ho, Daejeon 34122 (KR); KIM, Hoon Jun, Daejeon 34122 (KR); LEE, Woochul, Daejeon 34122 (KR); LEE, Hojung, Daejeon 34122 (KR)
(74) Representative: Hoffmann Eitle
(86) International application number: PCT/KR2022/009886
(87) International publication number: WO 2023/282676

(57) **Abstract**

The present specification relates to a compound and an organic light emitting device including the same.

## Description

### [Technical Field]

The present specification claims priority to and the benefit of Korean Patent Application No. 10-2021-0089233 filed in the Korean Intellectual Property Office on July 7, 2021, the entire contents of which are incorporated herein by reference.

The present specification relates to a compound and an organic light emitting device including the same.

### [Background Art]

An organic light emission phenomenon generally refers to a phenomenon converting electrical energy to light energy using an organic material. An organic light emitting device using the organic light emitting phenomenon usually has a structure including a first electrode, a second electrode, and an organic material layer interposed therebetween. Here, the organic material layer has in many cases a multi-layered structure composed of different materials in order to improve the efficiency and stability of the organic light emitting device, and for example, may be composed of a hole injection layer, a hole transport layer, a light emitting layer, an electron transport layer, an electron injection layer, and the like. In the structure of the organic light emitting device, if a voltage is applied between the two electrodes, holes are injected from the first electrode into the organic material layer and electrons are injected from the second electrode into the organic material layer, and when the injected holes and electrons meet each other, an exciton is formed, and light is emitted when the exciton falls down again to a ground state.

There is a continuous need for developing a new material for the aforementioned organic light emitting device.

### [Detailed Description of the Invention]

### [Technical Problem]

The present specification has been made in an effort to provide a compound and an organic light emitting device including the same.

### [Technical Solution]

An exemplary embodiment of the present specification provides a compound of the following Chemical Formula 1. 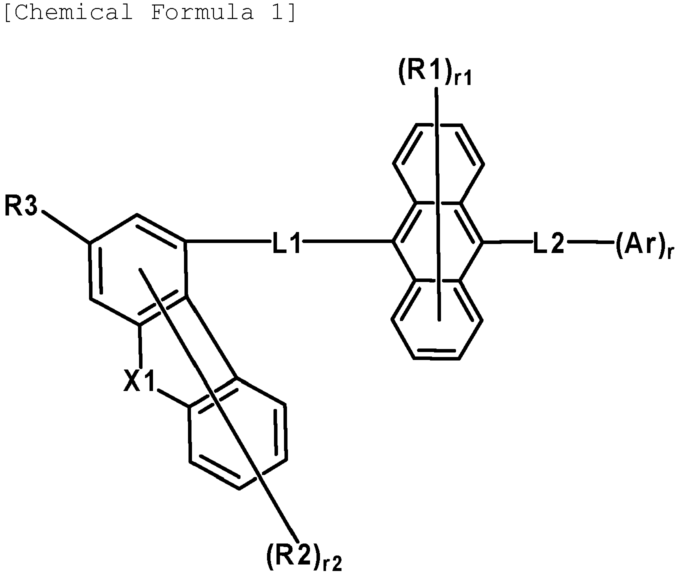 In Chemical Formula 1,
X1 is O or S,
R1 is hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group including at least one selected from N, O and S,
R2 is hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S, or adjacent groups may be bonded to each other to form a substituted or unsubstituted ring,
R3 is a polycyclic heterocyclic group comprising at least one selected from N, O and S; or a substituted or unsubstituted monocyclic heterocyclic group including at least one selected from O and S,
L1 and L2 are the same as or different from each other, and are each independently a direct bond, or a substituted or unsubstituted arylene group,
Ar is a substituted or unsubstituted aryl group,
r1 is an integer from 1 to 8,
r2 is an integer from 1 to 6,
when r1 is 2 or higher, R1's are the same as or different from each other,
when r2 is 2 or higher, R2's are the same as or different from each other, and
r is an integer from 1 to 4, and when r is 2 or higher, Ar's are the same as or different from each other.

Another exemplary embodiment of the present specification provides an organic light emitting device including: a first electrode; a second electrode; and an organic material layer having one or more layers provided between the first electrode and the second electrode, in which one or more layers of the organic material layer include the above-described compound.

### [Advantageous Effects]

An organic light emitting device using the compound according to an exemplary embodiment of the present specification can have a low driving voltage, high light emitting efficiency, or a long service life.

### [Brief Description of Drawings]

FIG. 1 illustrates a structure of an organic light emitting device according to an exemplary embodiment.
FIG. 2 illustrates a structure of an organic light emitting device according to another exemplary embodiment.

### <Explanation of Reference Numerals and Symbols>

1: Substrate
2: First electrode
2-1: Anode
3: Organic material layer
4: Second electrode
4-1: Cathode
5: First organic material layer
6: Light Emitting Layer
7: Second organic material layer
8: Hole injection layer
9: Hole transport layer
10: Hole adjusting layer
11: Electron adjusting layer
12: Electron transport layer
13: Electron injection layer
14: Capping layer

### [Best Mode]

Hereinafter, the present specification will be described in detail.

The present specification provides the compound represented by Chemical Formula 1.

The compound of Chemical Formula 1 of the present specification may elicit the improvement in the characteristics of injecting, transporting and adjusting holes and electrons by disposing substituents at a specific position as an anthracene blue fluorescent host in which an aryl group including fluorene is introduced together with dibenzofuran or dibenzothiophene which can be fused. Specifically, the compound of Chemical Formula 1 of the present specification may produce excellent device results by introducing a specific directional bond of dibenzofuran or dibenzothiophene and a heteroaryl group as a substituent into the unit to balance the movement of holes and electrons.

When one part "includes" one constituent element in the present specification, unless otherwise specifically described, this does not mean that another constituent element is excluded, but means that another constituent element may be further included.

When one member is disposed "on" another member in the present specification, this includes not only a case where the one member is brought into contact with another member, but also a case where still another member is present between the two members.

In the present specification, * or a dotted line means a site bonded or fused to another substituent or a bonding portion.

In the present specification, Cn means that the number of carbon atoms is n, and Cn-Cm means that the number of carbon atoms is n to m. In the present specification, the `layer' has a meaning compatible with a `film' usually used in the art, and means a coating covering a target region. The size of the `layer' is not limited, and the sizes of the respective 'layers' may be the same as or different from one another. According to an exemplary embodiment, the size of the `layer' may be the same as that of the entire device, may correspond to the size of a specific functional region, and may also be as small as a single sub-pixel.

In the present specification, when a specific A material is included in a B layer, this means both i) the fact that one or more A materials are included in one B layer and ii) the fact that the B layer is composed of one or more layers, and the A material is included in one or more layers of the multi-layered B layer.

In the present specification, when a specific A material is included in a C layer or a D layer, this means all of i) the fact that the A material is included in one or more layers of the C layer having one or more layers, ii) the fact that the A material is included in one or more layers of the D layer having one or more layers, and iii) the fact that the A material is included in each of the C layer having one or more layers and the D layer having one or more layers.

Examples of the substituents in the present specification will be described below, but are not limited thereto.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, the term "substituted or unsubstituted" means being substituted with one or two or more substituents selected from the group consisting of deuterium; a halogen group; a cyano group (-CN); a silyl group; a boron group; an alkyl group; a cycloalkyl group; an aryl group; and a heterocyclic group, being substituted with a substituent to which two or more substituents among the exemplified substituents are linked, or having no substituent. For example, "the substituent to which two or more substituents are linked" may be a biphenyl group. That is, the biphenyl group may also be an aryl group, and may be interpreted as a substituent to which two phenyl groups are linked.

In an exemplary embodiment of the present specification, the "substituted or unsubstituted" means being substituted with one or more substituents selected from the group consisting of deuterium; a halogen group; a cyano group (-CN); a silyl group; a C1-C20 alkyl group; a C3-C60 cycloalkyl group; a C6-C60 aryl group; and a C2-C60 heterocyclic group, being substituted with a substituent to which two or more groups selected from the above group are linked, or having no substituent.

In an exemplary embodiment of the present specification, the "substituted or unsubstituted" means being substituted with one or more substituents selected from the group consisting of deuterium; a halogen group; a cyano group (-CN); a silyl group; a C1-C20 alkyl group; a C3-C60 cycloalkyl group; a C6-C60 aryl group; and a C2-C60 heterocyclic group, being substituted with a substituent to which two or more groups selected from the above group are linked, or having no substituent.

In an exemplary embodiment of the present specification, the "substituted or unsubstituted" means being substituted with one or more substituents selected from the group consisting of deuterium; a halogen group; a cyano group (-CN); a silyl group; a C1-C10 alkyl group; a C3-C30 cycloalkyl group; a C6-C30 aryl group; and a C2-C30 heterocyclic group, being substituted with a substituent to which two or more groups selected from the above group are linked, or having no substituent.

In an exemplary embodiment of the present specification, the "substituted or unsubstituted" means being substituted with one or more substituents selected from the group consisting of deuterium; a halogen group; a cyano group (-CN); a silyl group; a C1-C6 alkyl group; a C3-C20 cycloalkyl group; a C6-C20 aryl group; and a C2-C20 heterocyclic group, being substituted with a substituent to which two or more groups selected from the above group are linked, or having no substituent.

In the present specification, the fact that two or more substituents are linked indicates that hydrogen of any one substituent is replaced with another substituent. For example, an isopropyl group and a phenyl group may be linked to each other to become a substituent of

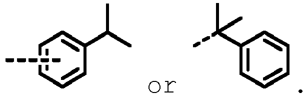

In the present specification, the fact that three substituents are linked to one another includes not only a case where (Substituent 1)-(Substituent 2)-(Substituent 3) are consecutively linked to one another, but also a case where (Substituent 2) and (Substituent 3) are linked to (Substituent 1). For example, two phenyl groups and an isopropyl group may be linked to each other to become a substituent of 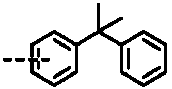 or 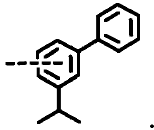 The same also applies to the case where four or more substituents are linked to one another.

In the present specification, "substituted with A or B" includes not only the case of being substituted with A alone or with B alone, but also the case of being substituted with A and B.

Examples of the substituents will be described below; however, the substituents are not limited thereto.

In the present specification, examples of a halogen group include fluorine (-F), chlorine (-Cl), bromine (-Br) or iodine (-I) .

In the present specification, a silyl group may be represented by a chemical formula of -SiYₐY_{b}Y_{c}, and the Yₐ, Y_{b}, and Y_{c} may be each hydrogen; a substituted or unsubstituted alkyl group; or a substituted or unsubstituted aryl group. Specific examples of the silyl group include a trimethylsilyl group, a triethylsilyl group, a tert-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, a boron group may be represented by a chemical formula of -BY_{d}Yₑ, and the Y_{d} and Yₑ may be each hydrogen; a substituted or unsubstituted alkyl group; or a substituted or unsubstituted aryl group. Specific examples of the boron group include a dimethylboron group, a diethylboron group, a tert-butylmethylboron group, a diphenylboron group, a phenylboron group, and the like, but are not limited thereto.

In the present specification, the alkyl group may be straight-chained or branched, and the number of carbon atoms thereof is not particularly limited, but is preferably 1 to 60. According to an exemplary embodiment, the number of carbon atoms of the alkyl group is 1 to 30. According to another exemplary embodiment, the number of carbon atoms of the alkyl group is 1 to 20. According to still another exemplary embodiment, the number of carbon atoms of the alkyl group is 1 to 10. Specific examples of the alkyl group include a methyl group, an ethyl group, a propyl group, an n-propyl group, an isopropyl group, a butyl group, an n-butyl group, an isobutyl group, a tert-butyl group, a pentyl group, an n-pentyl group, a hexyl group, an n-hexyl group, a heptyl group, an n-heptyl group, an octyl group, an n-octyl group, and the like, but are not limited thereto.

In the present specification, a cycloalkyl group is not particularly limited, but has preferably 3 to 60 carbon atoms, and according to an exemplary embodiment, the number of carbon atoms of the cycloalkyl group is 3 to 30. According to another exemplary embodiment, the number of carbon atoms of the cycloalkyl group is 3 to 20. According to another embodiment, the number of carbon atoms of the cycloalkyl group is from 3 to 6. Specific examples thereof may include a cyclopropyl group, a cyclobutyl group, a cyclopentyl group, a cyclohexyl group, a cycloheptyl group, a cyclooctyl group and the like, but are not limited thereto.

In the present specification, an aryl group is not particularly limited, but has preferably 6 to 60 carbon atoms, and may be a monocyclic aryl group or a polycyclic aryl group. According to one embodiment, the number of carbon atoms of the aryl group is from 6 to 30. According to one embodiment, the number of carbon atoms of the aryl group is from 6 to 20. Examples of a monocyclic aryl group as the aryl group include a phenyl group, a biphenyl group, a terphenyl group, and the like, but are not limited thereto. Examples of the polycyclic aryl group include a naphthyl group, an anthracenyl group, a phenanthrenyl group, a pyrenyl group, a perylenyl group, a triphenyl group, a chrysenyl group, a fluorenyl group, and the like, but are not limited thereto.

In the present specification, No. 9 carbon atom (C) of a fluorenyl group may be substituted with an alkyl group, an aryl group, or the like, and two substituents may be bonded to each other to form a spiro structure such as cyclopentane or fluorene.

In the present specification, the substituted aryl group may also include a form in which an aliphatic ring is condensed to the aryl group. For example, a tetrahydronaphthalene group or a dihydroindene group having the following structure is included in a substituted aryl group. In the following structure, one of the carbons of a benzene ring may be linked to another position.

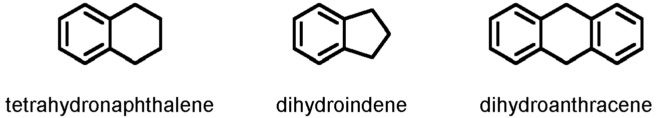

In the present specification, the above-described description on the aryl group may be applied to an arylene group except for a divalent arylene group.

In the present specification, a heterocyclic group is a cyclic group including one or more of N, O, P, S, Si, and Se as a heteroatom, and the number of carbon atoms thereof is not particularly limited, but is preferably 2 to 60. According to one embodiment, the number of carbon atoms of the heterocyclic group is from 2 to 30. According to one embodiment, the number of carbon atoms of the heterocyclic group is from 2 to 20. Examples of the heterocyclic group include a pyridyl group; a quinoline group; a thiophene group; a dibenzothiophene group; a furan group; a dibenzofuran group; a naphthobenzofuran group; a carbazole group; a benzocarbazole group; a naphthobenzothiophene group; a dibenzosilole group; a naphthobenzosilole group; a hexahydrocarbazole group; dihydroacridine group; a dihydrodibenzoazasiline group; a phenoxazine group; a phenothiazine group; a dihydrodibenzoazasiline group; a spiro(dibenzosilole-dibenzoazasiline) group; a spiro(acridine-fluorene) group, and the like, but are not limited thereto.

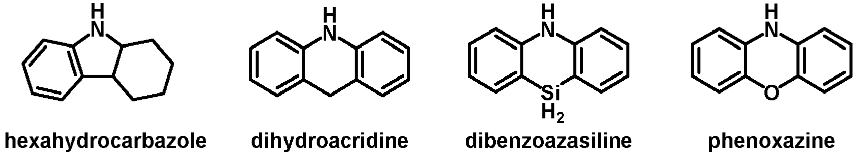

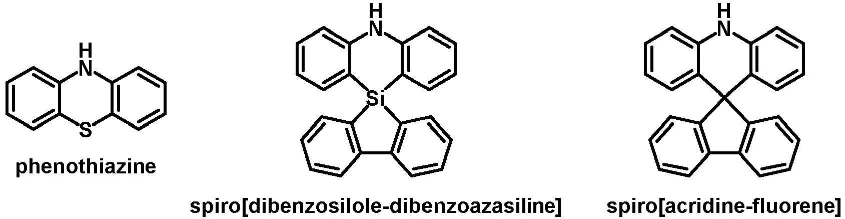

In the present specification, the above-described description on the heterocyclic group may be applied to a heteroaryl group except for an aromatic heteroaryl group.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other. In addition, substituents (four in total) linked to two consecutive carbons in an aliphatic ring may be interpreted as "adjacent" groups.

In the present specification, the "adjacent groups among the substituents are bonded to each other to form a ring" means that a ring including a substituent which participates in the formation of the ring is formed. This may include an additional ring fused to the ring including the substituent participating in the ring formation.

In the present specification, the "adjacent groups among the substituents are bonded to each other to form a ring" means that a group is bonded to an adjacent group to form a substituted or unsubstituted hydrocarbon ring; or a substituted or unsubstituted hetero ring, and specifically means that a group is bonded to an adjacent group to form a substituted or unsubstituted aromatic hydrocarbon ring; a substituted or unsubstituted aliphatic hydrocarbon ring; a substituted or unsubstituted aliphatic hetero ring; a substituted or unsubstituted aromatic hetero ring; or a combination thereof.

In the present specification, an aromatic hydrocarbon ring means a hydrocarbon ring in which pi electrons are completely conjugated and are planar, and the description on the aryl group may be applied to an aromatic hydrocarbon ring except for a divalent aromatic hydrocarbon ring.

In the present specification, an aliphatic hydrocarbon ring has a cyclically bonded structure, and means a nonaromatic ring. Examples of the aliphatic hydrocarbon ring include cycloalkyl or cycloalkene, and the above-described description on the cycloalkyl group or cycloalkenyl group may be applied to the aliphatic hydrocarbon ring except for a divalent aliphatic hydrocarbon ring. Further, a substituted aliphatic hydrocarbon ring also includes an aliphatic hydrocarbon ring in which aromatic rings are fused.

In the present specification, a fused ring of an aromatic hydrocarbon ring and an aliphatic hydrocarbon ring means that an aromatic hydrocarbon ring and an aliphatic hydrocarbon ring form a fused ring. Examples of the condensed ring of the aromatic ring and the aliphatic ring include a 1,2,3,4-tetrahydronaphthalene group, a 2,3-dihydro-1H-indene group, and the like, but are not limited thereto.

In the present specification, the aromatic hetero ring means a hetero ring which includes a heteroatom which is not carbon and hydrogen, and in which the pi electrons are fully conjugated and planar, and the aliphatic hetero ring means a ring which has a structure including a heteroatom other than carbon and hydrogen and being cyclically bonded, and is not aromatic.

In the present specification, when adjacent groups among substituents are bonded to each other to form a ring, it is possible to form any one of the following structures.

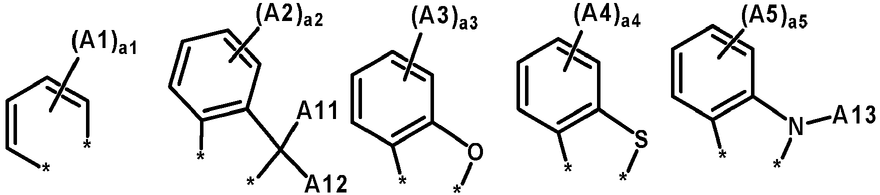

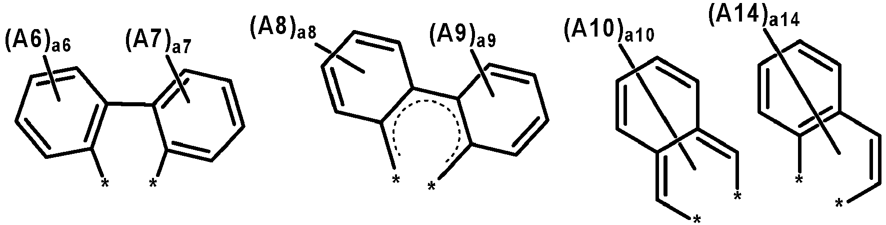

In the structures,
A1 to A14 are each independently hydrogen; deuterium; a halogen group; a cyano group; a substituted or unsubstituted alkyl group; a substituted or unsubstituted cycloalkyl group; a substituted or unsubstituted aryl group; or a substituted or unsubstituted heterocyclic group,
a1 to a9 are each an integer from 1 to 4,
a10 and a14 are each an integer from 1 to 6, and
* denotes a position that is substituted.

According to an exemplary embodiment of the present specification, A1 is hydrogen; or a substituted or unsubstituted aryl group.

According to an exemplary embodiment of the present specification, A1 is hydrogen; or a substituted or unsubstituted aryl group having 6 to 30 carbon atoms.

According to an exemplary embodiment of the present specification, A1 is hydrogen; or a substituted or unsubstituted phenyl group.

According to an exemplary embodiment of the present specification, A1 is hydrogen or a phenyl group.

According to an exemplary embodiment of the present specification, A2 to A10 and A14 are hydrogen.

According to an exemplary embodiment of the present specification, A11 to A13 are each independently a substituted or unsubstituted alkyl group; or a substituted or unsubstituted aryl group.

According to an exemplary embodiment of the present specification, A11 to A13 are each independently a substituted or unsubstituted alkyl group having 1 to 30 carbon atoms; or a substituted or unsubstituted aryl group having 6 to 30 carbon atoms.

According to an exemplary embodiment of the present specification, A11 and A12 are a methyl group.

According to an exemplary embodiment of the present specification, A13 is a substituted or unsubstituted benzene, or a substituted or unsubstituted naphthalene.

In the present specification, Chemical Formula 1 is any one of the following Chemical Formulae 1-1 to 1-3.

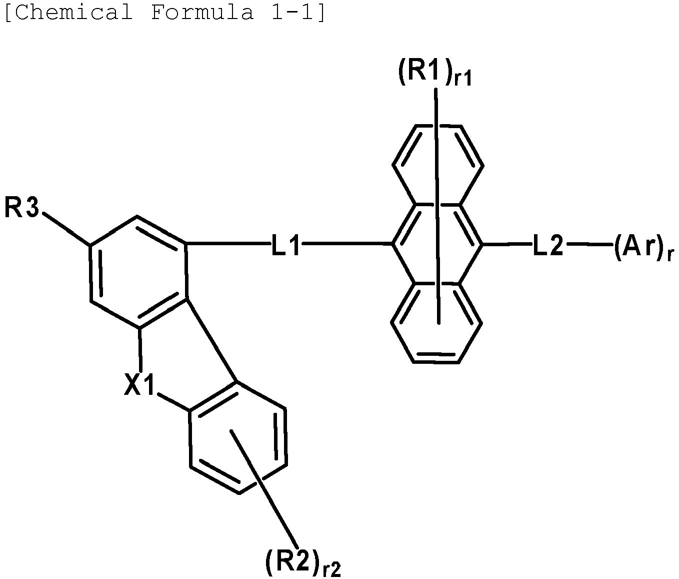

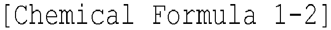

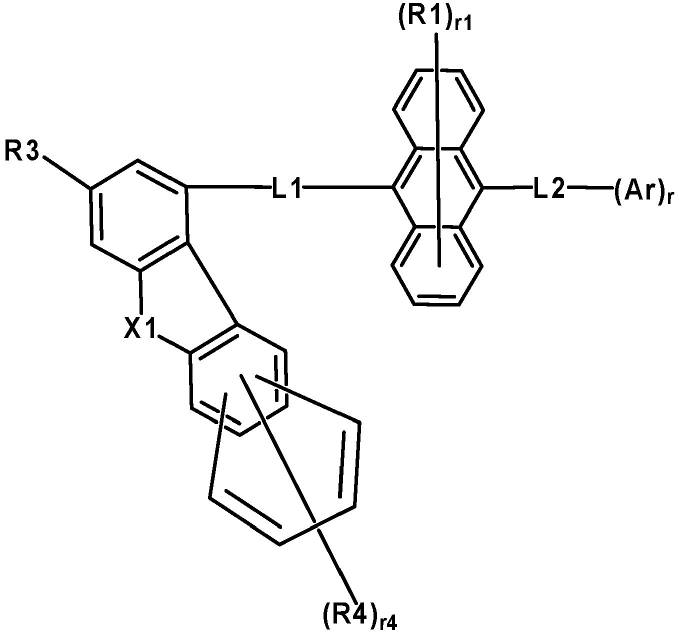

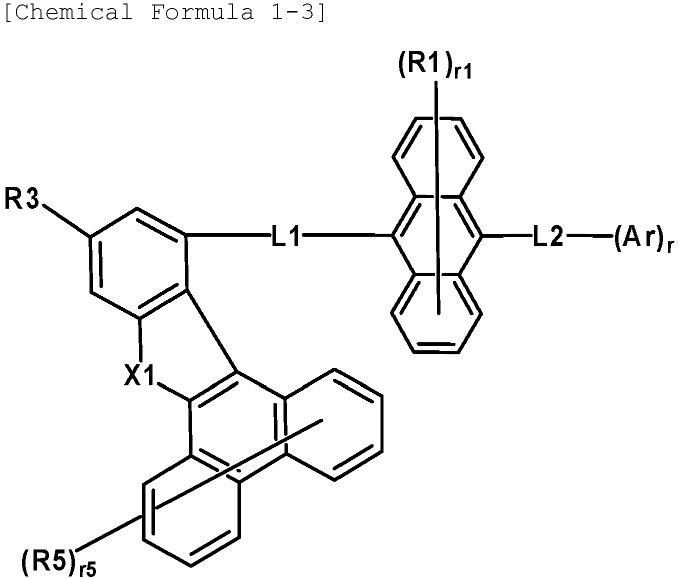

In Chemical Formulae 1-1 to 1-3,
X1, R1, R2, R3, r1, L1, L2, Ar and r are the same as the definitions in Chemical Formula 1,
R4 and R5 are the same as or different from each other, and are each independently hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S,
r2 is an integer from 1 to 4,
r4 is an integer from 1 to 6,
r5 is an integer from 1 to 8,
when r4 is 2 or higher, R4's are the same as or different from each other, and
when r5 is 2 or higher, R5's are the same as or different from each other.

In the present specification, Chemical Formula 1 is any one of the following Chemical Formulae 2 to 5.

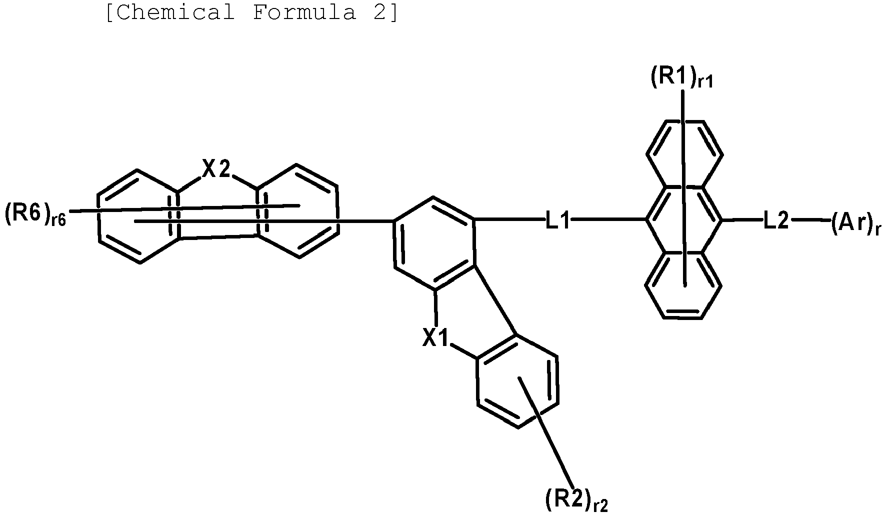

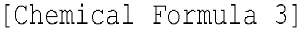

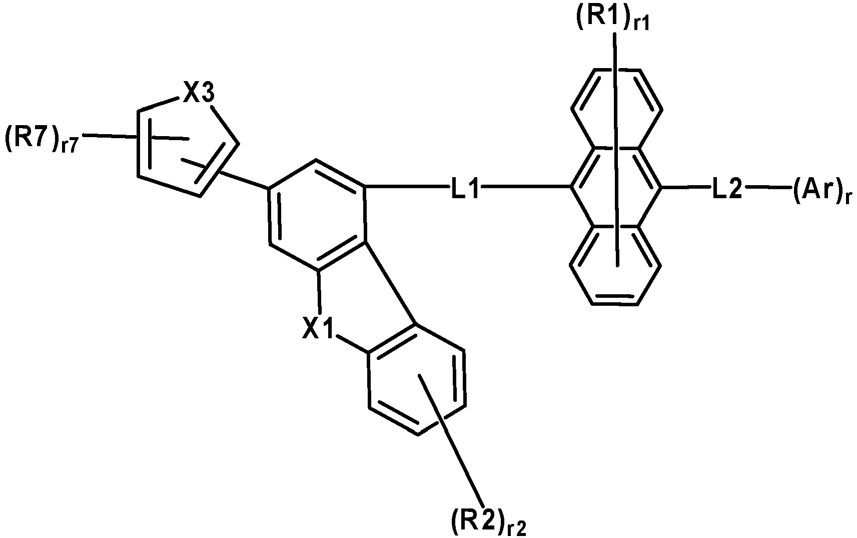

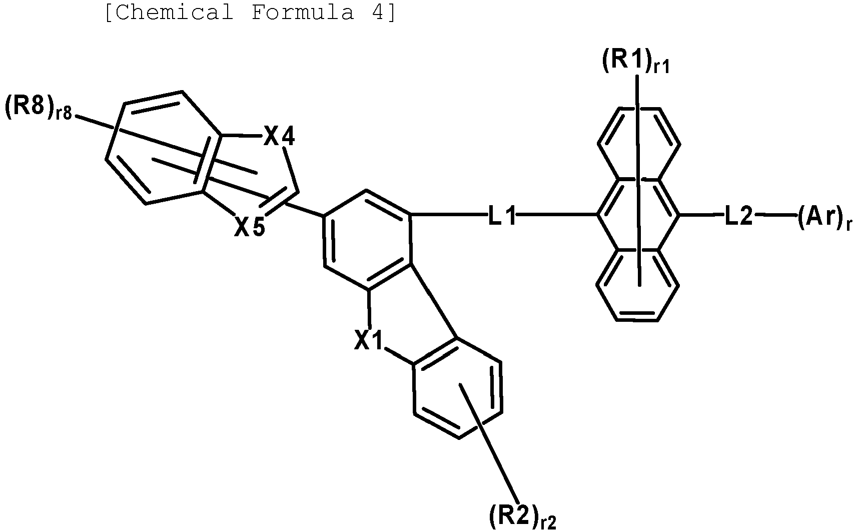

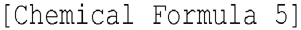

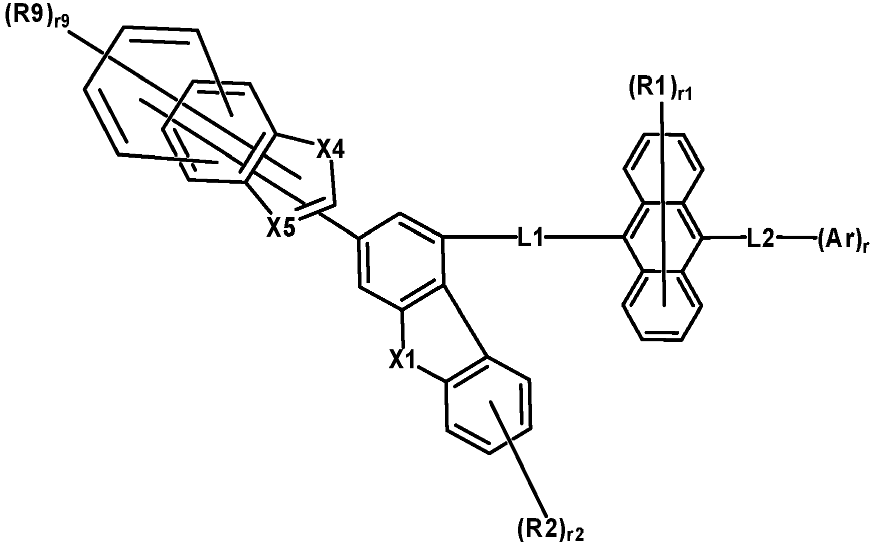

In Chemical Formulae 2 to 5,
X1, R1, R2, r1, L1, L2, Ar and r are the same as the definitions in Chemical Formula 1,
X2 is NR, O or S,
X3 and X4 are each independently O or S,
X5 is N or CR',
R, R' and R7 to R9 are the same as or different from each other, and are each independently hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S,
R6 is hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S, or adjacent groups are optionally bonded to each other to form a substituted or unsubstituted ring,
r2 is an integer from 1 to 4,
r6 is an integer from 1 to 7,
r7 is an integer from 1 to 3,
r8 is an integer from 1 to 4,
r9 is an integer from 1 to 6,
when r6 is 2 or higher, R6's are the same as or different from each other,
when r7 is 2 or higher, R7's are the same as or different from each other,
when r8 is 2 or higher, R8's are the same as or different from each other, and
when r9 is 2 or higher, R9's are the same as or different from each other.

In the present specification, Chemical Formula 1 is any one of the following Chemical Formulae 2-1 to 2-4.

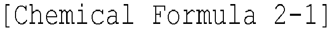

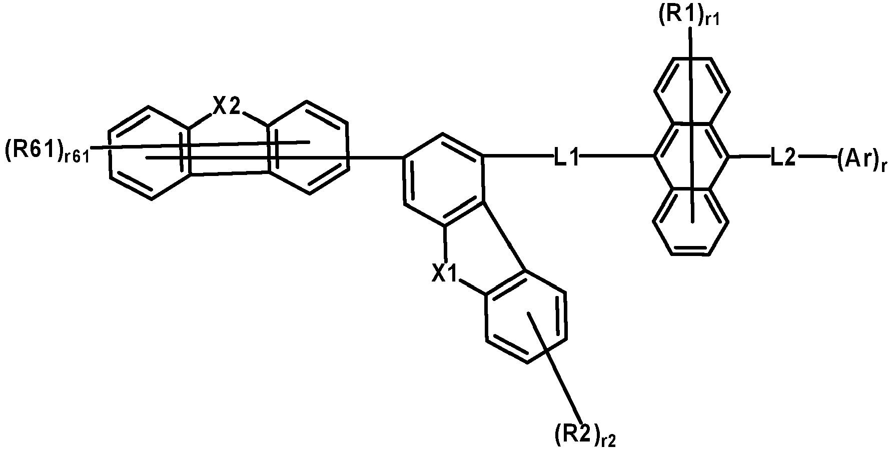

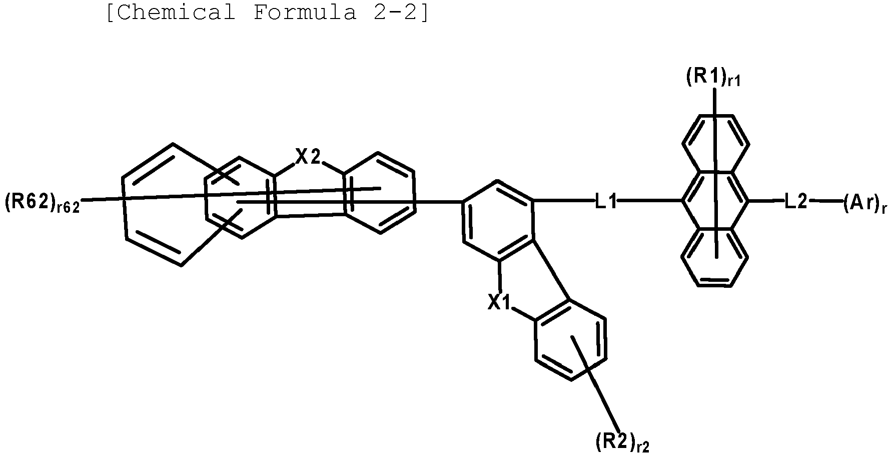

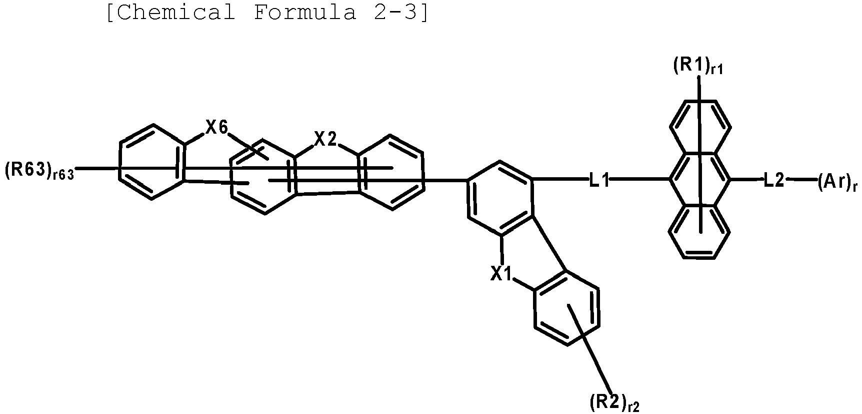

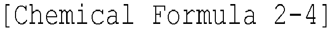

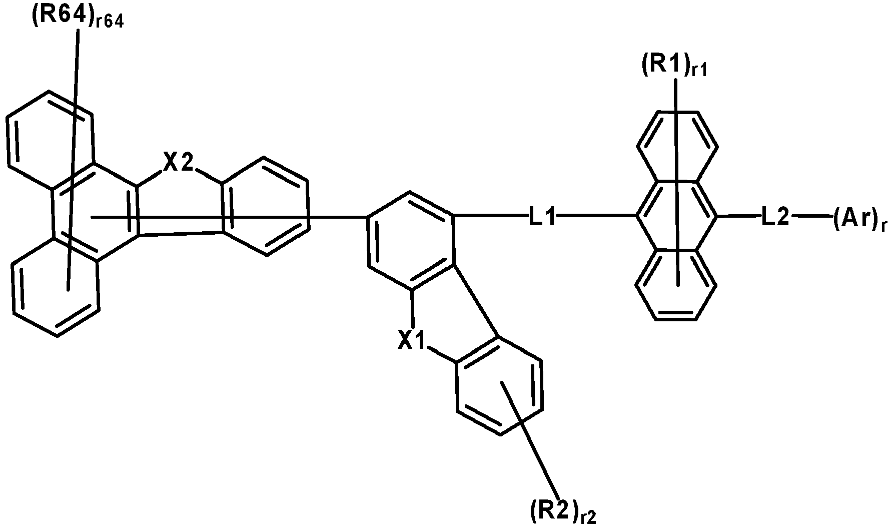

In Chemical Formulae 2-1 to 2-4,
X1, X2, R1, R2, r1, r2, L1, L2, Ar and r are the same as the definitions in Chemical Formula 2,
X6 is NR, O or S,
R and R61 to R64 are the same as or different from each other, and are each independently hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S,
r61 is an integer from 1 to 7,
r62 and r63 are each an integer from 1 to 9,
r64 is an integer from 1 to 11,
when r61 is 2 or higher, R61's are the same as or different from each other,
when r62 is 2 or higher, R62's are the same as or different from each other,
when r63 is 2 or higher, R63's are the same as or different from each other, and
when r64 is 2 or higher, R64's are the same as or different from each other.

In the present specification, X1 is O.

In the present specification, X1 is S.

In the present specification, R1 is hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group including at least one selected from N, O and S.

In the present specification, R1 is hydrogen, deuterium, a substituted or unsubstituted alkyl group, a substituted or unsubstituted cycloalkyl group, a substituted or unsubstituted aryl group, or a substituted or unsubstituted heterocyclic group including at least one selected from N, O and S.

In the present specification, R1 is hydrogen, deuterium, a substituted or unsubstituted alkyl group, a substituted or unsubstituted cycloalkyl group, or a substituted or unsubstituted aryl group.

In the present specification, R1 is hydrogen, deuterium, or a substituted or unsubstituted aryl group.

In the present specification, R1 is hydrogen, deuterium, or a substituted or unsubstituted aryl group having 6 to 30 carbon atoms.

In the present specification, R1 is hydrogen, deuterium, a substituted or unsubstituted phenyl group, a substituted or unsubstituted biphenyl group, a substituted or unsubstituted terphenyl group, a substituted or unsubstituted naphthyl group, a substituted or unsubstituted fluorenyl group, a substituted or unsubstituted phenanthrenyl group, a substituted or unsubstituted triphenylenyl group, or a substituted or unsubstituted pyrenyl group.

In the present specification, R1 is hydrogen, deuterium, a substituted or unsubstituted phenyl group, a substituted or unsubstituted biphenyl group, a substituted or unsubstituted terphenyl group, or a substituted or unsubstituted naphthyl group.

In the present specification, R2, R4 and R5 are the same as or different from each other, and are each independently hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group including at least one selected from N, O and S.

In the present specification, R2, R4 and R5 are the same as or different from each other, and are each independently hydrogen, deuterium, a substituted or unsubstituted alkyl group, a substituted or unsubstituted cycloalkyl group, a substituted or unsubstituted aryl group, or a substituted or unsubstituted heterocyclic group including at least one selected from N, O and S.

In the present specification, R2, R4 and R5 are the same as or different from each other, and are each independently hydrogen, deuterium, a substituted or unsubstituted alkyl group having 1 to 10 carbon atoms, a substituted or unsubstituted cycloalkyl group having 3 to 20 carbon atoms, a substituted or unsubstituted aryl group having 6 to 30 carbon atoms, or a substituted or unsubstituted heterocyclic group having 2 to 30 carbon atoms, which includes at least one selected from N, O and S.

In the present specification, R2, R4 and R5 are the same as or different from each other, and are each independently hydrogen, deuterium, a methyl group, an ethyl group, a propyl group, a t-butyl group, a cyclohexyl group, a tetrahydronaphthyl group unsubstituted or substituted with an alkyl group, a phenyl group, a biphenyl group, a terphenyl group, a naphthyl group, a furanyl group or a thiophenyl group.

In the present specification, when the adjacent groups in R2 are bonded to each other to form a substituted or unsubstituted ring, a substituted or unsubstituted hydrocarbon ring or a substituted or unsubstituted hetero ring may be formed.

In the present specification, when the adjacent groups in R2 are bonded to each other to form a substituted or unsubstituted ring, a substituted or unsubstituted benzene ring may be formed.

In the present specification, R3 is a polycyclic heterocyclic group including at least one selected from N, O and S; or a substituted or unsubstituted monocyclic heterocyclic group including at least one selected from O and S.

In the present specification, R3 is a substituted or unsubstituted furanyl group, a substituted or unsubstituted thiophenyl group, a substituted or unsubstituted benzofuranyl group, a substituted or unsubstituted benzothiophenyl group, a substituted or unsubstituted dibenzofuranyl group, a substituted or unsubstituted dibenzothiophenyl group, a substituted or unsubstituted benzoxazolyl group, a substituted or unsubstituted benzothiazolyl group, a substituted or unsubstituted naphtho oxazolyl group, or a substituted or unsubstituted naphthothiazolyl group, and among the substituents additionally substituted with these substituents, adjacent groups may be bonded to each other to form a substituted or unsubstituted ring.

In the present specification, R, R' and R6 to R9 are the same as or different from each other, and are each independently hydrogen, deuterium, a substituted or unsubstituted alkyl group, a substituted or unsubstituted cycloalkyl group, a substituted or unsubstituted aryl group, or a substituted or unsubstituted heterocyclic group including at least one selected from N, O and S.

In the present specification, R and R' are the same as or different from each other, and are each independently hydrogen, deuterium, or a substituted or unsubstituted aryl group.

In the present specification, R and R' are the same as or different from each other, and are each independently hydrogen, deuterium, or a substituted or unsubstituted aryl group having 6 to 30 carbon atoms.

In the present specification, R and R' are the same as or different from each other, and are each independently hydrogen, deuterium, a phenyl group, a biphenyl group, a terphenyl group, or a naphthyl group.

In the present specification, R and R' are the same as or different from each other, and are each independently hydrogen, deuterium, or a phenyl group.

In the present specification, R6 and R7 are the same as or different from each other, and are each independently hydrogen, deuterium, or a substituted or unsubstituted alkyl group.

In the present specification, R8 and R9 are the same as or different from each other, and are each independently hydrogen, deuterium, a substituted or unsubstituted alkyl group, a substituted or unsubstituted cycloalkyl group, a substituted or unsubstituted aryl group, or a substituted or unsubstituted heterocyclic group including at least one selected from N, O and S.

In the present specification, R8 and R9 are the same as or different from each other, and are each independently hydrogen, deuterium, a substituted or unsubstituted alkyl group having 1 to 10 carbon atoms, a substituted or unsubstituted cycloalkyl group having 3 to 20 carbon atoms, a substituted or unsubstituted aryl group having 6 to 30 carbon atoms, or a substituted or unsubstituted heterocyclic group having 2 to 30 carbon atoms, which includes at least one selected from N, O and S.

In the present specification, R8 and R9 are the same as or different from each other, and are each independently hydrogen, deuterium, a methyl group, an ethyl group, a propyl group, a t-butyl group, a cyclohexyl group, a cyclopentyl group, a tetrahydronaphthyl group unsubstituted or substituted with an alkyl group, a phenyl group, a biphenyl group, a terphenyl group, a naphthyl group, a furanyl group or a thiphenyl group.

In the present specification, R61 to R64 are the same as or different from each other, and are each independently hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group including at least one selected from N, O and S.

In the present specification, R61 to R64 are the same as or different from each other, and are each independently hydrogen, deuterium, a substituted or unsubstituted alkyl group, a substituted or unsubstituted cycloalkyl group, a substituted or unsubstituted aryl group, or a substituted or unsubstituted heterocyclic group including at least one selected from N, O and S.

In the present specification, R61 to R64 are the same as or different from each other, and are each independently hydrogen or deuterium.

In the present specification, L1 and L2 are the same as or different from each other, and are each independently a direct bond, or a substituted or unsubstituted arylene group.

In the present specification, L1 and L2 are the same as or different from each other, and are each independently a direct bond, or a substituted or unsubstituted arylene group having 6 to 30 carbon atoms.

In the present specification, L1 and L2 are the same as or different from each other, and are each independently a direct bond, a substituted or unsubstituted phenylene group, a substituted or unsubstituted biphenylene group, a substituted or unsubstituted naphthylene group, or a substituted or unsubstituted fluorenylene group.

According to an exemplary embodiment of the present specification, L1 and L2 are the same as or different from each other, and may be each independently represented by a direct bond or any one of the following structures.

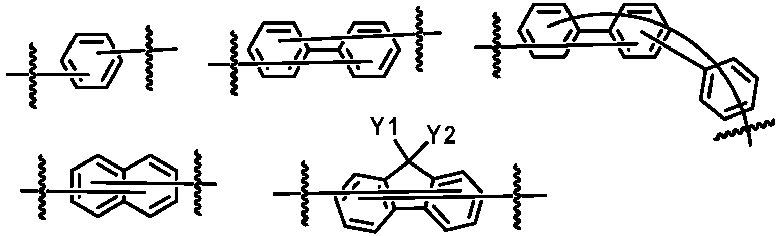

In the structures,
Y1 and Y2 are each independently hydrogen, deuterium, a substituted or unsubstituted alkyl group, or a substituted or unsubstituted aryl group.

According to an exemplary embodiment of the present specification, Ar is a substituted or unsubstituted aryl group.

In the present specification, Ar is a substituted or unsubstituted aryl group having 6 to 30 carbon atoms.

In the present specification, Ar is a substituted or unsubstituted phenyl group, a substituted or unsubstituted biphenyl group, a substituted or unsubstituted terphenyl group, a substituted or unsubstituted naphthyl group, a substituted or unsubstituted fluorenyl group, a substituted or unsubstituted phenanthrenyl group, a substituted or unsubstituted triphenylenyl group, or a substituted or unsubstituted pyrenyl group.

In the present specification, Ar may be represented by any one of the following structures.

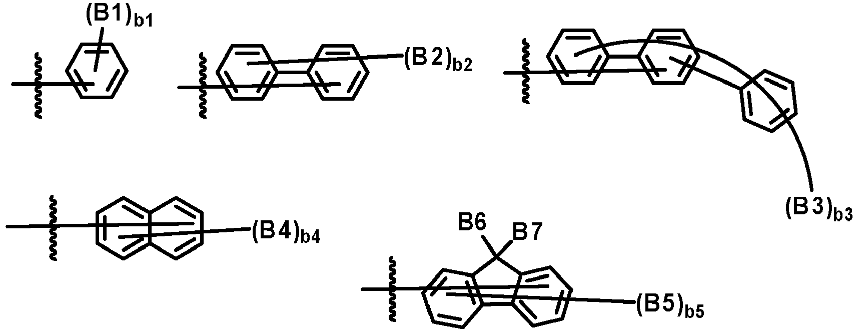

In the structures,
B1 to B7 are each independently hydrogen; deuterium; a halogen group; a cyano group; a substituted or unsubstituted alkyl group; or a substituted or unsubstituted aryl group,
b1 is an integer from 1 to 5,
b2 is an integer from 1 to 9,
b3 is an integer from 1 to 13,
b4 and b5 are each an integer from 0 to 7,
when b1 is 2 or higher, B1's are the same as or different from each other,
when b2 is 2 or higher, B2's are the same as or different from each other,
when b3 is 2 or higher, B3's are the same as or different from each other,
when b4 is 2 or higher, B4's are the same as or different from each other, and
when b5 is 2 or higher, B5's are the same as or different from each other.

According to an exemplary embodiment of the present specification, B1 to B5 are hydrogen or deuterium.

According to an exemplary embodiment of the present specification, B6 and B7 are each independently a substituted or unsubstituted alkyl group; or a substituted or unsubstituted aryl group.

According to an exemplary embodiment of the present specification, B6 and B7 are each independently a substituted or unsubstituted alkyl group having 1 to 60 carbon atoms; or a substituted or unsubstituted aryl group having 6 to 60 carbon atoms.

According to an exemplary embodiment of the present specification, B6 and B7 are each independently a substituted or unsubstituted alkyl group having 1 to 30 carbon atoms; or a substituted or unsubstituted aryl group having 6 to 30 carbon atoms.

In the present specification, the compound of Chemical Formula 1 is any one of the following structures.

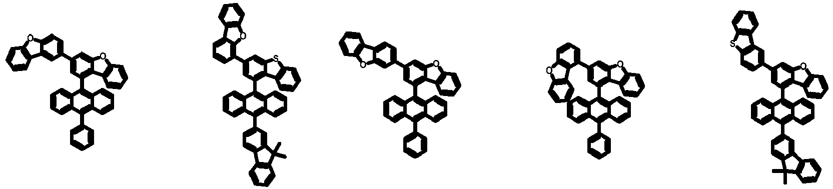

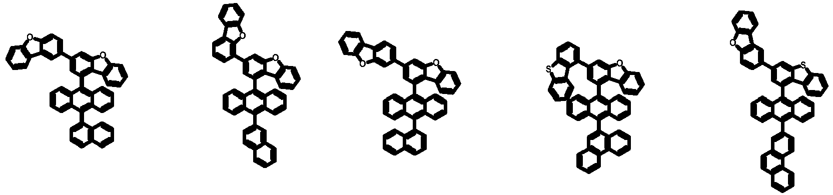

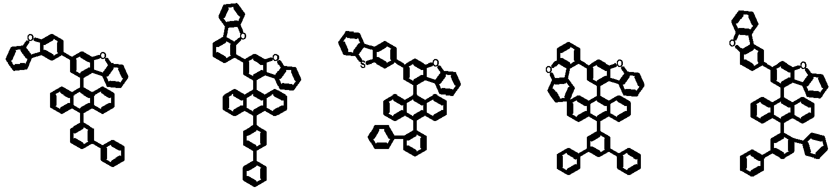

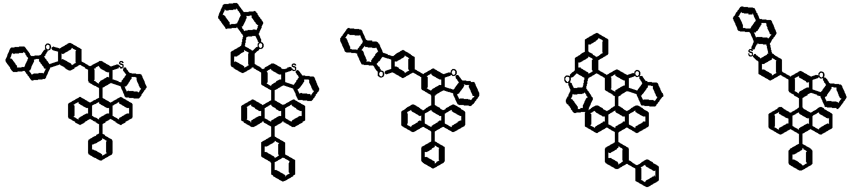

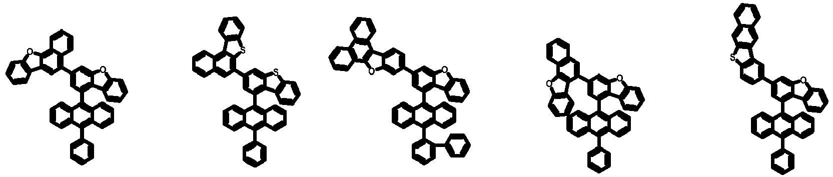

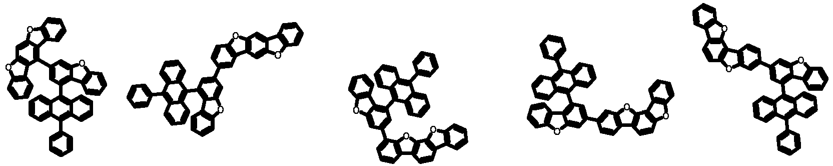

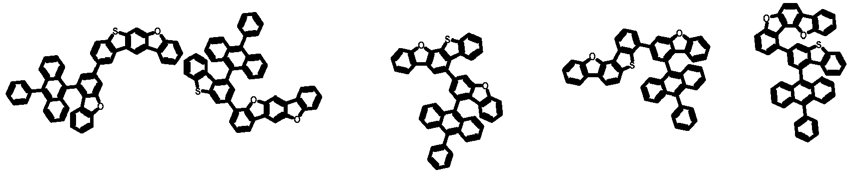

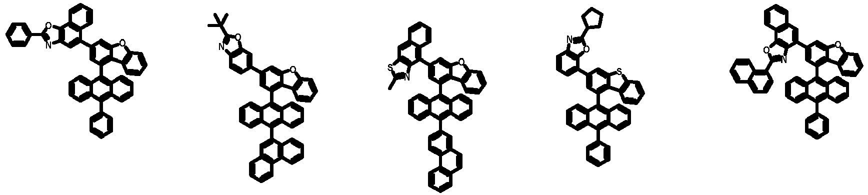

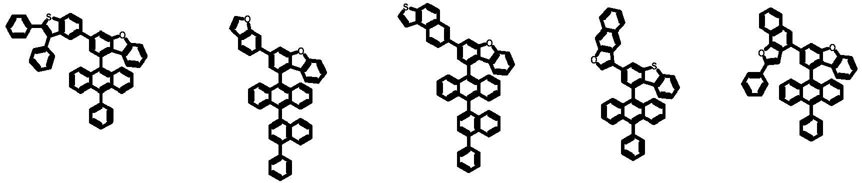

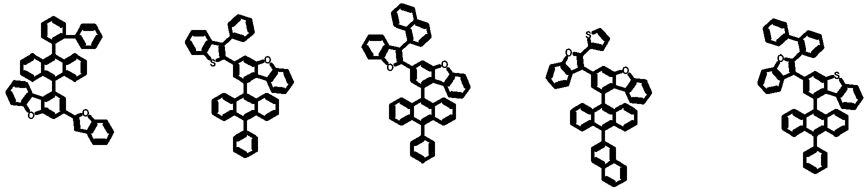

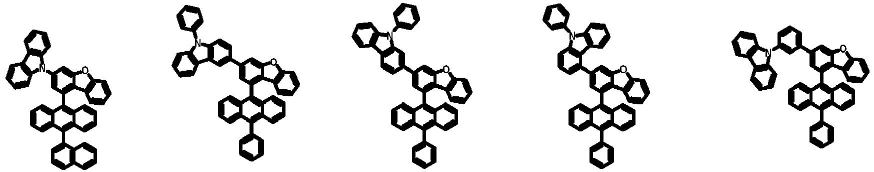

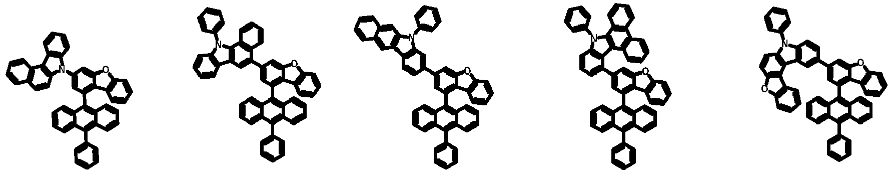

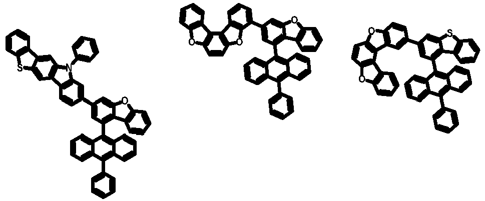

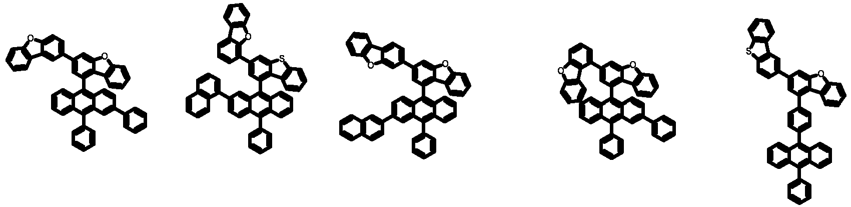

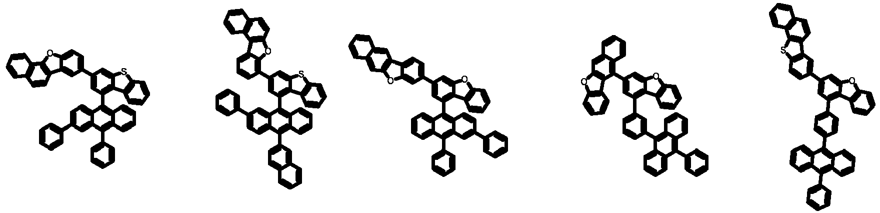

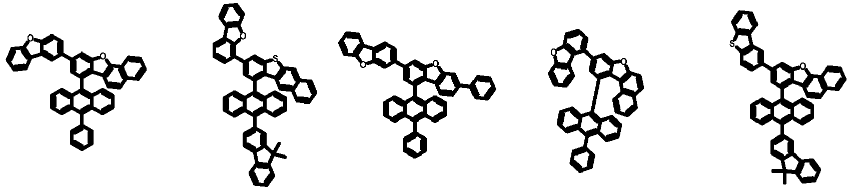

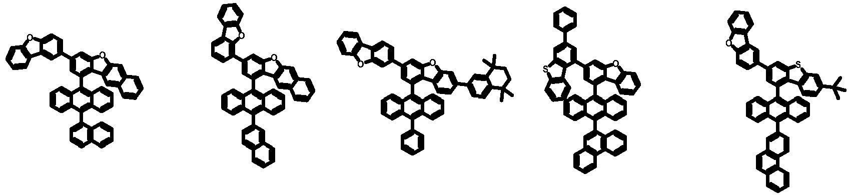

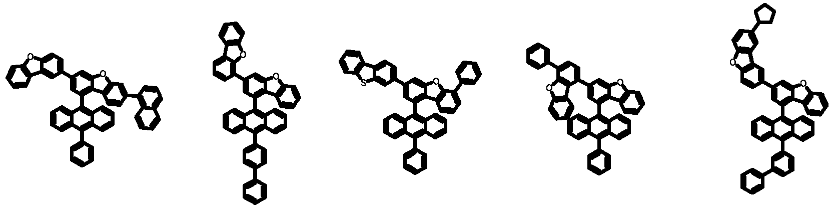

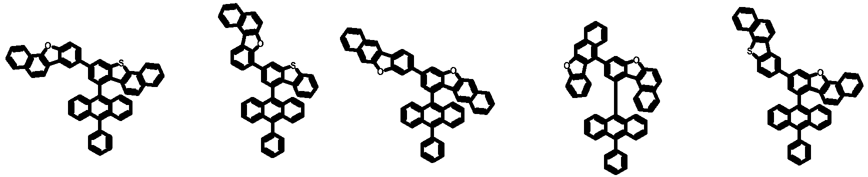

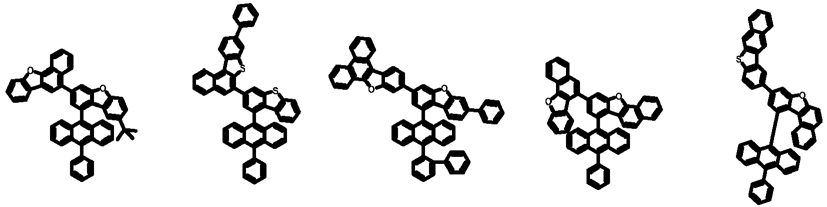

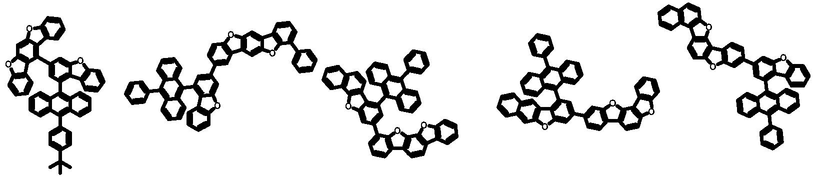

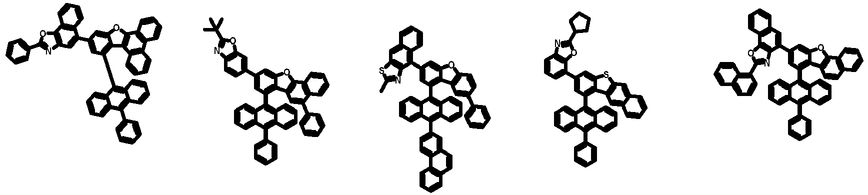

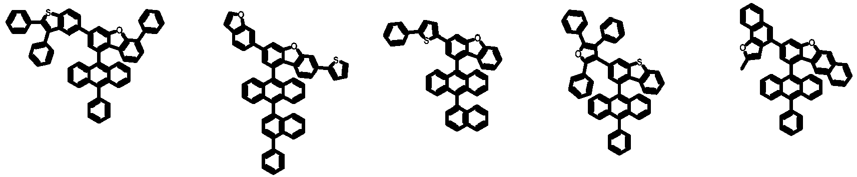

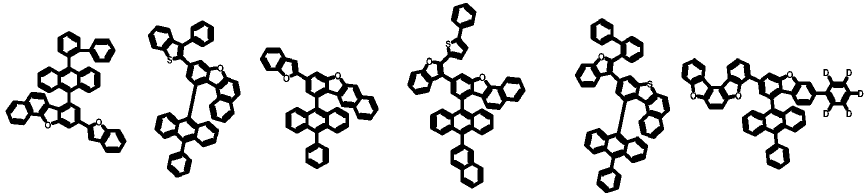

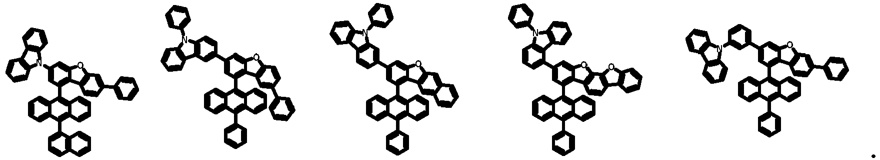

Further, the present specification provides an organic light emitting device including the above-described compound.

In an exemplary embodiment of the present specification, provided is an organic light emitting device including: a first electrode; a second electrode; and an organic material layer including one or more layers provided between the first electrode and the second electrode, in which one or more layers of the organic material layer include the compound.

The organic material layer of the organic light emitting device of the present specification may also have a single-layered structure, but may have a multi-layered structure in which two or more organic material layers are stacked. For example, the organic light emitting device of the present invention may have a structure including a hole injection layer, a hole transport layer, a light emitting layer, an electron transport layer, an electron injection layer, and the like as organic material layers. However, the structure of the organic light emitting device is not limited thereto, and may include a fewer number of organic layers.

In an exemplary embodiment of the present specification, the organic material layer includes a hole injection layer or a hole transport layer, and the hole injection layer or the hole transport layer includes the compound.

In an exemplary embodiment of the present specification, the organic material layer includes a light emitting layer, and the light emitting layer includes the compound. Specifically, the light emitting layer may include a dopant and a host including the compound.

In an exemplary embodiment of the present specification, the organic material layer includes an electron transport layer or an electron injection layer, and the electron transport layer or the electron injection layer includes the compound.

In an exemplary embodiment of the present specification, the organic material layer includes a light emitting layer, and further includes one or two or more layers selected from the group consisting of a hole injection layer, a hole transport layer, an electron transport layer, an electron injection layer, a hole adjusting layer, and an electron adjusting layer.

In an exemplary embodiment of the present specification, the organic light emitting device includes: an anode; a cathode; and an organic material layer having one or more layers provided between the anode and the cathode, the organic material layer includes: a light emitting layer; a hole transport region provided between the light emitting layer and the anode; and an electron transport region provided between the light emitting layer and the cathode, and the light emitting layer includes the compound.

In an exemplary embodiment of the present specification, as the organic material layer in the hole transport region, one or more may be selected from the group consisting of a hole transport layer, a hole injection layer, a layer which simultaneously transports and injects holes, and a hole adjusting layer.

In an exemplary embodiment of the present specification, as the organic material layer in the electron transport region, one or more may be selected from the group consisting of an electron transport layer, an electron injection layer, a layer which simultaneously transports and injects electrons, and an electron adjusting layer.

In another exemplary embodiment, the organic light emitting device may be a normal type organic light emitting device in which a first electrode, an organic material layer having one or more layers, and a second electrode are sequentially stacked on a substrate.

In still another exemplary embodiment, the organic light emitting device may be an inverted type organic light emitting device in which a second electrode, an organic material layer having one or more layers, and a first electrode are sequentially stacked on a substrate.

The organic light emitting device may have, for example, the stacking structure described below, but the stacking structure is not limited thereto.
(1) Anode/Hole transport layer/Light emitting layer/Cathode
(2) Anode/Hole injection layer/Hole transport layer/Light emitting layer/Cathode
(3) Anode/Hole injection layer/Hole buffer layer/Hole transport layer/Light emitting layer/Cathode
(4) Anode/Hole transport layer/Light emitting layer/Electron transport layer/Cathode
(5) Anode/Hole transport layer/Light emitting layer/Electron transport layer/Electron injection layer/Cathode
(6) Anode/Hole injection layer/Hole transport layer/Light emitting layer/Electron transport layer/Cathode
(7) Anode/Hole injection layer/Hole transport layer/Light emitting layer/Electron transport layer/Electron injection layer/Cathode
(8) Anode/Hole injection layer/Hole buffer layer/Hole transport layer/Light emitting layer/Electron transport layer/Cathode
(9) Anode/Hole injection layer/Hole buffer layer/Hole transport layer/Light emitting layer/Electron transport layer/Electron injection layer/Cathode
(10) Anode/Hole transport layer/Hole adjusting layer/Light emitting layer/Electron transport layer/Cathode
(11) Anode/Hole transport layer/Hole adjusting layer/Light emitting layer/Electron transport layer/Electron injection layer/Cathode
(12) Anode/Hole injection layer/Hole transport layer/Hole adjusting layer/Light emitting layer/Electron transport layer/Cathode
(13) Anode/Hole injection layer/Hole transport layer/Hole adjusting layer/Light emitting layer/Electron transport layer/Electron injection layer/Cathode
(14) Anode/Hole transport layer/Light emitting layer/Electron adjusting layer/Electron transport layer/Cathode
(15) Anode/Hole transport layer/Light emitting layer/Electron adjusting layer/Electron transport layer/Electron injection layer/Cathode
(16) Anode/Hole injection layer/Hole transport layer/Light emitting layer/Electron adjusting layer/Electron transport layer/Cathode
(17) Anode/Hole injection layer/Hole transport layer/Light emitting layer/Electron adjusting layer/Electron transport layer/Electron injection layer/Cathode
(18) Anode/Hole injection layer/Hole transport layer/Light emitting layer/Layer which simultaneously injects and transports electrons/Cathode
(19) Anode/Hole injection layer/Hole transport layer/Hole adjusting layer/Light emitting layer/Electron adjusting layer/Electron transport layer/Electron injection layer/Cathode
(20) Anode/Hole injection layer/Hole transport layer/Hole adjusting layer/Light emitting layer/Electron adjusting layer/Electron transport layer/Cathode
(21) Anode/Hole injection layer/Hole transport layer/Hole adjusting layer/Light emitting layer/Electron adjusting layer/Layer which simultaneously injects and transports electrons/Cathode

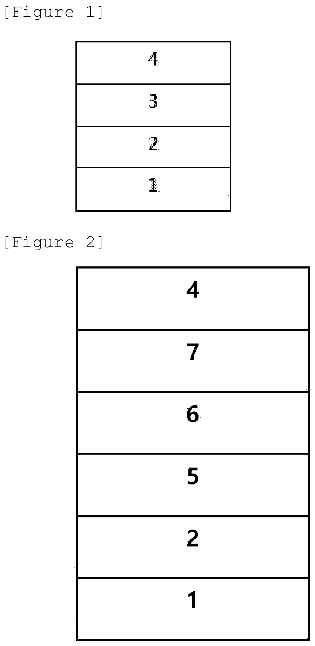

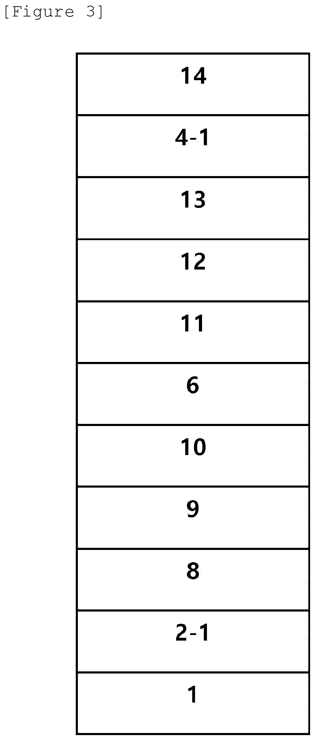

For example, the structure of the organic light emitting device according to an exemplary embodiment of the present specification is exemplified in FIGS. 1 and 2.

FIG. 1 exemplifies a structure of an organic light emitting device in which a first electrode 2, an organic material layer 3, and a second electrode 4 are sequentially stacked on a substrate 1.

FIG. 2 exemplifies a structure of an organic light emitting device in which the first electrode 2, a first organic material layer 5, a light emitting layer 6, a second organic material layer 7, and the second electrode 4 are sequentially stacked on the substrate 1. In the structure described above, the compound may be included in the light emitting layer 6.

FIG. 2 exemplifies an organic light emitting device, and the first organic material layer and the second organic material layer may serve as a hole transport region and an electron transport region, respectively, according to the first electrode and the second electrode. In this case, the hole transport region means an organic material layer between an anode and a light emitting layer, and includes, for example, at least one of a hole injection layer, a hole transport layer, and a hole adjusting layer, and the electron transport region means an organic material layer between the cathode and the light emitting layer, and includes, for example, at least one of an electron injection layer, an electron transport layer, and an electron adjusting layer.

The organic light emitting device of the present specification may be manufactured by the materials and methods known in the art, except that one or more layers of the organic material layer include the compound of the present specification, that is, the compound.

When the organic light emitting device includes a plurality of organic material layers, the organic material layers may be formed of the same material or different materials.

The organic light emitting device of the present specification may be manufactured by the materials and methods known in the art, except that one or more layers of the organic material layer include the compound, that is, the compound represented by Chemical Formula 1.

For example, the organic light emitting device of the present specification may be manufactured by sequentially stacking a first electrode, an organic material layer, and a second electrode on a substrate. In this case, the organic light emitting device may be manufactured by depositing a metal or a metal oxide having conductivity, or an alloy thereof on a substrate to form a first electrode, forming an organic material layer including a hole injection layer, a hole transport layer, a light emitting layer, and an electron transport layer thereon, and then depositing a material, which may be used as a second electrode, thereon, by using a physical vapor deposition (PVD) method such as sputtering or e-beam evaporation. In addition to the method described above, an organic light emitting device may be made by sequentially depositing a second electrode material, an organic material layer, and a first electrode material on a substrate.

Further, the compound of Chemical Formula 1 may be formed as an organic material layer by not only a vacuum deposition method, but also a solution application method when an organic light emitting device is manufactured. Here, the solution application method means spin coating, dip coating, doctor blading, inkjet printing, screen printing, a spray method, roll coating, and the like, but is not limited thereto.

In an exemplary embodiment of the present specification, the first electrode is an anode, and the second electrode is a cathode.

In another exemplary embodiment, the first electrode is a cathode, and the second electrode is an anode.

The anode is an electrode which injects holes, and as an anode material, materials having a high work function are usually preferred so as to facilitate the injection of holes into an organic material layer. Specific examples of the anode material which may be used in the present invention include: a metal, such as vanadium, chromium, copper, zinc, and gold, or an alloy thereof; a metal oxide, such as zinc oxide, indium oxide, indium tin oxide (ITO), and indium zinc oxide (IZO); a combination of a metal and an oxide, such as ZnO : Al or SnO₂ : Sb; a conductive polymer, such as poly(3-methylthiophene), poly[3,4-(ethylene-1,2-dioxy)thiophene] (PEDOT), polypyrrole, and polyaniline; and the like, but are not limited thereto.

The cathode is an electrode which injects electrons, and as a cathode material, materials having a low work function are usually preferred so as to facilitate the injection of electrons into an organic material layer. Specific examples of the cathode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

The hole injection layer is a layer which injects holes from an electrode, and a hole injection material is preferably a compound which has a capability of transporting holes and thus has an effect of injecting holes at a first electrode and an excellent effect of injecting holes into a light emitting layer or a light emitting material, prevents excitons produced from the light emitting layer from moving to an electron injection layer or an electron injection material, and is also excellent in the ability to form a thin film. The highest occupied molecular orbital (HOMO) of the hole injection material is preferably a value between the work function of the first electrode material and the HOMO of the neighboring organic material layer. Specific examples of the hole injection material include metal porphyrin, oligothiophene, arylamine-based organic materials, hexanitrile hexaazatriphenylene-based organic materials, quinacridone-based organic materials, perylene-based organic materials, anthraquinone, polyaniline-based and polythiophene-based conductive polymers, and the like, but are not limited thereto.

According to an exemplary embodiment of the present specification, the hole injection layer includes a compound represented by the following Chemical Formula HI-1, but is not limited thereto.

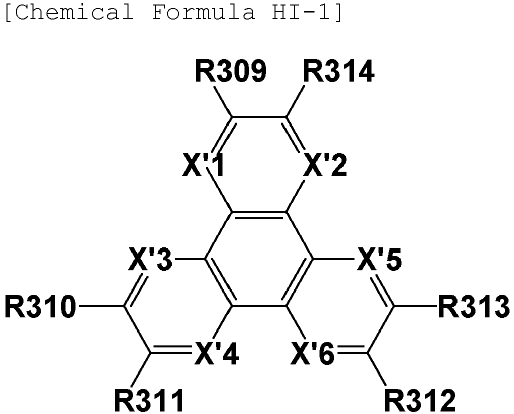

In Chemical Formula HI-1,
at least one of X'1 to X'6 is N, and the others are CH, and
R309 to R314 are the same as or different from each other, and are each independently hydrogen; deuterium; a cyano group; a substituted or unsubstituted alkyl group; a substituted or unsubstituted amine group; a substituted or unsubstituted aryl group; or a substituted or unsubstituted heteroaryl group, or are bonded to an adjacent group to form a substituted or unsubstituted ring.

According to an exemplary embodiment of the present specification, X'1 to X'6 are N.

According to an exemplary embodiment of the present specification, R309 to R314 are a cyano group.

According to an exemplary embodiment of the present specification, Chemical Formula HI-1 is represented by the following compound.

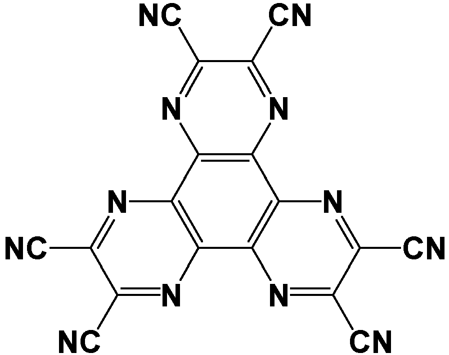

The hole transport layer is a layer which accepts holes from a hole injection layer and transports the holes to a light emitting layer, and a hole transport material is suitably a material having high hole mobility which may accept holes from a first electrode or a hole injection layer and transfer the holes to a light emitting layer. Specific examples thereof include arylamine-based organic materials, conductive polymers, block copolymers having both conjugated portions and nonconjugated portions, and the like, but are not limited thereto.

In an exemplary embodiment of the present specification, the hole transport layer includes a compound of the following Chemical Formula HT-1.

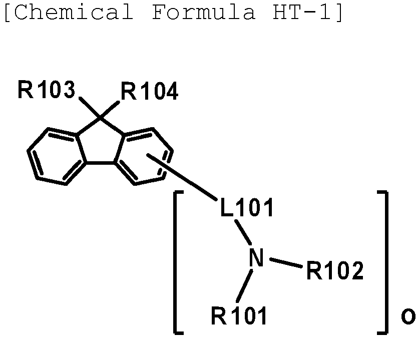

In Chemical Formula HT-1,
L101 is a direct bond; or a substituted or unsubstituted arylene group,
R101 and R102 are the same as or different from each other, and are each independently hydrogen; deuterium; a substituted or unsubstituted alkyl group; a substituted or unsubstituted amine group; a substituted or unsubstituted aryl group; or a substituted or unsubstituted heterocyclic group,
R103 and R104 are the same as or different from each other, and are each independently a substituted or unsubstituted alkyl group; or a substituted or unsubstituted aryl group, and
o is 1 or 2, and when o is 2, structures in the parenthesis are the same as or different from each other.

In an exemplary embodiment of the present specification, L101 is a direct bond; or a substituted or unsubstituted phenylene group; or a substituted or unsubstituted naphthylene group.

In an exemplary embodiment of the present specification, L101 is a direct bond; or a substituted or unsubstituted phenylene group.

In an exemplary embodiment of the present specification, L101 is a direct bond; or a phenylene group.

In an exemplary embodiment of the present specification, R101 and R102 are the same as or different from each other, and are each independently a substituted or unsubstituted aryl group.

In an exemplary embodiment of the present specification, R101 and R102 are the same as or different from each other, and are each independently a substituted or unsubstituted monocyclic aryl group; or a substituted or unsubstituted polycyclic aryl group.

In an exemplary embodiment of the present specification, R101 and R102 are the same as or different from each other, and are each independently a substituted or unsubstituted phenyl group; a substituted or unsubstituted biphenyl group; a substituted or unsubstituted terphenyl group; a substituted or unsubstituted naphthyl group; a substituted or unsubstituted anthracenyl group; a substituted or unsubstituted phenanthrene group; a substituted or unsubstituted triphenylene group; a substituted or unsubstituted pyrene group; or a substituted or unsubstituted fluorenyl group.

In an exemplary embodiment of the present specification, R101 and R102 are the same as or different from each other, and are each independently a substituted or unsubstituted phenyl group; a substituted or unsubstituted biphenyl group; or a substituted or unsubstituted fluorenyl group.

In an exemplary embodiment of the present specification, R101 and R102 are the same as or different from each other, and are each independently a phenyl group; a biphenyl group; or a fluorenyl group substituted with an alkyl group.

In an exemplary embodiment of the present specification, R103 and R104 are the same as or different from each other, and are each independently a substituted or unsubstituted alkyl group.

In an exemplary embodiment of the present specification, R103 and R104 are the same as or different from each other, and are each independently an alkyl group.

In an exemplary embodiment of the present specification, R103 and R104 are a methyl group.

In an exemplary embodiment of the present specification, Chemical Formula HT-1 is represented by the following compound.

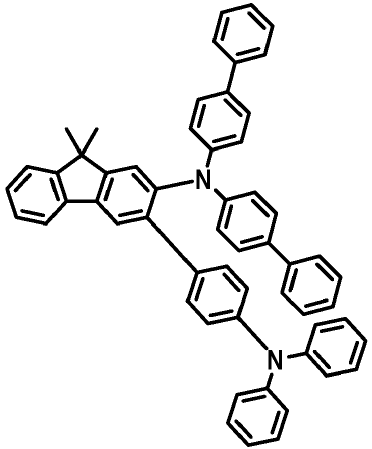

In an exemplary embodiment of the present specification, a hole adjusting layer may be provided between the hole transport layer and the light emitting layer. For the hole adjusting layer, materials known in the art may be used.

According to an exemplary embodiment of the present specification, the hole adjusting layer includes a compound represented by the following Chemical Formula EB-1, but is not limited thereto.

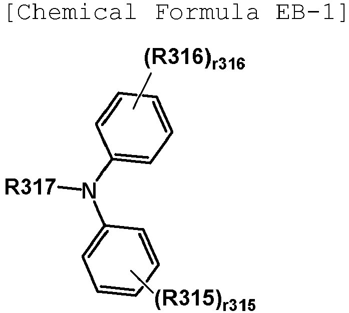

In Chemical Formula EB-1,
R315 to R317 are the same as or different from each other, and are each independently any one selected from the group consisting of hydrogen; deuterium; a substituted or unsubstituted alkyl group; a substituted or unsubstituted aryl group; a substituted or unsubstituted heteroaryl group; and a combination thereof, or are bonded to an adjacent group to form a substituted or unsubstituted ring,
r315 is an integer from 1 to 5, and when r315 is 2 or higher, two or more R315's are the same as or different from each other, and
r316 is an integer from 1 to 5, and when r316 is 2 or higher, two or more R316's are the same as or different from each other.

According to an exemplary embodiment of the present specification, R317 is any one selected from the group consisting of a substituted or unsubstituted aryl group; a substituted or unsubstituted heteroaryl group; and a combination thereof.

According to an exemplary embodiment of the present specification, R317 is any one selected from the group consisting of a phenyl group; a biphenyl group; and a combination thereof.

According to an exemplary embodiment of the present specification, R315 and R316 are the same as or different from each other, and are each independently a substituted or unsubstituted aryl group.

According to an exemplary embodiment of the present specification, R315 and R316 are each independently a substituted or unsubstituted polycyclic aryl group.

According to an exemplary embodiment of the present specification, R315 and R316 are each independently a substituted or unsubstituted phenanthrenyl group.

According to an exemplary embodiment of the present specification, R315 and R316 are a phenanthrenyl group.

According to an exemplary embodiment of the present specification, Chemical Formula EB-1 is represented by the following compound.

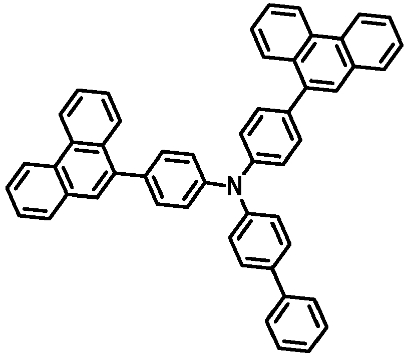

In an exemplary embodiment of the present specification, the light emitting layer includes a host and a dopant.

Examples of a host material for the light emitting layer include fused aromatic ring derivatives, or hetero ring-containing compounds, and the like. Specifically, examples of the fused aromatic ring derivative include anthracene derivatives, pyrene derivatives, naphthalene derivatives, pentacene derivatives, phenanthrene compounds, fluoranthene compounds, and the like, and examples of the hetero ring-containing compound include carbazole derivatives, dibenzofuran, dibenzofuran derivatives, dibenzothiophene, dibenzothiophene derivatives, ladder-type furan compounds, pyrimidine derivatives, and the like, but the examples thereof are not limited thereto.

For the dopant for the light emitting layer, when the light emitting layer emits red light, it is possible to use a phosphorescent material such as bis(1-phenylisoquinoline)acetylacetonate iridium (PIQIr(acac)), bis(1-phenylquinoline)acetylacetonate iridium (PQIr(acac)), tris(1-phenylquinoline)iridium (PQIr), or octaethylporphyrin platinum (PtOEP), or a fluorescent material such as tris(8-hydroxyquinolino)aluminum (Alq₃) as a light emitting dopant, but the light emitting dopant is not limited thereto. When the light emitting layer emits green light, it is possible to use a phosphorescent material such as fac tris(2-phenylpyridine)iridium (Ir(ppy)₃), or a fluorescent material such as tris(8-hydroxyquinolino)aluminum (Alq₃), as the light emitting dopant, but the light emitting dopant is not limited thereto. When the light emitting layer emits blue light, phosphorescent materials such as (4,6-F2ppy)₂Irpic, or fluorescent materials such as spiro-DPVBi, spiro-6P, distyrylbenzene (DSB), distyrylarylene (DSA), PFO-based polymers or PPV-based polymers may be used as the light emitting dopant, however, the light emitting dopant is not limited thereto.

In an exemplary embodiment of the present specification, the host includes one or more compounds of Chemical Formula 1 of the present application.

In an exemplary embodiment of the present specification, the host includes a first anthracene derivative substituted with deuterium and a second anthracene derivative unsubstituted with deuterium, and any one of the first anthracene derivative and the second anthracene derivative includes the compound of Chemical Formula 1 of the present application.

In an exemplary embodiment of the present specification, when the host of the light emitting layer includes a first anthracene derivative and a second anthracene derivative, the mass ratio of the first anthracene derivative and the second anthracene derivative may be 99:1 to 1:99, 90:10 to 10:90, 80:20 to 20:80, 70:30 to 30:70, or 60:40 to 40:60.

In an exemplary embodiment of the present specification, the first anthracene derivative substituted with deuterium includes the compound of Chemical Formula 1 of the present application.

In an exemplary embodiment of the present specification, the dopant used with the compound of Chemical Formula 1 of the present application is a fluorescent dopant.

In an exemplary embodiment of the present specification, the dopant used with the compound of Chemical Formula 1 of the present application includes a pyrene-based compound or a non-pyrene-based compound.

In an exemplary embodiment of the present specification, the non-pyrene-based compound used with the compound of Chemical Formula 1 of the present application includes a boron-based compound.

In an exemplary embodiment of the present specification, as the pyrene-based compound, it is possible to apply those selected from among compounds exemplified in Japanese Patent No. 4205059 (JP 4205059 B2), Japanese Patent No. 4267623 (JP 4267623 B2), Japanese Patent No. 4188401 (JP 4188401 B2), Japanese Patent No. 4848134 (JP 4848134 B2), Japanese Patent No. 6082179 (JP 6082179 B2) and Japanese Patent No. 5587302 (JP 5587302 B2).

In an exemplary embodiment of the present specification, the pyrene-based compound used with the compound of Chemical Formula 1 of the present application may be a compound of the following Chemical Formula D1.

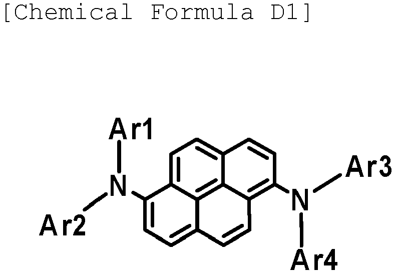

In Chemical Formula D1, Ar1 to Ar4 are the same as or different from each other, and are each independently a substituted or unsubstituted aryl group, or a substituted or unsubstituted heterocyclic group.

In an exemplary embodiment of the present specification, Ar1 and Ar3 are the same as or different from each other, and are each independently a substituted or unsubstituted aryl group.

In an exemplary embodiment of the present specification, Ar1 and Ar3 are the same as or different from each other, and are each independently an aryl group unsubstituted or substituted with an alkyl group.

In an exemplary embodiment of the present specification, Ar1 and Ar3 are the same as or different from each other, and are each independently a phenyl group unsubstituted or substituted with a methyl group.

In an exemplary embodiment of the present specification, Ar2 and Ar4 are the same as or different from each other, and are each independently a substituted or unsubstituted heterocyclic group.

In an exemplary embodiment of the present specification, Ar2 and Ar4 are the same as or different from each other, and are each independently a substituted or unsubstituted dibenzofuranyl group.

In an exemplary embodiment of the present specification, Ar2 and Ar4 may be a dibenzofuranyl group.

In an exemplary embodiment of the present specification, Ar1 and Ar3 may be the same as each other.

In an exemplary embodiment of the present specification, Ar2 and Ar4 may be the same as each other.

In an exemplary embodiment of the present specification, the compound of Chemical Formula D1 may be the following compound.

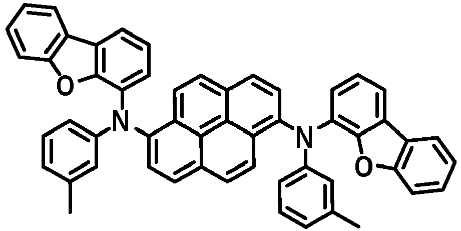

In an exemplary embodiment of the present specification, as the boron-based compound, it is possible to apply those selected from among compounds exemplified in Japanese Patent No. 5935199 (JP 5935199 B2), International Publication No. WO2017-138526A1, Japanese Patent No. 6611825 (JP 6611825 B2), International Publication No. WO2018-047639A1, International Publication No. WO2018-110497A1 and Japanese Patent Laid-Open Publication No. 2020-097561 (JP 2020-097561 A).

In an exemplary embodiment of the present specification, the pyrene-based compound used with the compound of Chemical Formula 1 of the present application may be a compound of the following Chemical Formula D2.

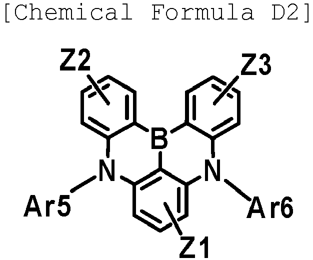

In Chemical Formula D2,
Ar5 and Ar6 are the same as or different from each other, and are each independently a substituted or unsubstituted aryl group, or a substituted or unsubstituted heterocyclic group,
Z1 to Z3 are hydrogen, deuterium, a halogen group, a cyano group, a nitro group, a hydroxyl group, a substituted or unsubstituted silyl group, a substituted or unsubstituted amine group, a substituted or unsubstituted alkyl group, a substituted or unsubstituted cycloalkyl group, a substituted or unsubstituted aryl group, or a substituted or unsubstituted heterocyclic group, or adjacent groups are bonded to each other to form a substituted or unsubstituted ring.

In an exemplary embodiment of the present specification, Ar5 and Ar6 are the same as or different from each other, and are each independently a substituted or unsubstituted aryl group.

In an exemplary embodiment of the present specification, Ar5 and Ar6 are the same as or different from each other, and are each independently an aryl group unsubstituted or substituted with an alkyl group.

In an exemplary embodiment of the present specification, Ar5 and Ar6 are the same as or different from each other, and are each independently a phenyl group unsubstituted or substituted with a methyl group.

In an exemplary embodiment of the present specification, Z1 to Z3 are the same as or different from each other, and are each independently a substituted or unsubstituted alkyl group.

In an exemplary embodiment of the present specification, Z1 to Z3 are a methyl group.

In an exemplary embodiment of the present specification, the compound of Chemical Formula D2 may be the following compound.

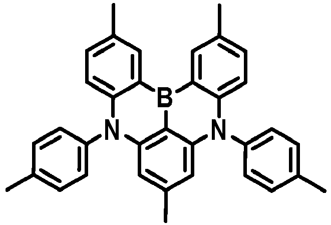

In an exemplary embodiment of the present specification, the weight ratio of the host and the dopant in the light emitting layer may be 50:50, 60:40, 70:30, 80:20, 90:10, 91:9, 92:8, 93:7, 94:6, 95:5, 96:4, 97:3, 98:2, or 99:1.

In an exemplary embodiment of the present specification, the compound is a host, specifically a fluorescent host, and more specifically a blue fluorescent host.

In an exemplary embodiment of the present specification, the maximum emission peak (λₘₐₓ) of the light emitting layer including the compound represented by Chemical Formula 1 is 400 nm to 470 nm.

In an exemplary embodiment of the present specification, the light emitting layer includes a host and a dopant at a mass ratio of 99:1 to 1:99.

In an exemplary embodiment of the present specification, the light emitting layer includes a host and a dopant at a mass ratio of 99:1 to 10:90.

In an exemplary embodiment of the present specification, the light emitting layer includes a host and a dopant at a mass ratio of 99:1 to 50:50.

In an exemplary embodiment of the present specification, an electron adjusting layer may be provided between the electron transport layer and the light emitting layer. The electron adjusting layer is a layer blocking holes from reaching a cathode, and may be generally formed under the same condition as the hole injection layer. Specific examples thereof include oxadiazole derivatives, triazole derivatives, phenanthroline derivatives, BCP, aluminum complexes and the like, but are not limited thereto.

In an exemplary embodiment of the present specification, the electron adjusting layer includes a compound of the following Chemical Formula HB-1.

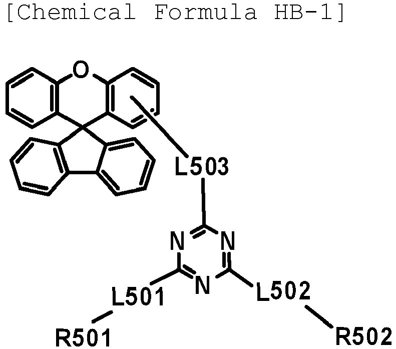

In Chemical Formula HB-1,
L501 to L503 are the same as or different from each other, and are each independently a direct bond; or a substituted or unsubstituted arylene group, and
R501 and R502 are the same as or different from each other, and are each independently hydrogen; deuterium; a substituted or unsubstituted alkyl group; a substituted or unsubstituted amine group; a substituted or unsubstituted aryl group; or a substituted or unsubstituted heterocyclic group.

In an exemplary embodiment of the present specification, L501 to L503 are the same as or different from each other, and are each independently a direct bond; a substituted or unsubstituted phenylene group; or a substituted or unsubstituted naphthylene group.

In an exemplary embodiment of the present specification, L501 to L503 are the same as or different from each other, and are each independently a direct bond; or a substituted or unsubstituted phenylene group.

In an exemplary embodiment of the present specification, L501 to L503 are the same as or different from each other, and are each independently a direct bond; or a phenylene group.

In an exemplary embodiment of the present specification, R501 and R502 are the same as or different from each other, and are each independently a substituted or unsubstituted aryl group.

In an exemplary embodiment of the present specification, R501 and R502 are the same as or different from each other, and are each independently a substituted or unsubstituted monocyclic aryl group; or a substituted or unsubstituted polycyclic aryl group.

In an exemplary embodiment of the present specification, R501 and R502 are the same as or different from each other, and are each independently a substituted or unsubstituted phenyl group; a substituted or unsubstituted biphenyl group; a substituted or unsubstituted terphenyl group; a substituted or unsubstituted naphthyl group; a substituted or unsubstituted anthracenyl group; a substituted or unsubstituted phenanthrene group; a substituted or unsubstituted triphenylene group; a substituted or unsubstituted pyrene group; or a substituted or unsubstituted fluorenyl group.

In an exemplary embodiment of the present specification, R501 and R502 are the same as or different from each other, and are each independently a substituted or unsubstituted phenyl group; or a substituted or unsubstituted naphthyl group.

In an exemplary embodiment of the present specification, R501 and R502 are the same as or different from each other, and are each independently a phenyl group; or a naphthyl group.

In an exemplary embodiment of the present specification, Chemical Formula HB-1 is represented by the following compound.

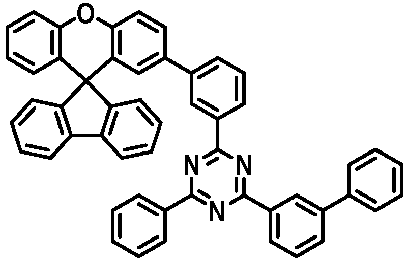

In an exemplary embodiment of the present specification, the electron transport layer is a layer which accepts electrons from an electron injection layer and transports the electrons to a light emitting layer, and an electron transport material is suitably a material having high electron mobility which may proficiently accept electrons from a second electrode and transfer the electrons to a light emitting layer. Specific examples thereof include: Al complexes of 8-hydroxyquinoline; complexes including Alq3; organic radical compounds; hydroxyflavone-metal complexes, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

In an exemplary embodiment of the present specification, the electron transport layer includes a compound of the following Chemical Formula ET-1.

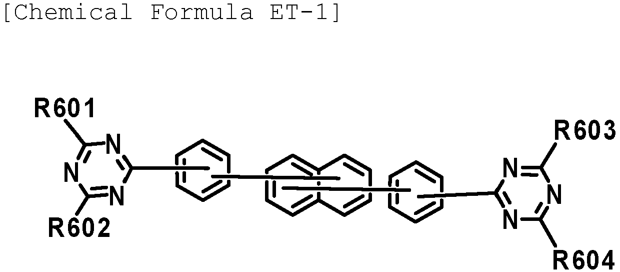

In Chemical Formula ET-1,
R601 to R604 are the same as or different from each other, and are each independently hydrogen; deuterium; a substituted or unsubstituted alkyl group; a substituted or unsubstituted aryl group; or a substituted or unsubstituted heterocyclic group.

In an exemplary embodiment of the present specification, R601 to R604 are the same as or different from each other, and are each independently a substituted or unsubstituted aryl group; or a substituted or unsubstituted heterocyclic group.

In an exemplary embodiment of the present specification, R601 to R604 are the same as or different from each other, and are each independently a substituted or unsubstituted aryl group.

In an exemplary embodiment of the present specification, R601 to R604 are the same as or different from each other, and are each independently a substituted or unsubstituted aryl group having 6 to 30 carbon atoms.

In an exemplary embodiment of the present specification, R601 to R604 are the same as or different from each other, and are each independently a substituted or unsubstituted aryl group having 6 to 20 carbon atoms.

In an exemplary embodiment of the present specification, R601 to R604 are the same as or different from each other, and are each independently a substituted or unsubstituted aryl group having 6 to 10 carbon atoms.

In an exemplary embodiment of the present specification, R601 to R604 are a phenyl group.

In an exemplary embodiment of the present specification, the compound of Chemical Formula ET-1 is represented by the following compound.

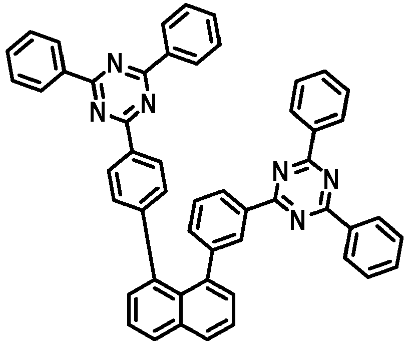

In an exemplary embodiment of the present specification, the electron transport layer may include a lithium complex together with the compound of Chemical ET-1.

The electron injection layer is a layer which injects electrons from an electrode, and an electron injection material is preferably a compound which has a capability of transporting electrons, an effect of injecting electrons from a second electrode, and an excellent effect of injecting electrons into a light emitting layer or a light emitting material, prevents excitons produced from a light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

The metal complex compound includes 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato) (o-cresolato)gallium, bis(2-methyl-8-quinolinato) (1-naphtholato)aluminum, bis(2-methyl-8-quinolinato) (2-naphtholato)gallium and the like, but is not limited thereto.

In an exemplary embodiment of the present specification, a capping layer may be formed on the cathode using an organic material. For example, the capping layer may include a carbazole-based compound.

In an exemplary embodiment of the present specification, the capping layer includes a compound of the following Chemical Formula CL-1.

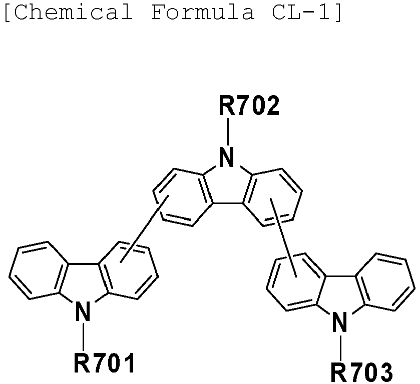

In Chemical Formula CL-1,
R701 to R703 are the same as or different from each other, and are each independently hydrogen; deuterium; a substituted or unsubstituted alkyl group; a substituted or unsubstituted aryl group; or a substituted or unsubstituted heterocyclic group.

In an exemplary embodiment of the present specification, R701 to R703 are the same as or different from each other, and are each independently a substituted or unsubstituted aryl group; or a substituted or unsubstituted heterocyclic group.

In an exemplary embodiment of the present specification, R701 to R703 are the same as or different from each other, and are each independently a substituted or unsubstituted aryl group.

In an exemplary embodiment of the present specification, R701 to R703 are the same as or different from each other, and are each independently a substituted or unsubstituted aryl group having 6 to 30 carbon atoms.

In an exemplary embodiment of the present specification, R701 to R703 are the same as or different from each other, and are each independently a substituted or unsubstituted aryl group having 6 to 20 carbon atoms.

In an exemplary embodiment of the present specification, R701 to R703 are the same as or different from each other, and are each independently a substituted or unsubstituted aryl group having 6 to 10 carbon atoms.

In an exemplary embodiment of the present specification, R701 to R703 are a phenyl group.

In an exemplary embodiment of the present specification, the compound of Chemical Formula CL-1 is represented by the following compound.

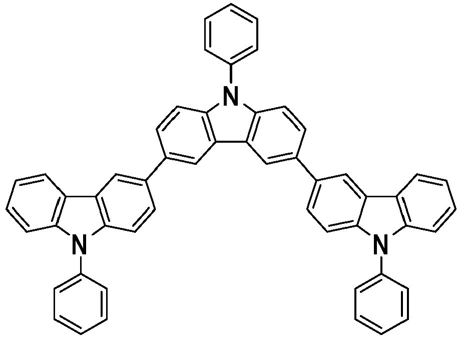

The organic light emitting device according to the present specification may be a top-emission type, a bottom-emission type or a dual-emission type depending on the materials used.

The organic light emitting device according to the present specification may be included and used in various electronic devices. For example, the electronic device may be a display panel, a touch panel, a solar module, a lighting device, and the like, and is not limited thereto.

A core structure may be prepared, as in the reaction schemes of the following Preparation Examples 1 to 7, from the compound represented by Chemical Formula 1 of the present specification. The substituent may be bonded by a method known in the art, and the type and position of the substituent and the number of substituents may be changed according to the technology known in the art.

### [Mode for Invention]

Hereinafter, the present specification will be described in more detail through Examples. However, the following Examples are provided only for exemplifying the present specification, but are not intended to limit the present specification.

### Preparation Example 1 (Synthesis of A1 to A8)

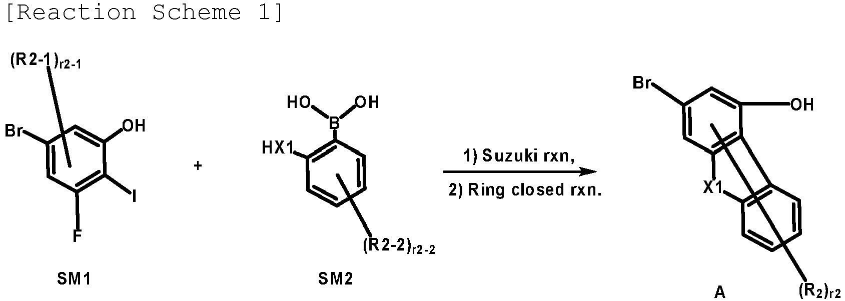

Here, R2, r2 and X1 are the same as the definitions in Chemical Formula 1, and

R2-1 and R2-2 are the same as the definition of R2, r2-1 is 1 or 2, and r2-2 is an integer from 1 to 4.

After SM1 (1 eq) and SM2 (1.05 eq) were added to excess tetrahydrofuran (THF), a 2 M aqueous potassium carbonate solution (30 volume ratio compared to THF) was added thereto, and tetrakis triphenyl-phosphino palladium (2 mol%) was added thereto, and then the resulting mixture was heated and stirred for 10 hours. After the temperature was lowered to room temperature and the reaction was terminated, the aqueous potassium carbonate solution was removed to separate the layers. After the solvent was removed, dimethylacetamide (DMAc, excess) and potassium carbonate (3 eq) were added thereto, and the resulting mixture was stirred and heated under reflux for 5 hours. After the completion of the reaction was confirmed, the temperature was lowered to room temperature, water (1.5-fold volume ratio compared to DMAc) was added thereto, and a solid obtained by filtering the resulting mixture was purified by column chromatography using methylene chloride and hexane to prepare Intermediate A.

Intermediates A1 to A8 were synthesized in the same manner as in the method of synthesizing Chemical Formula A, except that SM1 and SM2 were changed as shown in the following Table A.

**[Table A]**

| | SM1 | SM2 | Intermediate A | Yield | MS[M+H]⁺ |
|---|---|---|---|---|---|
| A1 | 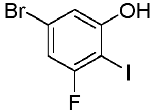 | 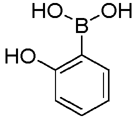 | 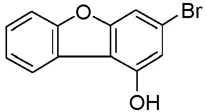 | 53% | 264.09 |
| A2 | 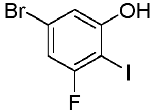 | 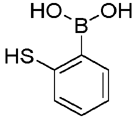 | 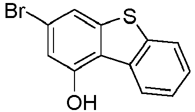 | 45% | 280.15 |
| A3 | 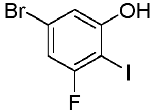 | 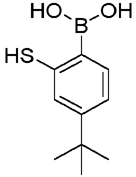 | 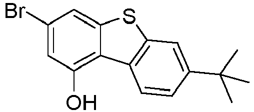 | 48% | 336.26 |
| A4 | 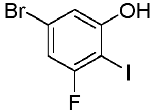 | 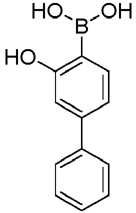 | 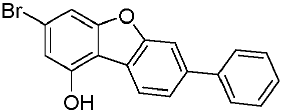 | 52% | 340.19 |
| A5 | 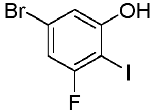 | 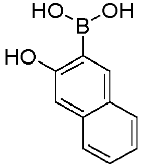 | 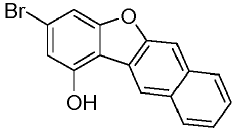 | 50% | 314.15 |
| A6 | 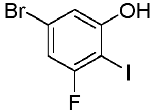 | 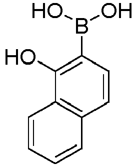 | 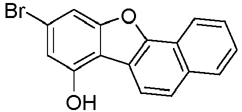 | 53% | 314.15 |
| A7 | 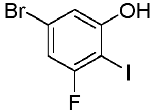 | 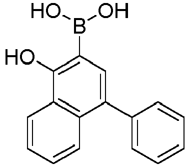 | 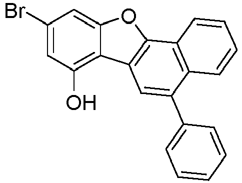 | 49% | 390.25 |
| A8 | 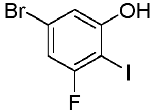 | 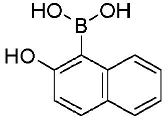 | 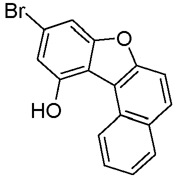 | 39% | 314.15 |

### Preparation Example 2

### Preparation Example 2-1 (Synthesis of B1 to B32)

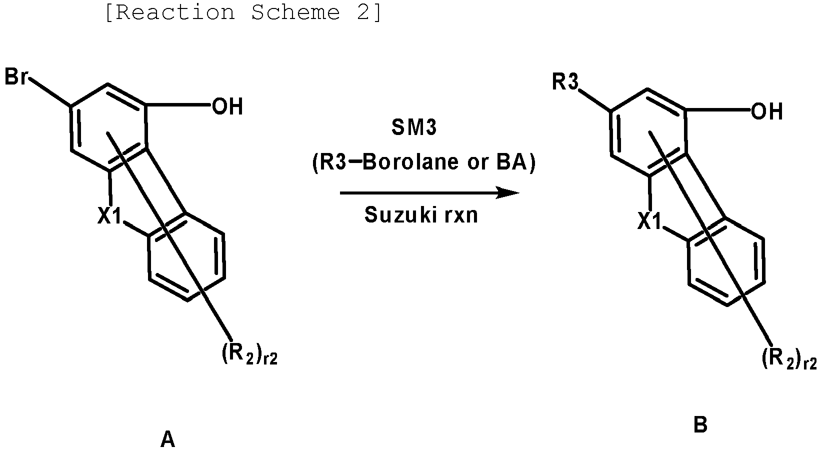

Here, R2, R3, r2 and X1 are the same as the definitions in Chemical Formula 1.

After Intermediate A (1 eq) synthesized in Preparation Example 1 and SM3 (1.05 eq) were added to excess tetrahydrofuran, a 2 M aqueous potassium carbonate solution (30 volume ratio compared to THF) was added thereto, and tetrakis triphenyl-phosphino palladium (2 mol%) was added thereto, and then the resulting mixture was heated and stirred for 10 hours. After the temperature was lowered to room temperature and the reaction was terminated, the aqueous potassium carbonate solution was removed to separate the layers. Thereafter, the solvent was removed and the residue was purified by recrystallization using chloroform and hexane to prepare Intermediate B.

B1 to B32 in [Table B] were synthesized in the same manner as in the method of synthesizing Intermediate B, except A and SM3 were changed as shown in the following Table B.

**[Table B]**

| | A | SM3 | Intermediate B | Yield | MS [M+H]⁺ |
|---|---|---|---|---|---|
| B1 | A1 | 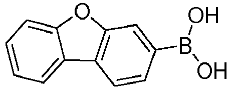 | 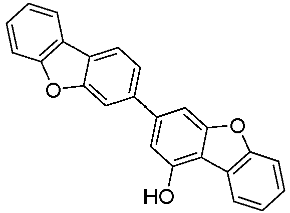 | 80% | 351.37 |
| B2 | A1 | 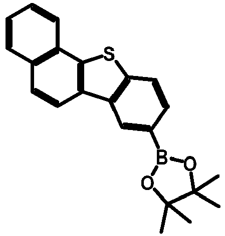 | 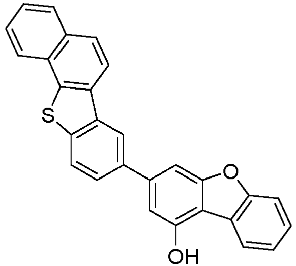 | 79% | 417.49 |
| B3 | A1 | 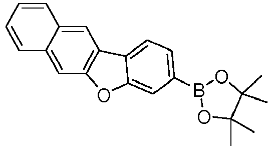 | 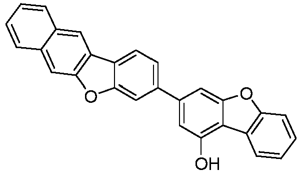 | 82% | 401.43 |
| B4 | A1 | 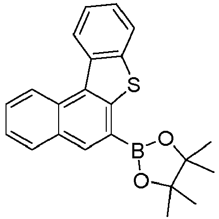 | 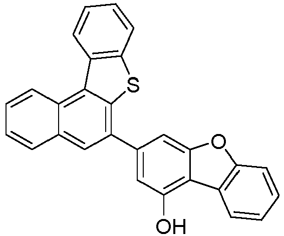 | 85% | 417.49 |
| B5 | A1 | 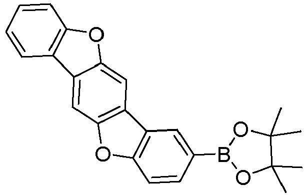 | 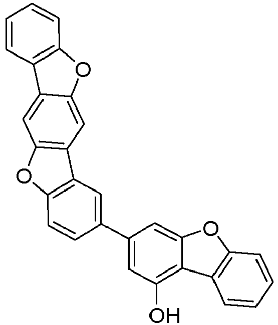 | 84% | 441.45 |
| B6 | A2 | 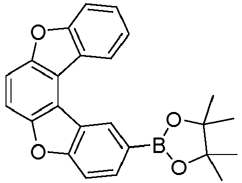 | 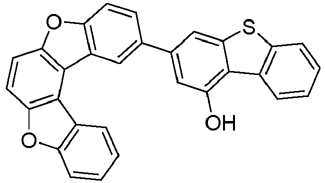 | 80% | 457.52 |
| B7 | A1 | 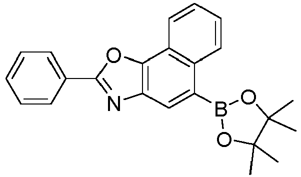 | 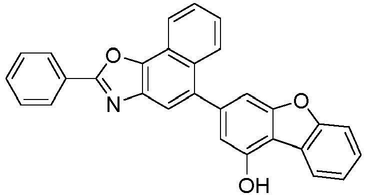 | 79% | 428.46 |
| B8 | A1 | 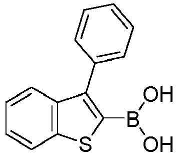 | 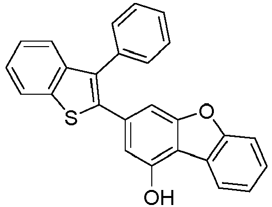 | 81% | 393.47 |
| B9 | A1 | 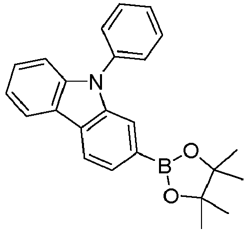 | 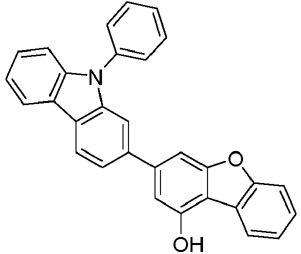 | 80% | 426.49 |
| B10 | A1 | 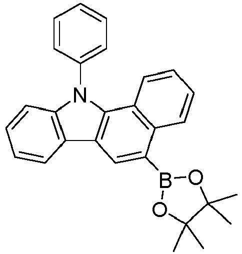 | 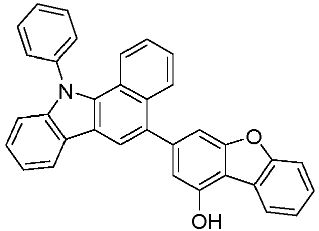 | 82% | 476.55 |
| B11 | A1 | 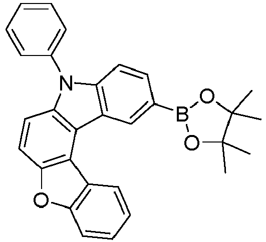 | 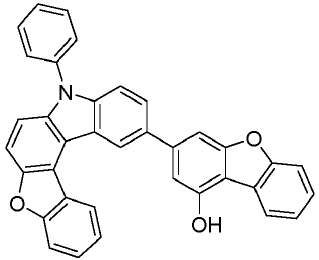 | 79% | 516.57 |
| B12 | A1 | 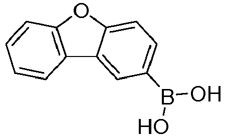 | 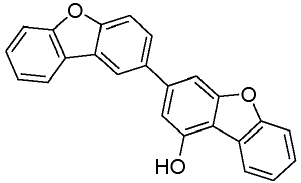 | 83% | 351.37 |
| B13 | A1 | 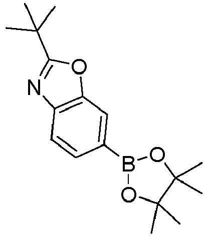 | 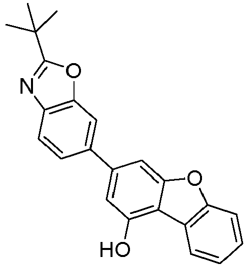 | 80% | 358.41 |
| B14 | A1 | 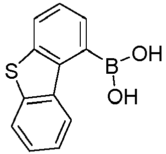 | 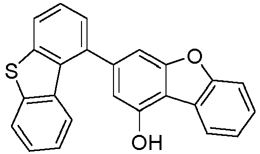 | 84% | 367.43 |
| B15 | A1 | 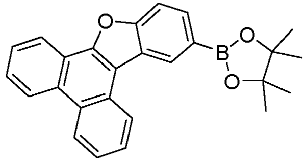 | 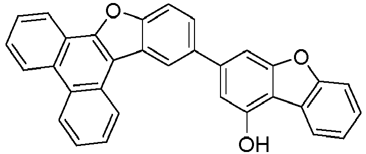 | 80% | 450.49 |
| B16 | A1 | 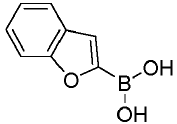 | 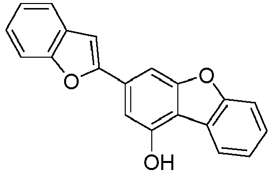 | 83% | 301.31 |
| B17 | A1 | 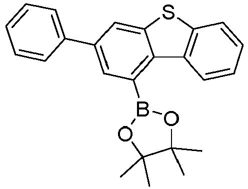 | 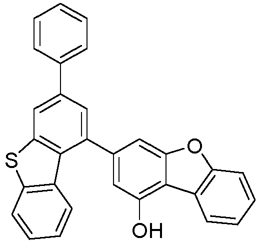 | 82% | 443.53 |
| B18 | A1 | 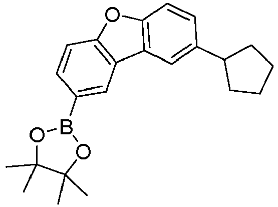 | 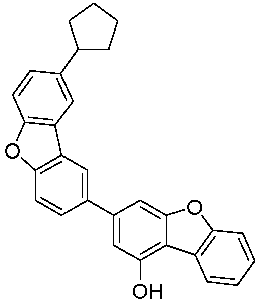 | 78% | 419.49 |
| B19 | A1 | 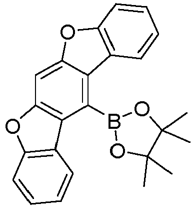 | 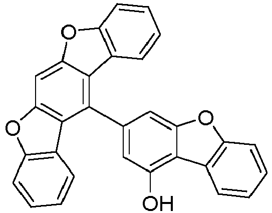 | 77% | 441.45 |
| B20 | A1 | 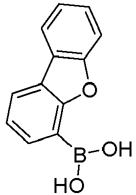 | 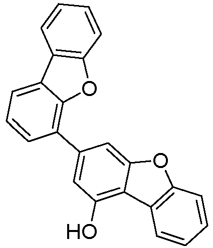 | 80% | 351.37 |
| B21 | A1 | 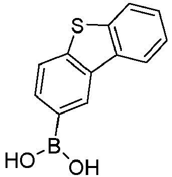 | 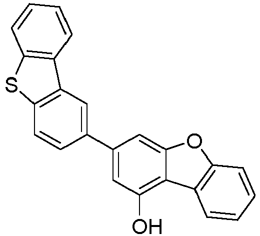 | 83% | 367.43 |
| B22 | A1 | 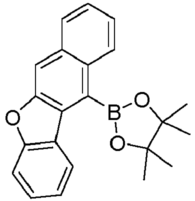 | 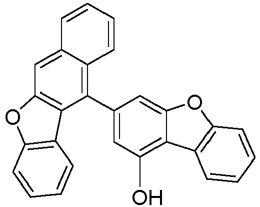 | 80% | 401.43 |
| B23 | A3 | 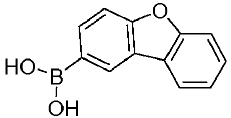 | 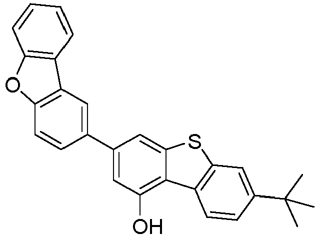 | 73% | 423.54 |
| B24 | A4 | 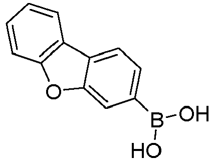 | 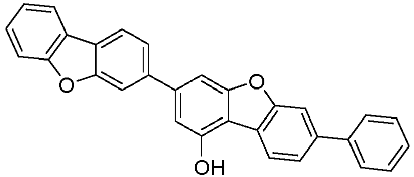 | 76% | 427.47 |
| B25 | A5 | 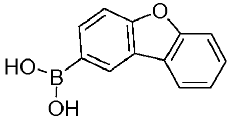 | 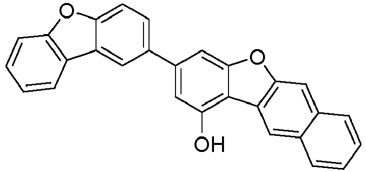 | 80% | 401.43 |
| B26 | A6 | 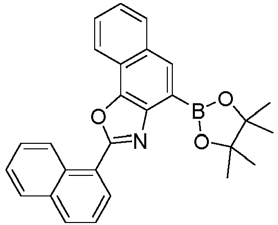 | 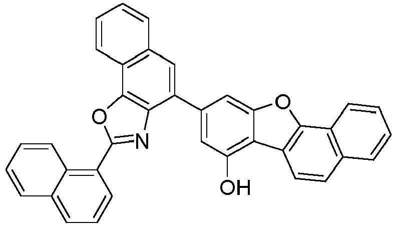 | 71% | 528.58 |
| B27 | A6 | 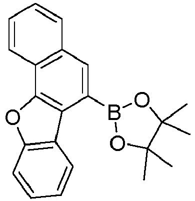 | 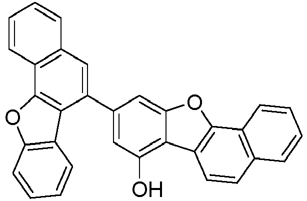 | 80% | 451.49 |
| B28 | A7 | 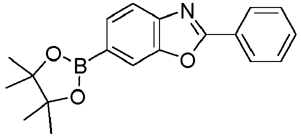 | 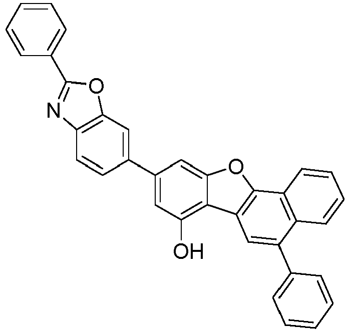 | 73% | 504.56 |
| B29 | A8 | 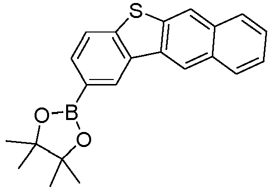 | 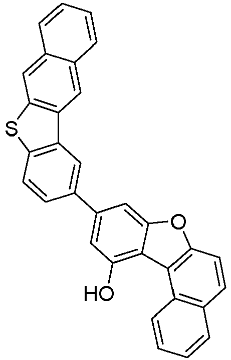 | 79% | 467.55 |
| B30 | A1 | 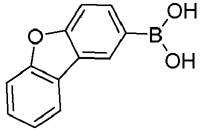 | 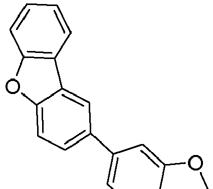 | 73% | 351.37 |
| B31 | A2 | 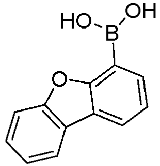 | 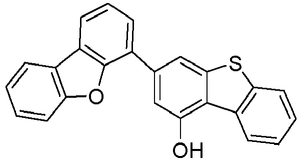 | 75% | 367.43 |
| B32 | A1 | 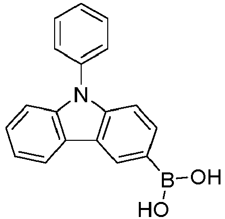 | 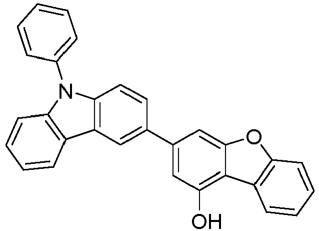 | 71% | 426.49 |

### Preparation Example 2-2 (Synthesis of Compound B33)

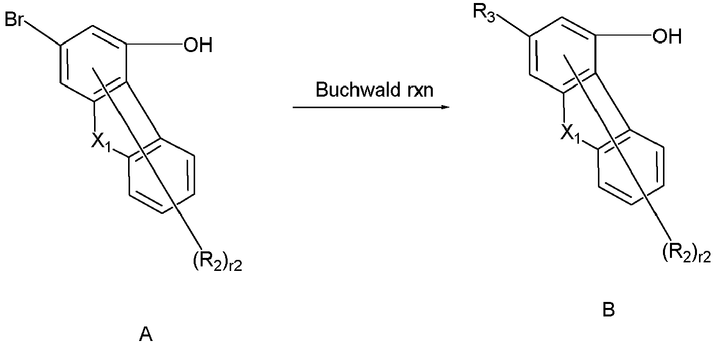

Here, R2, R3, r2 and X1 are the same as the definitions in Chemical Formula 1.

Intermediate A (1 eq) synthesized in Preparation Example 1, SM3 (1.05 eq) and sodium t-butoxide (1.5 eq) were put into toluene, the resulting mixture was heated and stirred, and then refluxed, and [1,1'-bis(diphenylphosphino)ferrocene]dichloropalladium (658 mg. 1 mol%) was added thereto. The temperature was lowered to room temperature, the reaction was terminated, and then the resulting product was recrystallized using tetrahydrofuran and ethyl acetate to prepare B33 in [Table B-2].

**[Table B-2]**

| | A | SM3 | Intermediate B | Yield | MS [M+H]+ |
|---|---|---|---|---|---|
| B33 | A1 | 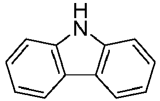 | 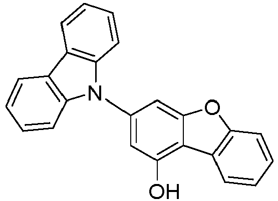 | 80% | 350.39 |

### Preparation Example 3 (Synthesis of Compounds C1 to C33)

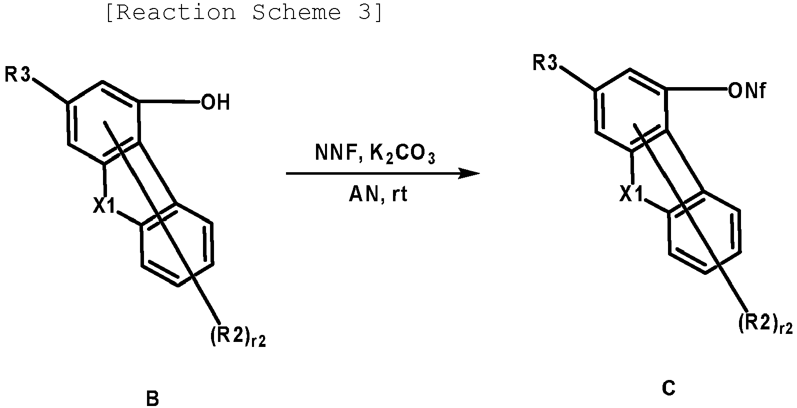

Here, R2, R3, r2 and X1 are the same as the definitions in Chemical Formula 1, and Nf is a nonafluorobutyl group.

After Intermediate B (1 eq) synthesized in Preparation Example 2 and potassium carbonate (2.3 eq) were added to excess acetonitrile (AN), 2 eq of perfluorobutanesulfonic acid (NNF) was added dropwise thereto under stirring at room temperature (RT). After the completion of the reaction was confirmed 5 hours later, water was added thereto, then the resulting mixture was stirred and filtered, and then a solid was extracted with chloroform (excess), and then purified by recrystallization using chloroform and ethanol to prepare Intermediate C.

Intermediates C1 to C33 in the following [Table C] were synthesized in the same manner as in the method of synthesizing Intermediate C, except that B was changed as shown in the following Table C.

**[Table C]**

| | B | Intermediate C | Yield | MS[M+H]⁺ |
|---|---|---|---|---|
| C1 | B1 | 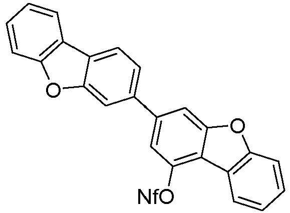 | 90% | 633.45 |
| C2 | B2 | 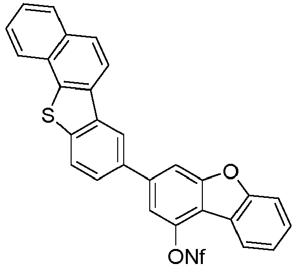 | 89% | 699.57 |
| C3 | B3 | 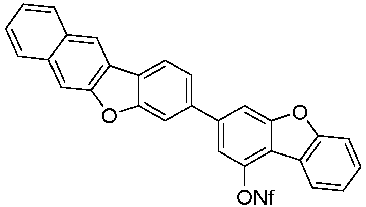 | 92% | 683.51 |
| C4 | B4 | 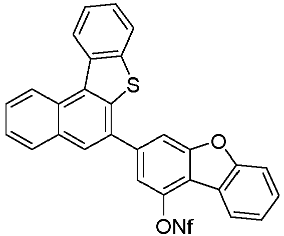 | 91% | 699.57 |
| C5 | B5 | 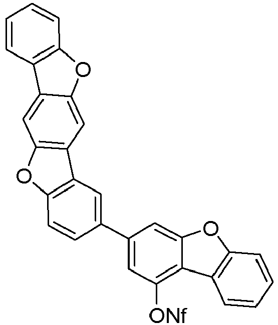 | 93% | 723.53 |
| C6 | B6 | 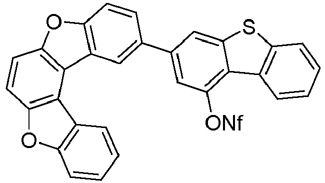 | 89% | 739.59 |
| C7 | B7 | 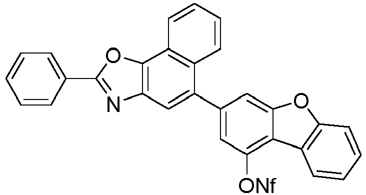 | 88% | 710.54 |
| C8 | B8 | 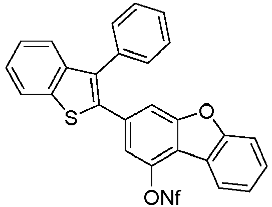 | 85% | 675.55 |
| C9 | B9 | 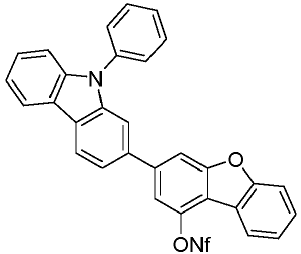 | 90% | 708.57 |
| C10 | B10 | 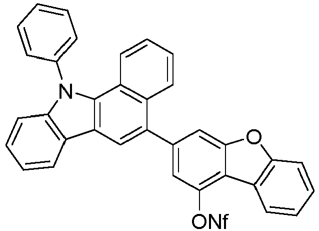 | 81% | 758.63 |
| C11 | B11 | 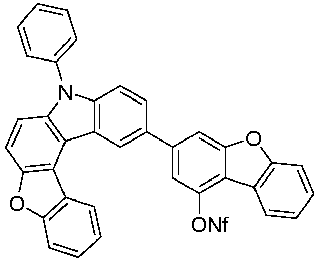 | 90% | 798.65 |
| C12 | B12 | 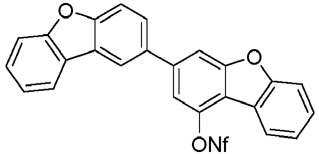 | 86% | 633.45 |
| C13 | B13 | 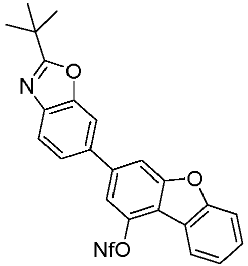 | 88% | 640.49 |
| C14 | B14 | 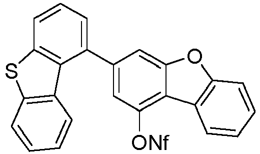 | 87% | 649.51 |
| C15 | B15 | 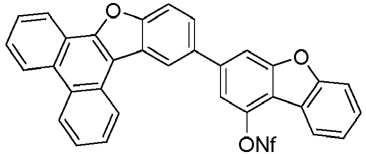 | 89% | 733.57 |
| C16 | B16 | 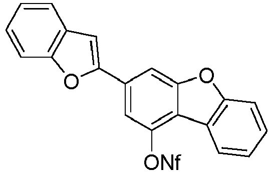 | 80% | 583.39 |
| C17 | B17 | 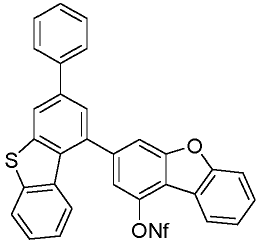 | 88% | 725.61 |
| C18 | B18 | 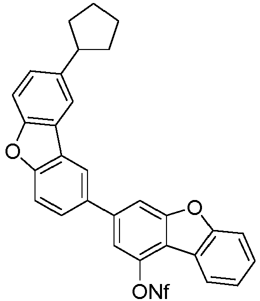 | 83% | 701.57 |
| C19 | B19 | 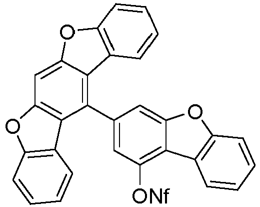 | 81% | 723.53 |
| C20 | B20 | 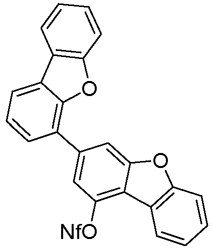 | 83% | 633.45 |
| C21 | B21 | 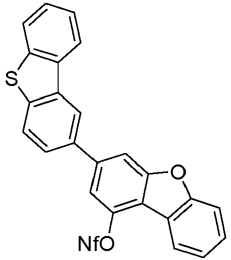 | 85% | 649.51 |
| C22 | B22 | 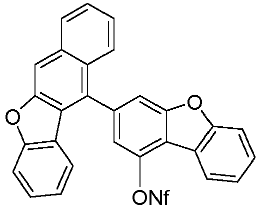 | 88% | 683.51 |
| C23 | B23 | 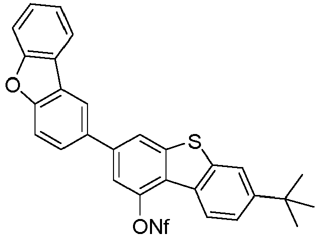 | 90% | 705.62 |
| C24 | B24 | 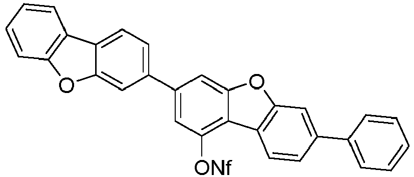 | 81% | 709.55 |
| C25 | B25 | 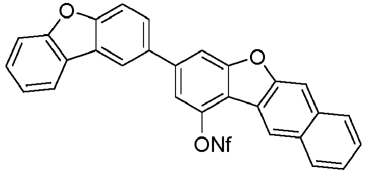 | 88% | 683.51 |
| C26 | B26 | 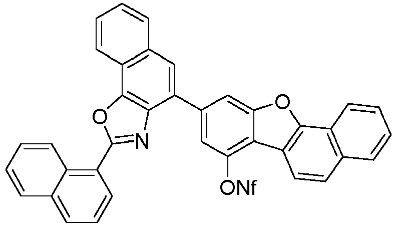 | 83% | 810.66 |
| C27 | B27 | 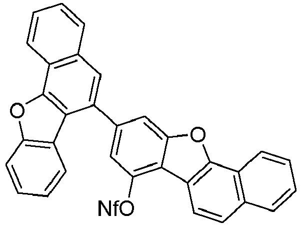 | 86% | 733.57 |
| C28 | B28 | 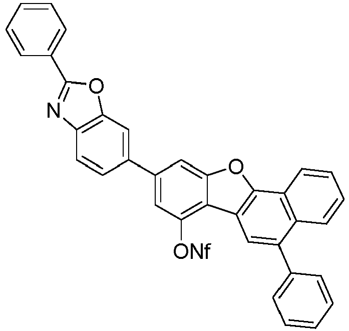 | 85% | 786.64 |
| C29 | B29 | 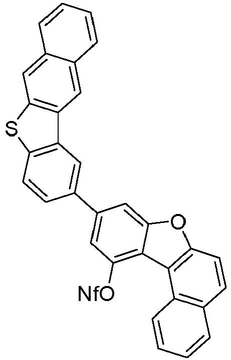 | 84% | 749.63 |
| C30 | B30 | 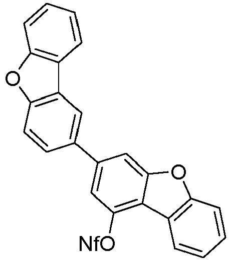 | 85% | 633.45 |
| C31 | B31 | 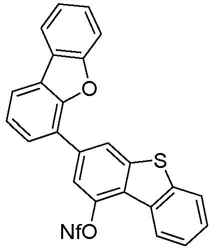 | 88% | 649.51 |
| C32 | B32 | 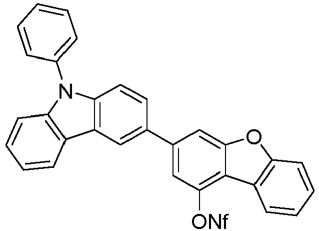 | 83% | 708.57 |
| C33 | B33 | 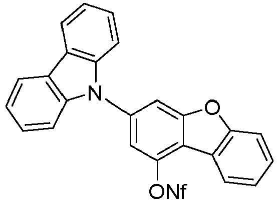 | 84% | 632.47 |

### Preparation Example 4 (Synthesis of Compounds D1 to D2)

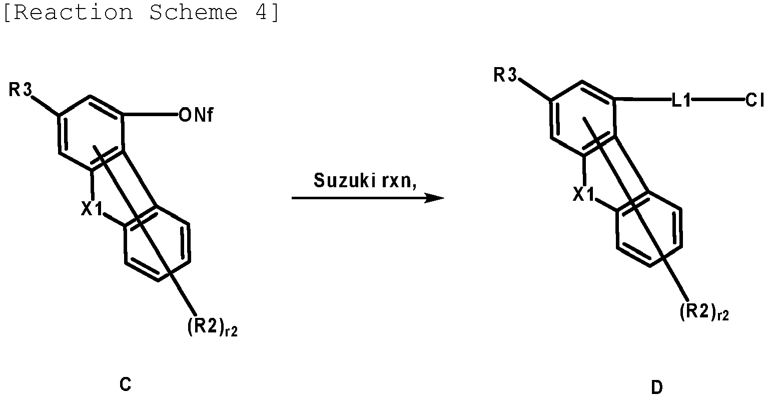

Here, R2, R3, r2, X1 and L1 are the same as the definitions in Chemical Formula 1, and Nf is a nonafluorobutyl group.

After Intermediate C21 or C22 (1 eq) among Intermediates C's synthesized in Preparation Example 3 and SM4 (1.05 eq) were added to tetrahydrofuran (excess), a 2 M aqueous potassium carbonate solution (30 volume ratio compared to THF) was added thereto, and tetrakis triphenyl-phosphino palladium (2 mol%) was added thereto, and then the resulting mixture was heated and stirred for 10 hours. After the temperature was lowered to room temperature and the reaction was terminated, the aqueous potassium carbonate solution was removed to separate the layers. Thereafter, the solvent was removed and the residue was purified by recrystallization using chloroform and ethanol to prepare Intermediate D (D1 and D2) as summarized in the following Table D.

**[Table D]**

| | C | SM4 | Intermediate D | Yield | MS[M+H]⁺ |
|---|---|---|---|---|---|
| D1 | C21 | 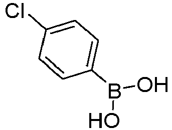 | 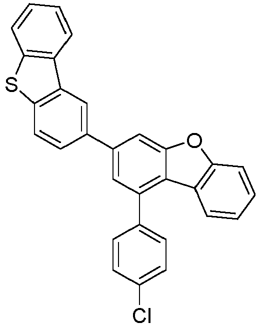 | 80% | 461.98 |
| D2 | C22 | 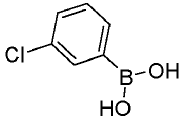 | 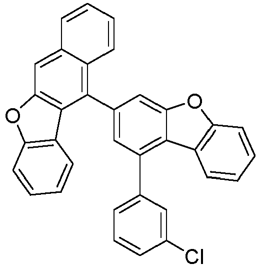 | 79% | 495.97 |

### Preparation Example 5 (Synthesis of Compounds E1 to E33)

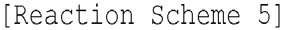

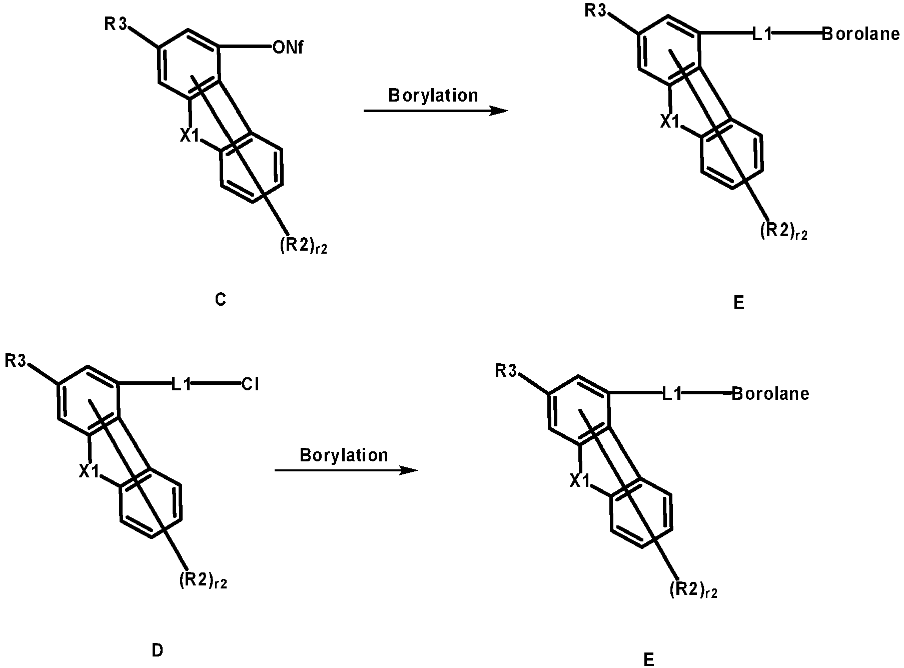

Here, R2, R3, r2, X1 and L1 are the same as the definitions in Chemical Formula 1, and Nf is a nonafluorobutyl group.

SM5 (1 eq) and SM6 (1.3 eq) corresponding to intermediate C or D synthesized in Preparation Example 3 or 4 were put into 1,4-dioxane (12-fold <mass ratio> compared to SM5), potassium acetate (3 eq) was added thereto, and the resulting mixture was stirred and refluxed. After palladium acetate (0.02 eq) and tricyclohexylphosphine (0.04 eq) are stirred in 1,4-dioxane for 5 minutes, the resulting mixture was added thereto, and 2 hours later, the mixture was cooled to room temperature after confirming that the reaction was terminated. After ethanol and water were added thereto, the resulting mixture was filtered and purified by recrystallization with ethyl acetate and ethanol to prepare Intermediate E.

Intermediates E1 to E33 in [Table E] were synthesized in the same manner as in the method of synthesizing Intermediate E, except that SM5 and SM6 were changed as in the following Table E.

**[Table E]**

| | SM5 | SM6 | Intermediate E | Yield | MS[M+H] + |
|---|---|---|---|---|---|
| E1 | C1 | 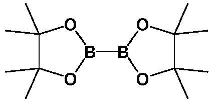 | 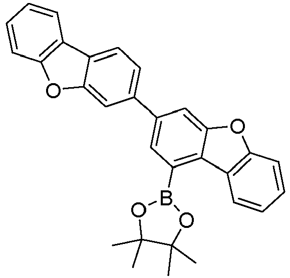 | 88% | 461.34 |
| E2 | C2 | 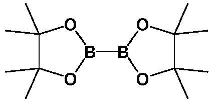 | 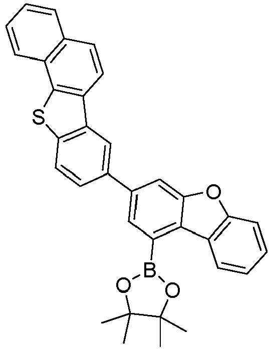 | 89% | 527.46 |
| E3 | C3 | 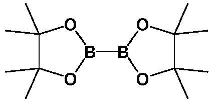 | 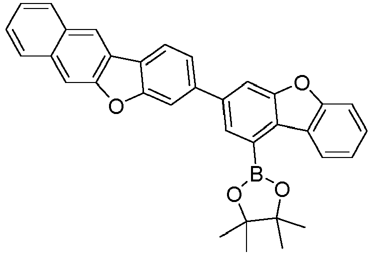 | 85% | 511.40 |
| E4 | C4 | 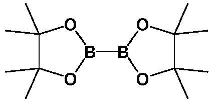 | 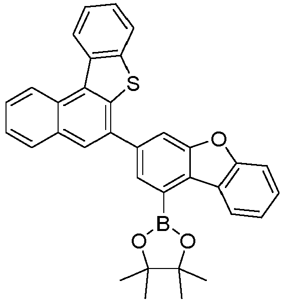 | 88% | 527.46 |
| E5 | C5 | 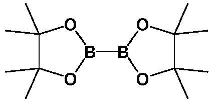 | 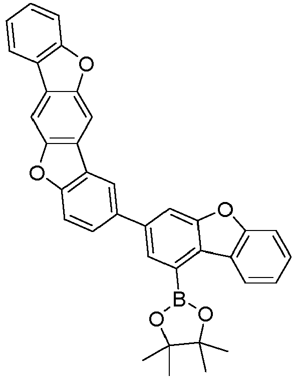 | 84% | 551.42 |
| E6 | C6 | 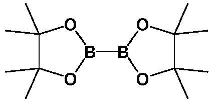 | 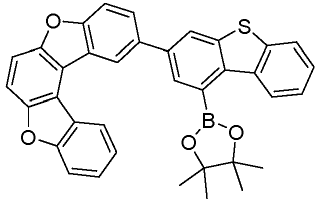 | 85% | 567.48 |
| E7 | C7 | 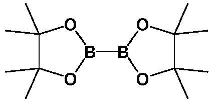 | 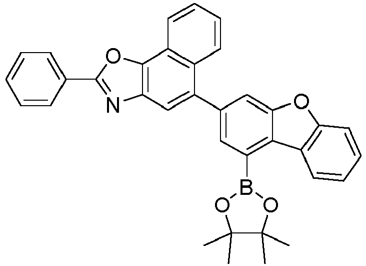 | 89% | 538.42 |
| E8 | C8 | 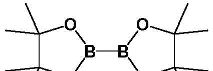 | 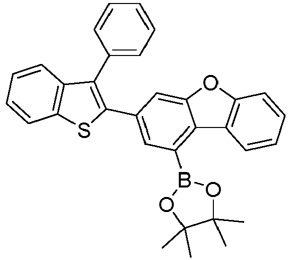 | 88% | 504.44 |
| E9 | C9 | 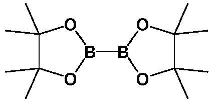 | 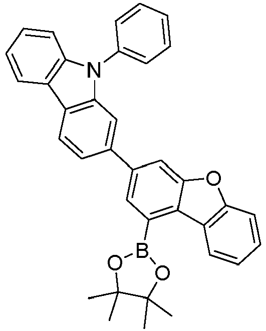 | 87% | 536.45 |
| E10 | C10 | 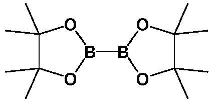 | 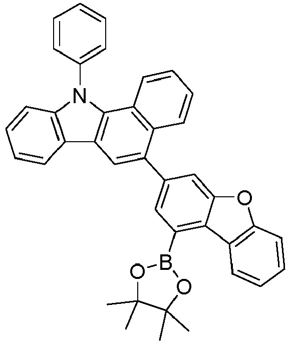 | 89% | 586.51 |
| E11 | C11 | 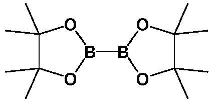 | 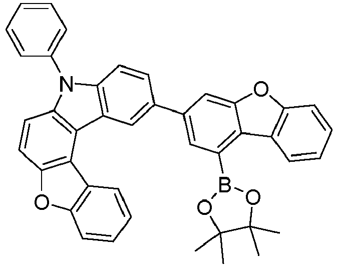 | 90% | 626.53 |
| E12 | C12 | 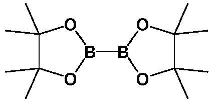 | 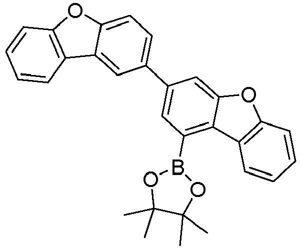 | 88% | 461.34 |
| E13 | C13 | 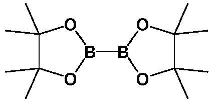 | 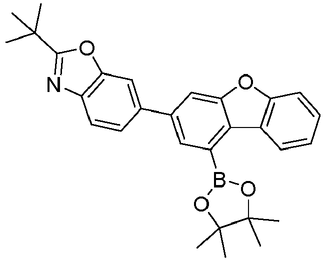 | 87% | 468.37 |
| E14 | C14 | 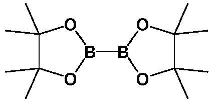 | 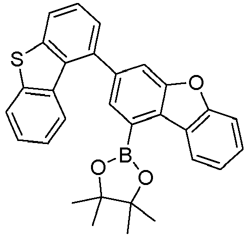 | 88% | 477.40 |
| E15 | C15 | 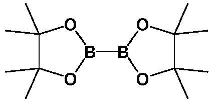 | 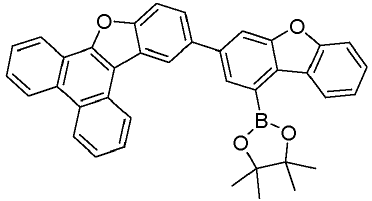 | 84% | 561.46 |
| E16 | C16 | 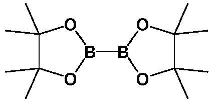 | 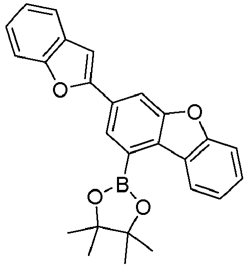 | 89% | 411.28 |
| E17 | C17 | 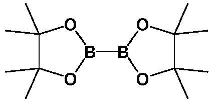 | 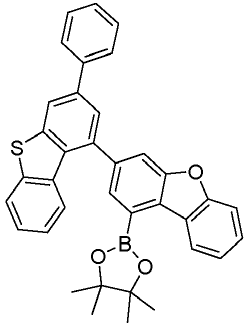 | 87% | 553.50 |
| E18 | C18 | 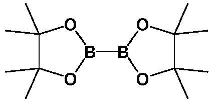 | 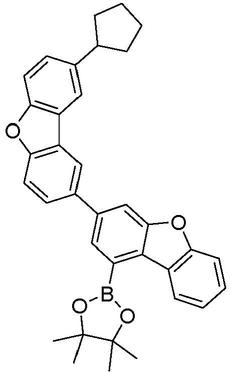 | 84% | 529.46 |
| E19 | C19 | 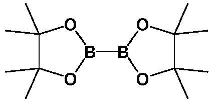 | 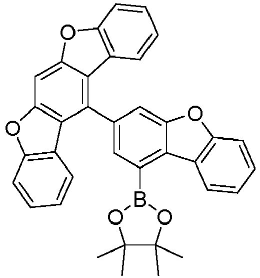 | 89% | 551.42 |
| E20 | C20 | 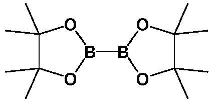 | 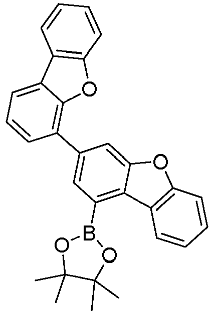 | 87% | 461.34 |
| E21 | D1 | 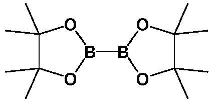 | 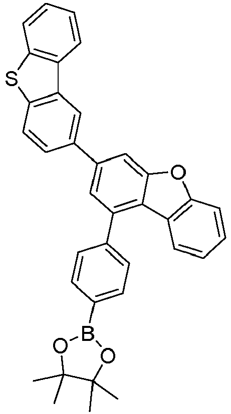 | 88% | 553.50 |
| E22 | D2 | 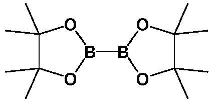 | 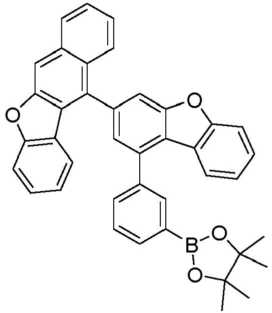 | 86% | 587.49 |
| E23 | C23 | 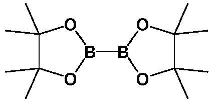 | 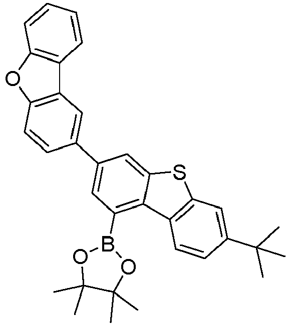 | 88% | 533.51 |
| E24 | C24 | 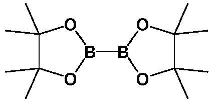 | 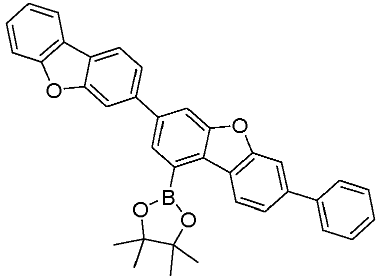 | 87% | 537.43 |
| E25 | C25 | 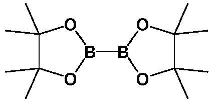 | 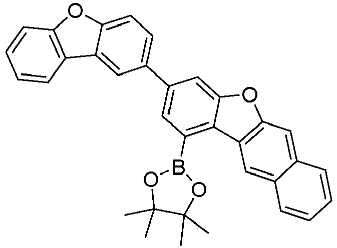 | 90% | 511.40 |
| E26 | C26 | 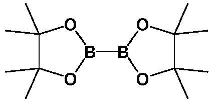 | 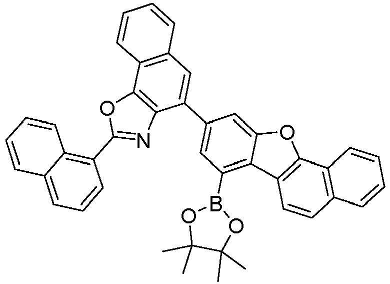 | 85% | 638.54 |
| E27 | C27 | 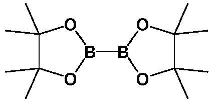 | 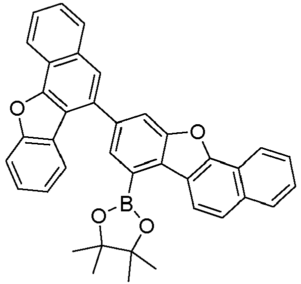 | 84% | 561.46 |
| E28 | C28 | 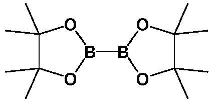 | 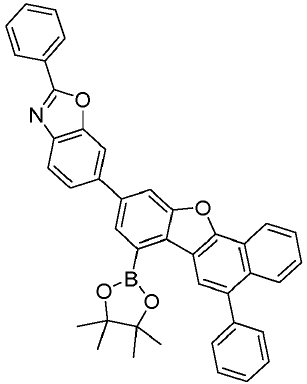 | 89% | 614.52 |
| E29 | C29 | 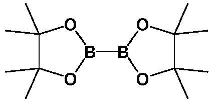 | 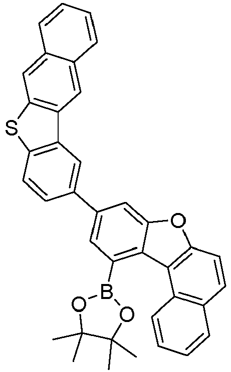 | 87% | 577.52 |
| E30 | C30 | 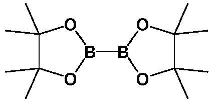 | 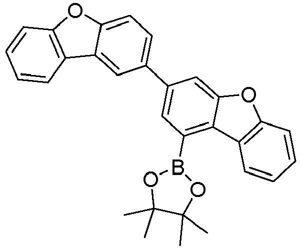 | 85% | 461.34 |
| E31 | C31 | 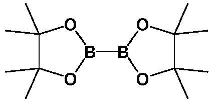 | 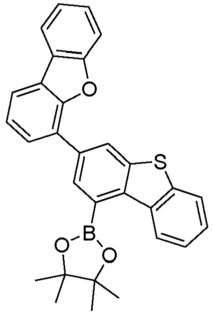 | 79% | 477.40 |
| E32 | C32 | 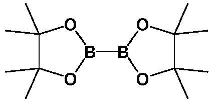 | 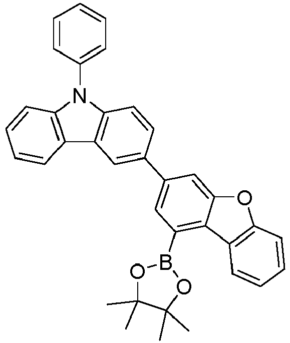 | 83% | 536.45 |
| E33 | C33 | 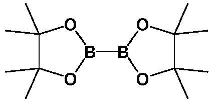 | 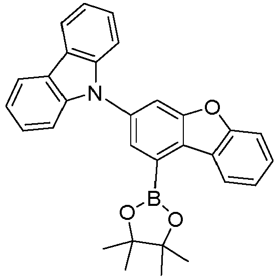 | 80% | 460.35 |

### Preparation Example 6 (Synthesis of Compounds 1 to 29 and 33 to 36)

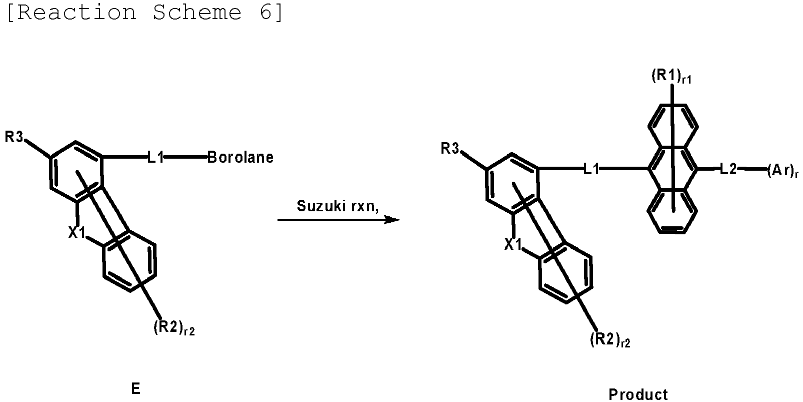

Here, R1, R2, R3, Ar, r, r1, r2, X1, L1 and L2 are the same as the definitions in Chemical Formula 1.

After Intermediate E (1 eq) synthesized in Preparation Example 5 and SM7 (1.05 eq) were added to tetrahydrofuran (excess), a 2 M aqueous potassium carbonate solution (30 volume ratio compared to THF) was added thereto, and tetrakis triphenyl-phosphino palladium (2 mol%) was added thereto, and then the resulting mixture was heated and stirred for 10 hours. After the temperature was lowered to room temperature and the reaction was terminated, the aqueous potassium carbonate solution was removed to separate the layers. Thereafter, the solvent was removed and the residue was purified by recrystallization using toluene to prepare a product.

Compounds 1 to 29 and 33 to 36 of the following [Table 1] were synthesized in the same manner as described above, except that E and SM7 were changed as shown in the following Table 1.

**[Table 1]**

| Compound | E | SM7 | Product | Yield | MS [M+H] ⁺ |
|---|---|---|---|---|---|
| **1** | **E1** | 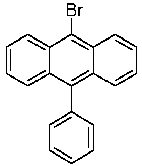 | 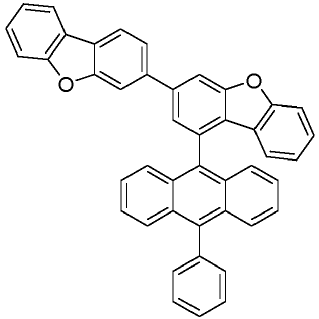 | 66% | 587.69 |
| **2** | **E2** | 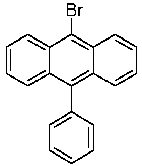 | 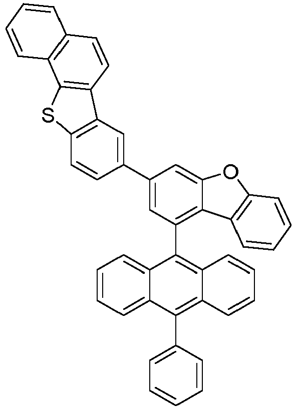 | 63% | 653.81 |
| **3** | **E3** | 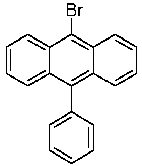 | 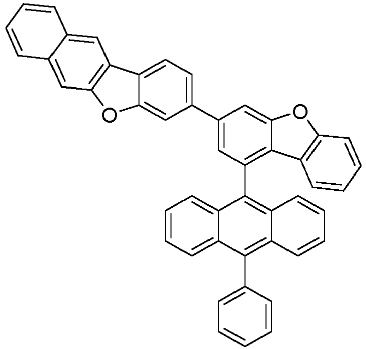 | 65% | 637.75 |
| **4** | **E4** | 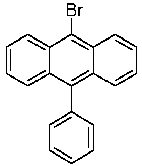 | 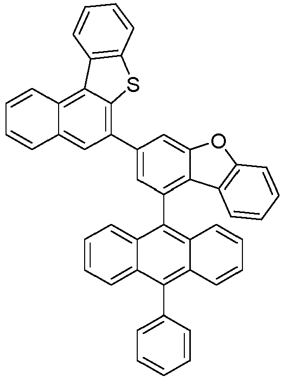 | 64% | 653.81 |
| **5** | **E5** | 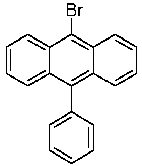 | 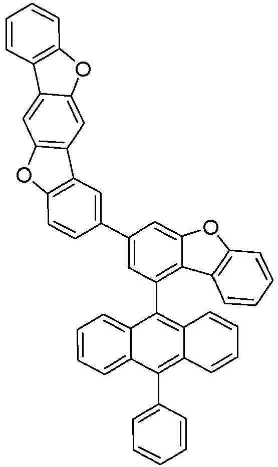 | 60% | 677.77 |
| **6** | **E6** | 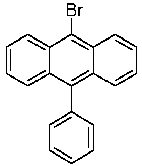 | 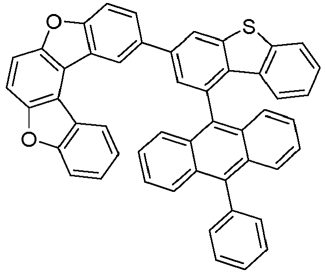 | 68% | 693.83 |
| **7** | **E7** | 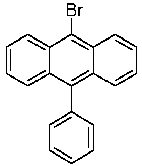 | 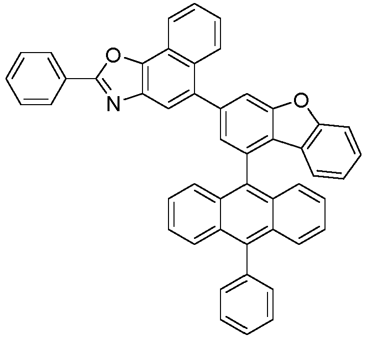 | 63% | 664.78 |
| **8** | **E8** | 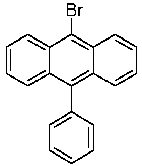 | 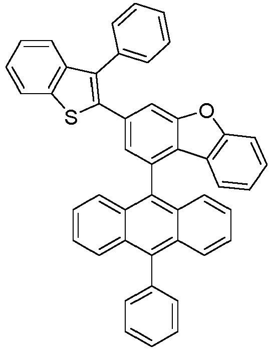 | 65% | 629.79 |
| **9** | **E9** | 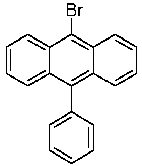 | 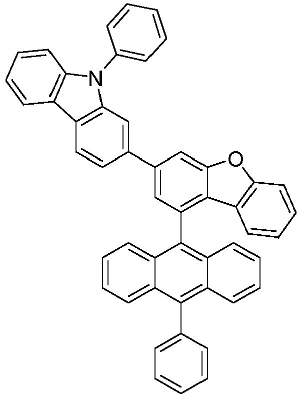 | 67% | 662.80 |
| **10** | **E10** | 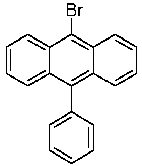 | 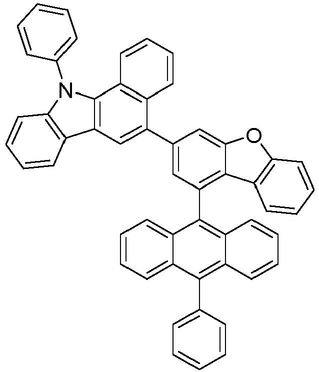 | 60% | 712.86 |
| **11** | **E11** | 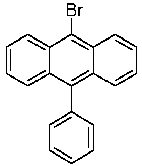 | 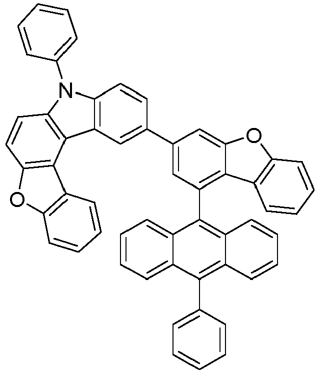 | 70% | 752.89 |
| **12** | **E12** | 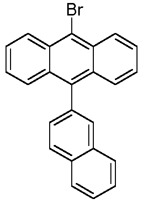 | 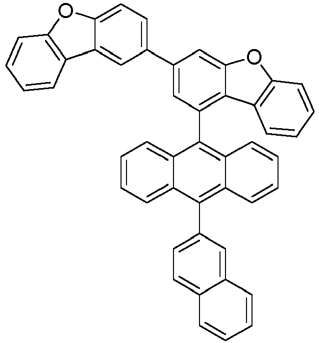 | 63% | 637.75 |
| **13** | **E13** | 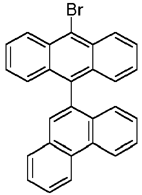 | 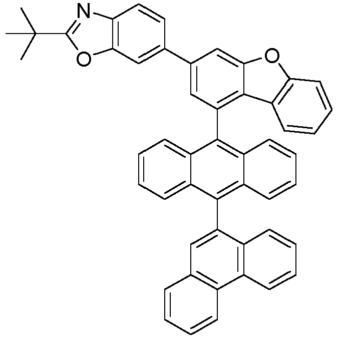 | 66% | 694.85 |
| **14** | **E14** | 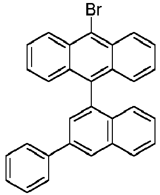 | 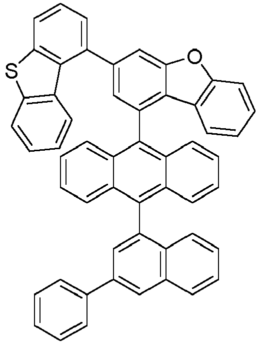 | 65% | 729.91 |
| **15** | **E15** | 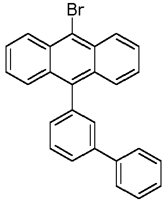 | 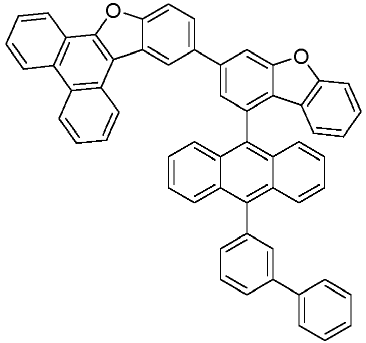 | 68% | 763.91 |
| **16** | **E16** | 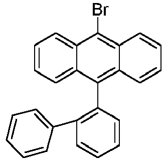 | 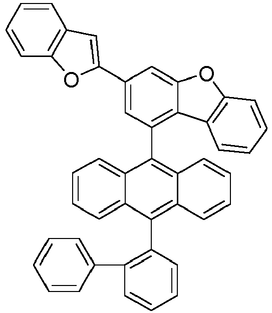 | 62% | 613.73 |
| **17** | **E17** | 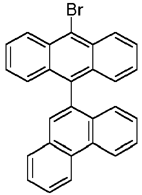 | 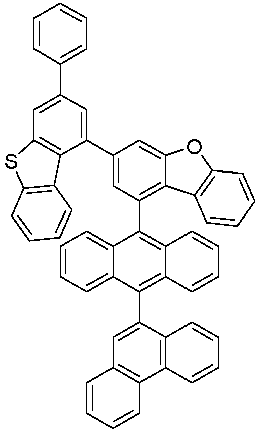 | 60% | 779.97 |
| **18** | **E18** | 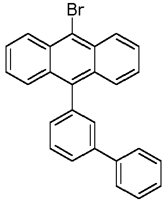 | 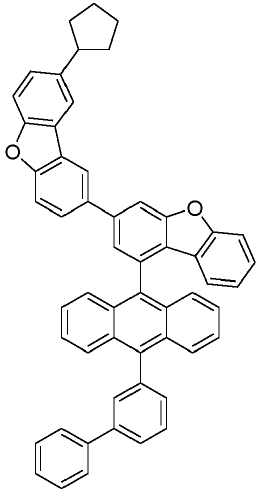 | 66% | 731.91 |
| **19** | **E19** | 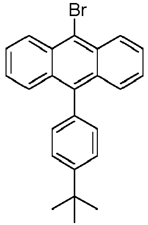 | 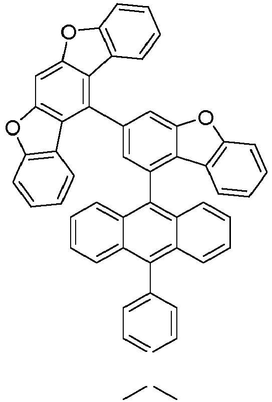 | 60% | 733.88 |
| **20** | **E20** | 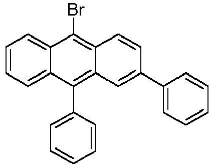 | 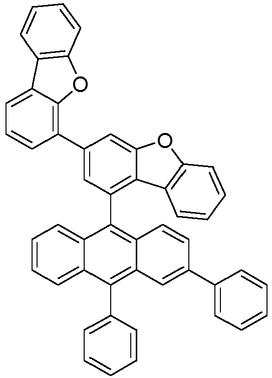 | 63% | 663.79 |
| **21** | **E21** | 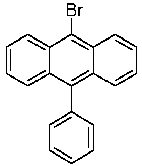 | 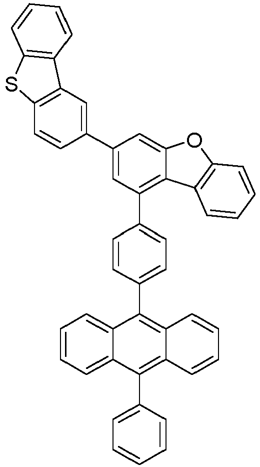 | 67% | 679.85 |
| **22** | **E22** | 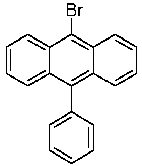 | 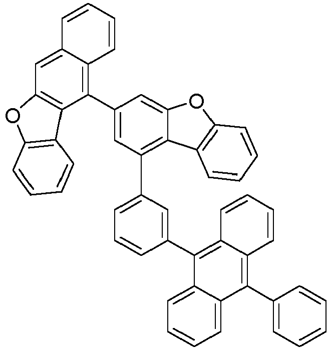 | 64% | 713.85 |
| **23** | **E23** | 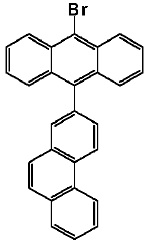 | 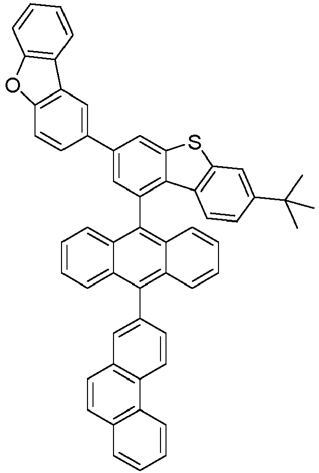 | 65% | 759.98 |
| **24** | **E24** | 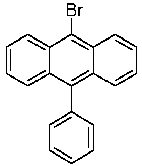 | 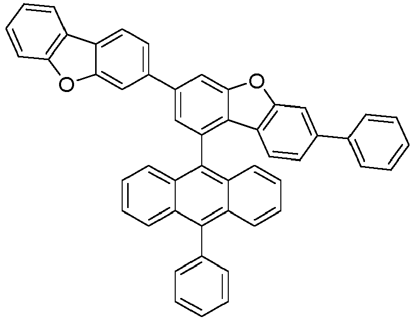 | 66% | 663.79 |
| **25** | **E25** | 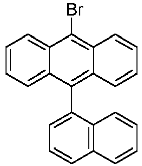 | 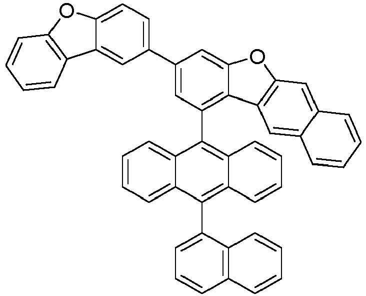 | 68% | 687.81 |
| **26** | **E26** | 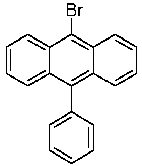 | 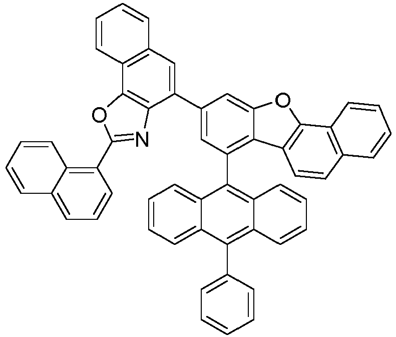 | 69% | 674.90 |
| **27** | **E27** | 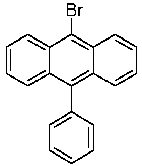 | 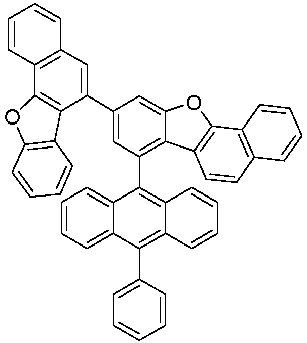 | 63% | 687.81 |
| **28** | **E28** | 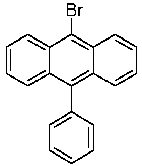 | 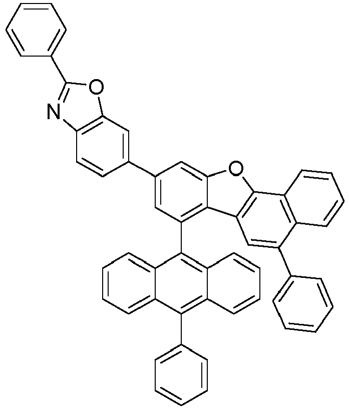 | 60% | 740.87 |
| **29** | **E29** | 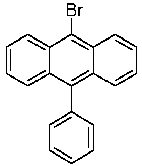 | 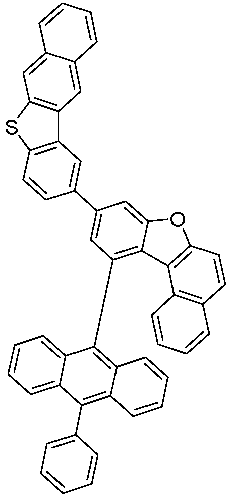 | 61% | 703.87 |
| **33** | **E30** | 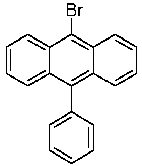 | 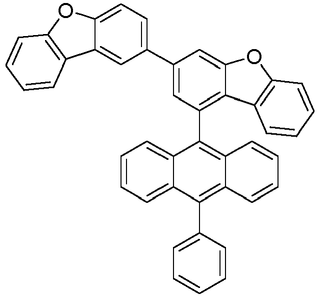 | 60% | 587.69 |
| **34** | **E31** | 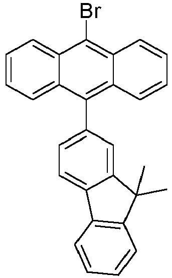 | 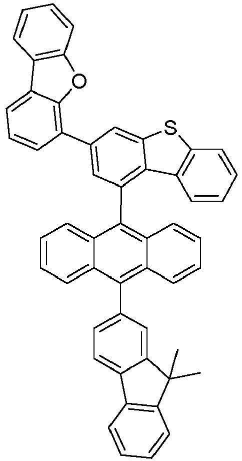 | 66% | 719.91 |
| **35** | **E32** | 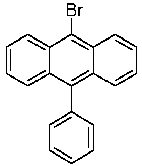 | 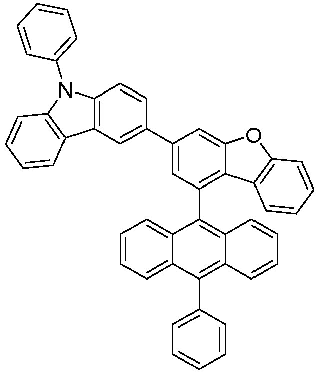 | 65% | 662.80 |
| **36** | **E33** | 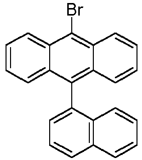 | 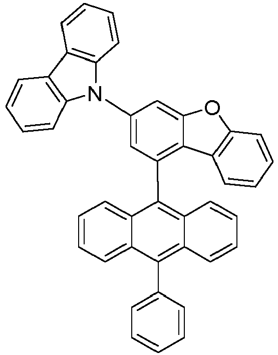 | 63% | 636.77 |

### Preparation Example 7 (Synthesis of Compounds 30 to 32)

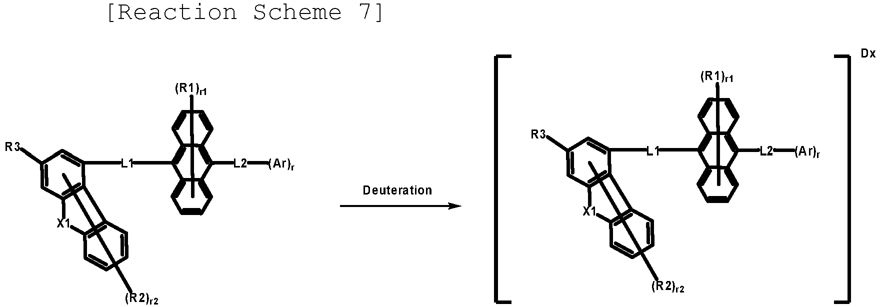

Here, R1, R2, R3, Ar, r, r1, r2, X1, L1 and L2 are the same as the definitions in Chemical Formula 1, D is deuterium, and n means the number of deuterium atoms substituted.

The reactant (1 eq) and trifluoromethanesulfonic acid (cat.) were put into C₆D₆ (10- to 50-fold mass ratio compared to the reactant), and the resulting mixture was stirred at 70°C for 10 minutes to 100 minutes. After the reaction was completed, D₂O (excess) was added thereto, the resulting solution was stirred for 30 minutes, and then trimethylamine (excess) was added dropwise thereto. The reaction solution was transferred to a separatory funnel, and an extraction with water and chloroform was performed. The extract was dried over MgSO₄, and then recrystallized by heating with toluene to obtain Compounds 30 to 32 of the following Table 2.

**[Table 2]**

| Compound | 30 | 31 | 32 |
|---|---|---|---|
| Reactant | Compound 1 | Compound 12 | Compound 24 |
| Product | 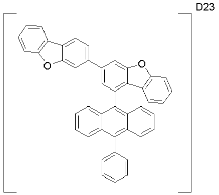 | 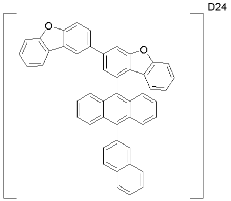 | 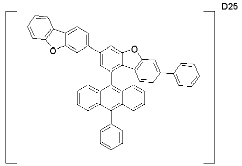 |
| Reactant | 586 m/z | 636m/z | 662m/z |
| Product theoretical max | 612 m/z | 664 m/z | 692 m/z |
| Reaction time | 50 min | 50 min | 50 min |
| Product actual max | 609 m/z | 660 m/z | 687 m/z |
| Number of total hydrogens | 26 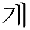 | 28 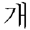 | 30 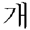 |
| D substitution number | 23 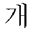 | 24 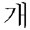 | 25 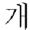 |
| D substitution ratio | 88% | 86% | 83% |
| Yield | 70% | 66% | 67% |

The deuterium substitution reaction for the aforementioned products was performed with reference to the prior literature of KR 1538534 B1, and since the degree of deuterium substitution for each product differs depending on the reaction time, the overall reaction time was adjusted to 50 minutes to determine the substitution rate according to the maximum m/z (M+) value.

### <Examples> Manufacture of OLED

As an anode, a substrate on which ITO/Ag/ITO were deposited to have a thickness of 70/1,000/70 Å was cut into a size of 50 mm x 50 mm x 0.5 mm, put into distilled water in which a detergent was dissolved, and washed with ultrasonic waves. A product manufactured by Fischer Co., was used as a detergent, and distilled water twice filtered using a filter manufactured by Millipore Co., was used as the distilled water. After the indium tin oxide (ITO) was washed for 30 minutes, ultrasonic washing was repeated twice with distilled water for 10 minutes. After the washing using distilled water was completed, ultrasonic washing was conducted using isopropyl alcohol, acetone, and methanol solvents in this order, and drying was then conducted.

HI-1 was thermally vacuum-deposited to have a thickness of 50 Å on the anode thus prepared, thereby forming a hole injection layer, and HT1 as a material which transports holes was vacuum-deposited thereon to have a thickness of 1,150 Å, thereby forming a hole transport layer, Next, a hole adjusting layer was formed using EB1 (150 Å), and then while using the compound synthesized in Preparation Example 6 or 7 as a host, the host was vacuum-deposited along with a dopant (2 wt%) to have a thickness of 360 Å, thereby forming a light emitting layer. Thereafter, ET1 was deposited to have a thickness of 50 Å, thereby forming an electron adjusting layer, and Compounds ET2 and Liq were mixed at a ratio of 7:3, thereby forming an electron transport layer having a thickness of 250 Å. Sequentially, magnesium and lithium fluoride (LiF) were deposited to have a thickness of 50 Å to form a film as an electron injection layer <EIL>, magnesium and silver (1 : 4) were used to form a negative electrode having a thickness of 200 Å, and then CP1 was deposited to have a thickness of 600 Å, thereby completing a device. In the aforementioned procedure, the deposition rates of the organic materials were each maintained at 1 Å/sec.

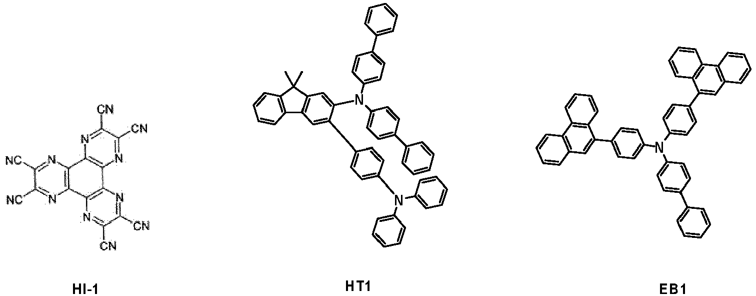

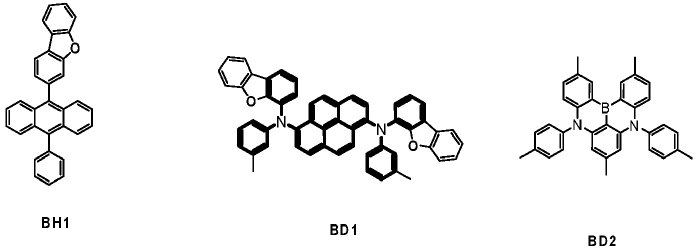

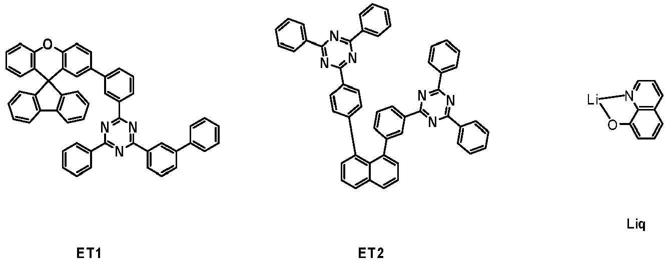

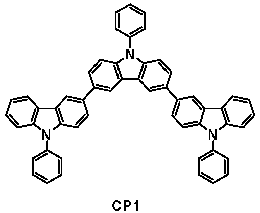

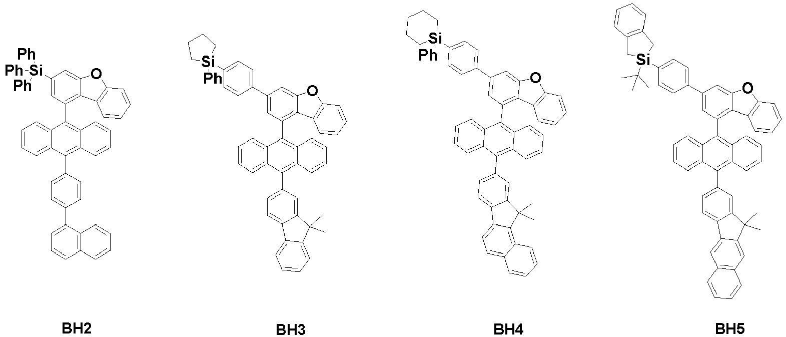

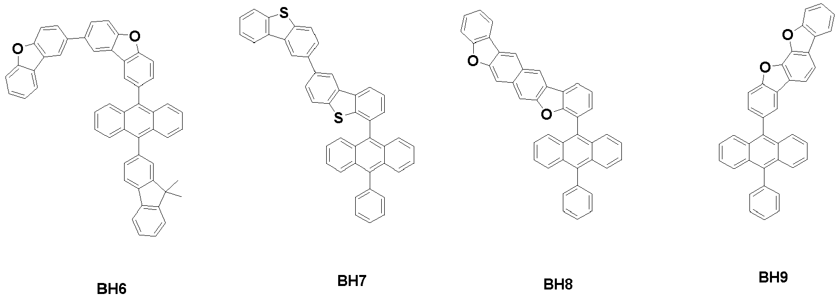

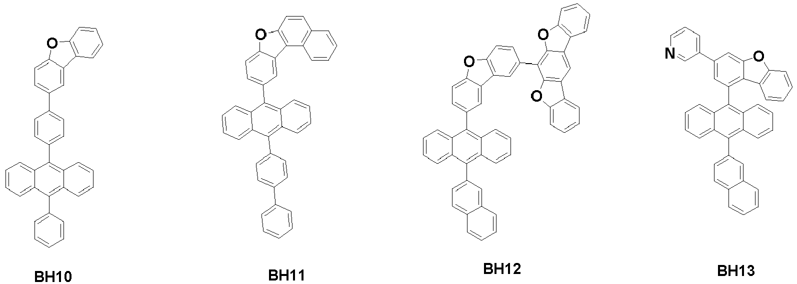

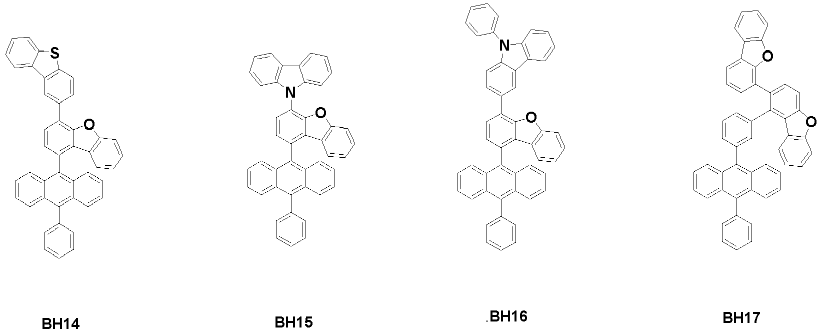

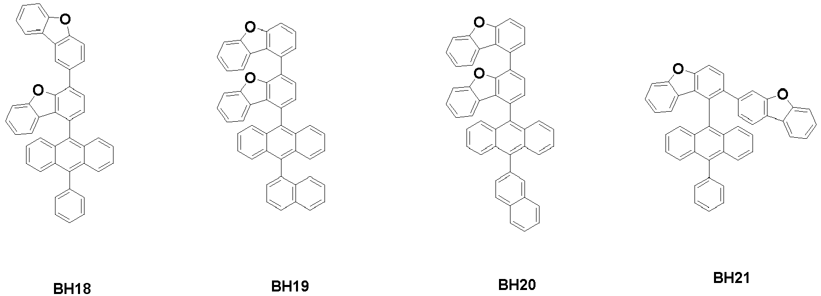

Examples 1 to 42 and Comparative Examples 1 to 23, which are the experimental results of an organic light emitting device manufactured using any one of Compounds 1 to 36 synthesized in Preparation Examples 6 and 7 as a host material for the light emitting layer and using BD1 or BD2 as a dopant material for the light emitting layer, are shown in [Table 3].

In this case, for the organic light emitting devices, the driving voltage and the light emitting efficiency were measured at a current density of 20 mA/cm², and a time (T95) for reaching a 98% value compared to the initial luminance was measured at a current density of 20 mA/cm².

**[Table 3]**

| Experimental Example 20 mA/cm² | Host | Dopant | Voltage (V) (@20 mA/ cm²) | Cd/A (@20 mA/ cm²) | Color coordinate (x,y) | Service life (T95, h) (@20 mA/ cm²) |
|---|---|---|---|---|---|---|
| Example 1 | Compound 1 | BD1 | 3.35 | 6.67 | (0.135, 0.142) | 53.4 |
| Example 2 | Compound 2 | BD1 | 3.34 | 6.59 | (0.134, 0.143) | 55.1 |
| Example 3 | Compound 3 | BD1 | 3.40 | 6.60 | (0.135, 0.142) | 56.3 |
| Example 4 | Compound 4 | BD1 | 3.38 | 6.59 | (0.135, 0.142) | 52.8 |
| Example 5 | Compound 5 | BD1 | 3.34 | 6.54 | (0.136, 0.155) | 53.0 |
| Example 6 | Compound 6 | BD1 | 3.33 | 6.55 | (0.135, 0.156) | 54.2 |
| Example 7 | Compound 7 | BD1 | 3.39 | 6.57 | (0.136, 0.155) | 55.2 |
| Example 8 | Compound 8 | BD1 | 3.38 | 6.54 | (0.135, 0.142) | 53.8 |
| Example 9 | Compound 9 | BD1 | 3.31 | 6.70 | (0.135, 0.141) | 56.1 |
| Example 10 | Compound 10 | BD1 | 3.32 | 6.59 | (0.136, 0.155) | 56.8 |
| Example 11 | Compound 11 | BD1 | 3.35 | 6.60 | (0.136, 0.155) | 58.3 |
| Example 12 | Compound 12 | BD1 | 3.33 | 6.69 | (0.135, 0.141) | 55.2 |
| Example 13 | Compound 13 | BD1 | 3.34 | 6.59 | (0.135, 0.143) | 56.1 |
| Example 14 | Compound 14 | BD1 | 3.38 | 6.51 | (0.135, 0.141) | 56.0 |
| Example 15 | Compound 15 | BD1 | 3.33 | 6.55 | (0.134, 0.142) | 53.9 |
| Example 16 | Compound 16 | BD1 | 3.35 | 6.57 | (0.134, 0.144) | 57.1 |
| Example 17 | Compound 17 | BD1 | 3.34 | 6.53 | (0.135, 0.141) | 55.6 |
| Example 18 | Compound 18 | BD1 | 3.31 | 6.58 | (0.134, 0.142) | 56.4 |
| Example 19 | Compound 19 | BD1 | 3.32 | 6.56 | (0.136, 0.150) | 54.1 |
| Example 20 | Compound 20 | BD1 | 3.33 | 6.55 | (0.135, 0.143) | 56.5 |
| Example 21 | Compound 21 | BD1 | 3.35 | 6.71 | (0.134, 0.142) | 53.8 |
| Example 22 | Compound 22 | BD1 | 3.37 | 6.59 | (0.136, 0.156) | 55.1 |
| Example 23 | Compound 23 | BD1 | 3.35 | 6.60 | (0.134, 0.142) | 54.0 |
| Example 24 | Compound 24 | BD1 | 3.33 | 6.61 | (0.136, 0.151) | 56.2 |
| Example 25 | Compound 25 | BD1 | 3.36 | 6.57 | (0.136, 0.151) | 53.1 |
| Example 26 | Compound 26 | BD1 | 3.32 | 6.60 | (0.136, 0.156) | 54.2 |
| Example 27 | Compound 27 | BD1 | 3.31 | 6.58 | (0.136, 0.151) | 55.2 |
| Example 28 | Compound 28 | BD1 | 3.33 | 6.55 | (0.136, 0.151) | 53.6 |
| Example 29 | Compound 29 | BD1 | 3.36 | 6.54 | (0.136, 0.150) | 56.0 |
| Example 31 | Compound 30 | BD1 | 3.36 | 6.67 | (0.135, 0.142) | 70.2 |
| Example 32 | Compound 31 | BD1 | 3.33 | 6.68 | (0.135, 0.141) | 72.8 |
| Example 33 | Compound 32 | BD1 | 3.34 | 6.61 | (0.136, 0.151) | 73.9 |
| Example 34 | Compound 1 | BD2 | 3.47 | 7.12 | (0.135, 0.138) | 48.5 |
| Example 35 | Compound 9 | BD2 | 3.45 | 7.20 | (0.135, 0.136) | 50.3 |
| Example 36 | Compound 15 | BD2 | 3.44 | 7.08 | (0.134, 0.137) | 46.7 |
| Example 37 | Compound 23 | BD2 | 3.42 | 7.10 | (0.134, 0.136) | 47.1 |
| Example 38 | Compound 28 | BD2 | 3.41 | 7.09 | (0.136, 0.145) | 47.0 |
| Example 39 | Compound 33 | BD1 | 3.35 | 6.61 | (0.135, 0.143) | 55.1 |
| Example 40 | Compound 34 | BD1 | 3.32 | 6.54 | (0.135, 0.143) | 53.2 |
| Example 41 | Compound 35 | BD1 | 3.34 | 6.58 | (0.135, 0.143) | 50.8 |
| Example 42 | Compound 36 | BD1 | 3.33 | 6.60 | (0.134, 0.142) | 55.4 |
| Comparative Example 1 | BH1 | BD1 | 3.75 | 6.01 | (0.134, 0.139) | 32.8 |
| Comparative Example 2 | BH2 | BD1 | 3.72 | 5.12 | (0.134, 0.142) | 21.8 |
| Comparative Example 3 | BH3 | BD1 | 3.60 | 5.00 | (0.134, 0.147) | 14.5 |
| Comparative Example 4 | BH4 | BD1 | 3.61 | 5.10 | (0.134, 0.148) | 15.8 |
| Comparative Example 5 | BH5 | BD1 | 3.62 | 4.90 | (0.134, 0.147) | 14.7 |
| Comparative Example 6 | BH6 | BD1 | 3.60 | 5.23 | (0.134, 0.150) | 18.5 |
| Comparative Example 7 | BH7 | BD1 | 3.73 | 5.30 | (0.134, 0.151) | 30.5 |
| Comparative Example 8 | BH8 | BD1 | 3.68 | 5.28 | (0.134, 0.156) | 28.9 |
| Comparative Example 9 | BH9 | BD1 | 3.66 | 5.30 | (0.134, 0.152) | 29.4 |
| Comparative Example 10 | BH10 | BD1 | 3.74 | 5.81 | (0.134, 0.139) | 31.5 |
| Comparative Example 11 | BH11 | BD1 | 3.69 | 5.88 | (0.134, 0.140) | 37.2 |
| Comparative Example 12 | BH12 | BD1 | 3.70 | 5.21 | (0.134, 0.153) | 23.1 |
| Comparative Example 13 | BH13 | BD1 | 3.84 | 4.01 | (0.134, 0.140) | 15.6 |
| Comparative Example 14 | BH14 | BD1 | 3.72 | 5.74 | (0.134, 0.148) | 26.1 |
| Comparative Example 15 | BH15 | BD1 | 3.70 | 4.98 | (0.134, 0.146) | 30.2 |
| Comparative Example 16 | BH16 | BD1 | 3.71 | 5.78 | (0.134, 0.149) | 33.5 |
| Comparative Example 17 | BH17 | BD1 | 3.70 | 5.51 | (0.134, 0.154) | 31.5 |
| Comparative Example 18 | BH1 | BD2 | 3.83 | 6.12 | (0.134, 0.137) | 18.1 |
| Comparative Example 19 | BH2 | BD2 | 3.80 | 5.48 | (0.134, 0.138) | 10.4 |
| Comparative Example 20 | BH18 | BD1 | 3.72 | 5.23 | (0.135, 0.147) | 25.1 |
| Comparative Example 21 | BH19 | BD1 | 3.70 | 5.33 | (0.134, 0.148) | 23.4 |
| Comparative Example 22 | BH20 | BD1 | 3.73 | 5.28 | (0.135, 0.147) | 26.7 |
| Comparative Example 23 | BH21 | BD1 | 3.75 | 5.60 | (0.134, 0.144) | 18.7 |

In the present invention, it was confirmed that the injecting, transporting, and adjusting characteristics of holes and electrons could be improved by substituting units in a specific direction as an anthracene blue fluorescent host into which dibenzofuran or dibenzothiophene, which can be fused, and an aryl group such as fluorene were introduced, and introducing an additional substituent In the examples satisfying the substitution positions, which are the characteristics of the present specification, it is possible to observe a clear improvement in the device performance of the blue devices compared to Comparative Examples 1 to 19. The hosts of Comparative Examples 2 to 5 are examples in which the bonding position of anthracene and the substituent in the dibenzofuran unit is the same as that of the compound of Chemical Formula 1 of the present specification, but a structure into which a substituent including a silicon atom is introduced is used as a blue fluorescent host. It can be seen through the device results that the bonding position and substitution effects as in the compound of Chemical Formula 1 of the present specification do not appear in units including silicon atoms.

The structure combination of the present specification produced excellent device results by introducing a specific directional bond of dibenzofuran or dibenzothiophene and a heteroaryl group as a substituent into the unit to balance the movement of holes and electrons in the blue fluorescent host.

The hosts of Comparative Examples 6 to 17 are the results of showing, as the comparative examples, the examples in which the bonding positions of anthracene and dibenzofuran units are different from those of the compound of Chemical Formula 1 of the present specification, and the unit combination is similar to that of the compound of Chemical Formula 1 of the present specification. However, it can be seen that the combination effect of the bonding position of the dibenzofuran unit with anthracene and the substitution position of the substituent of the dibenzofuran unit results in a smaller improvement in device characteristics than in the compound of Chemical Formula 1 of the present specification.

The organic electroluminescent device combined with the compound derivative of the chemical formula according to the present specification may adjust the role of smoothly injecting holes into the light emitting layer by using the compounds of Preparation Examples 6 and 7 as the host of the blue organic electroluminescent device, and the device according to the present invention exhibits excellent characteristics in terms of efficiency, driving voltage and stability through the balance between holes and electrons of the organic light emitting device according to the chemical structure.

## Claims

1. A compound of the following Chemical Formula 1: 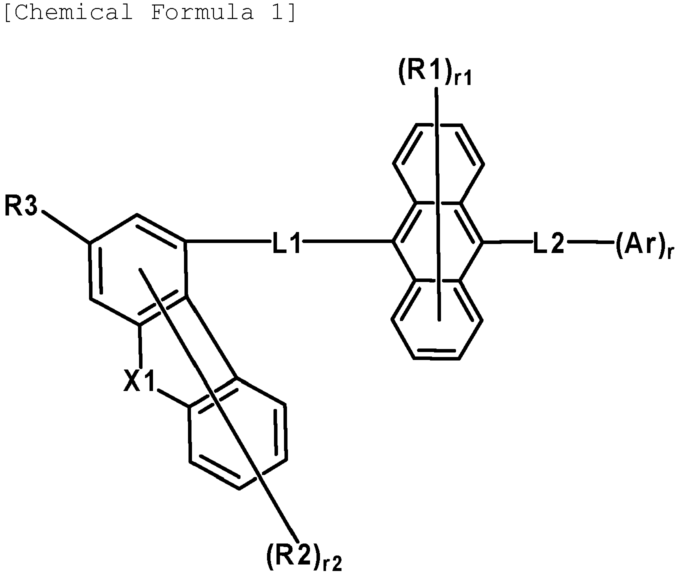 wherein, in Chemical Formula 1,
X1 is O or S,
R1 is hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S,
R2 is hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S, or adjacent groups are optionally bonded to each other to form a substituted or unsubstituted ring,
R3 is a polycyclic heterocyclic group comprising at least one selected from N, O and S; or a substituted or unsubstituted monocyclic heterocyclic group comprising at least one selected from O and S,
L1 and L2 are the same as or different from each other, and are each independently a direct bond, or a substituted or unsubstituted arylene group,
Ar is a substituted or unsubstituted aryl group,
r1 is an integer from 1 to 8,
r2 is an integer from 1 to 6,
when r1 is 2 or higher, R1's are the same as or different from each other,
when r2 is 2 or higher, R2's are the same as or different from each other, and
r is an integer from 1 to 4, and when r is 2 or higher, Ar's are the same as or different from each other.

2. The compound of claim 1, wherein Chemical Formula 1 is any one of the following Chemical Formulae 1-1 to 1-3: 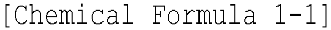 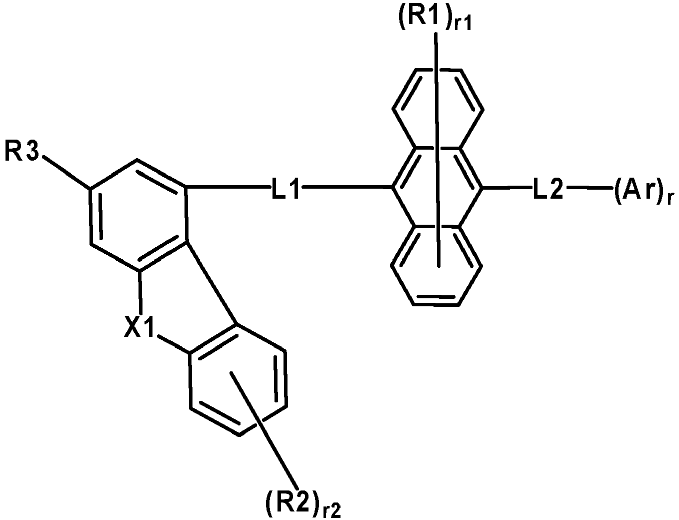 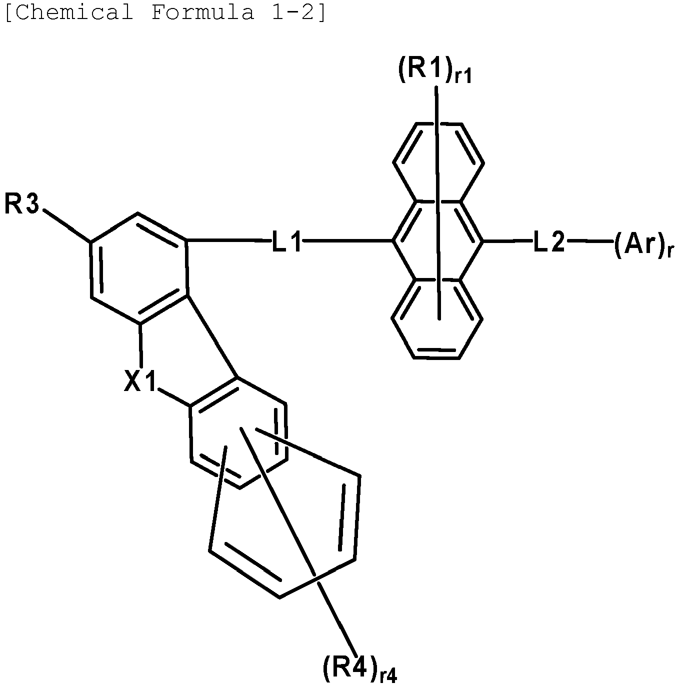 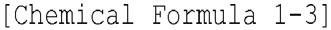 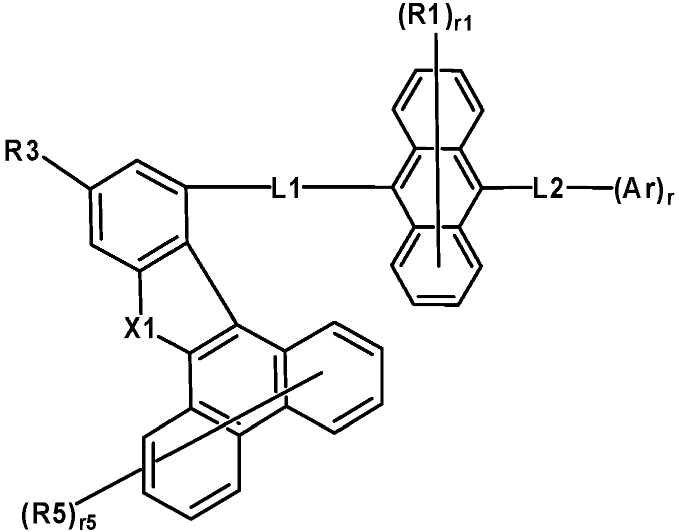 in Chemical Formulae 1-1 to 1-3,
X1, R1, R2, R3, r1, L1, L2, Ar and r are the same as the definitions in Chemical Formula 1,
R4 and R5 are the same as or different from each other, and are each independently hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S,
r2 is an integer from 1 to 4,
r4 is an integer from 1 to 6,
r5 is an integer from 1 to 8,
when r4 is 2 or higher, R4's are the same as or different from each other, and
when r5 is 2 or higher, R5's are the same as or different from each other.

3. The compound of claim 1, wherein Chemical Formula 1 is any one of the following Chemical Formulae 2 to 5: 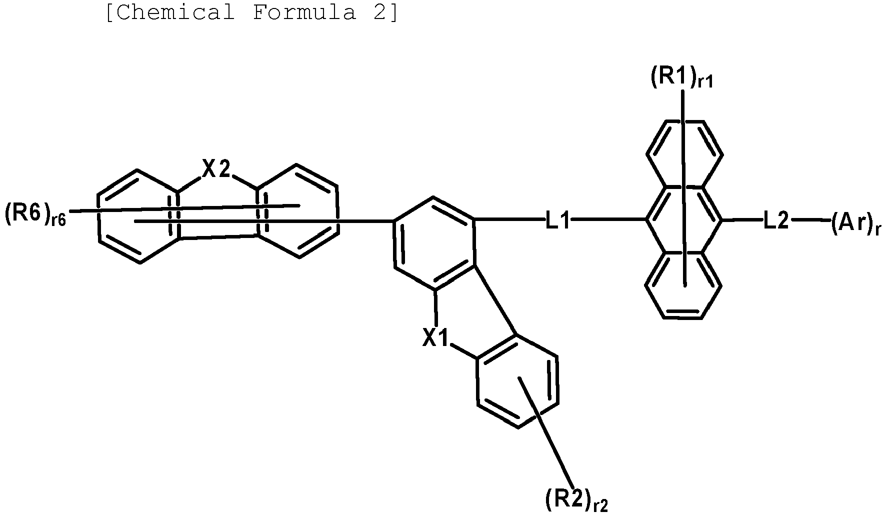 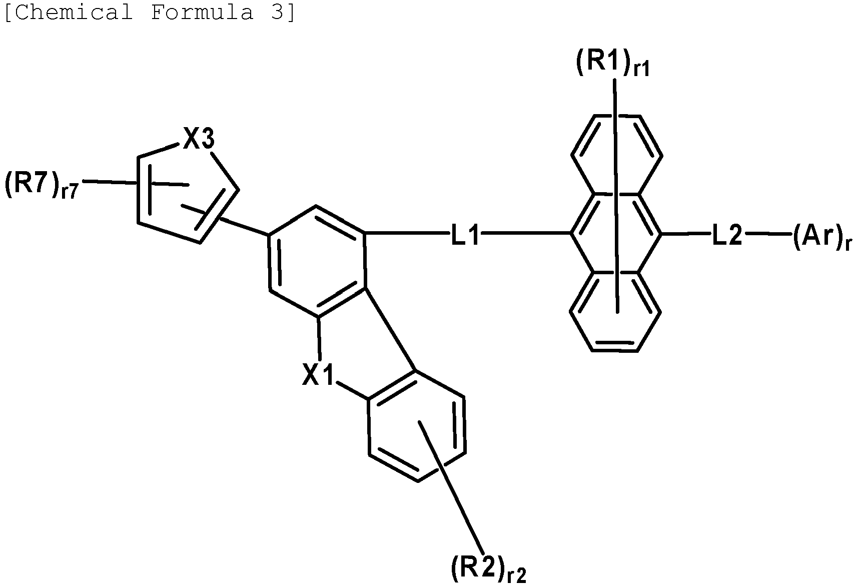 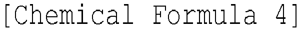 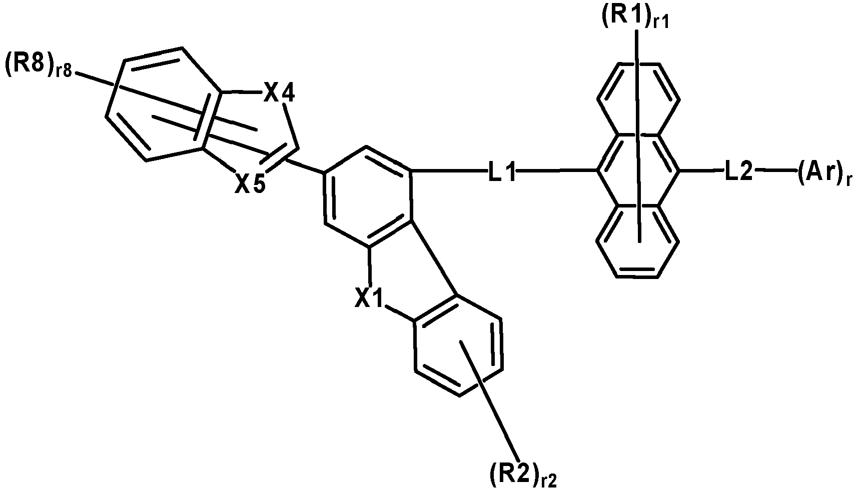 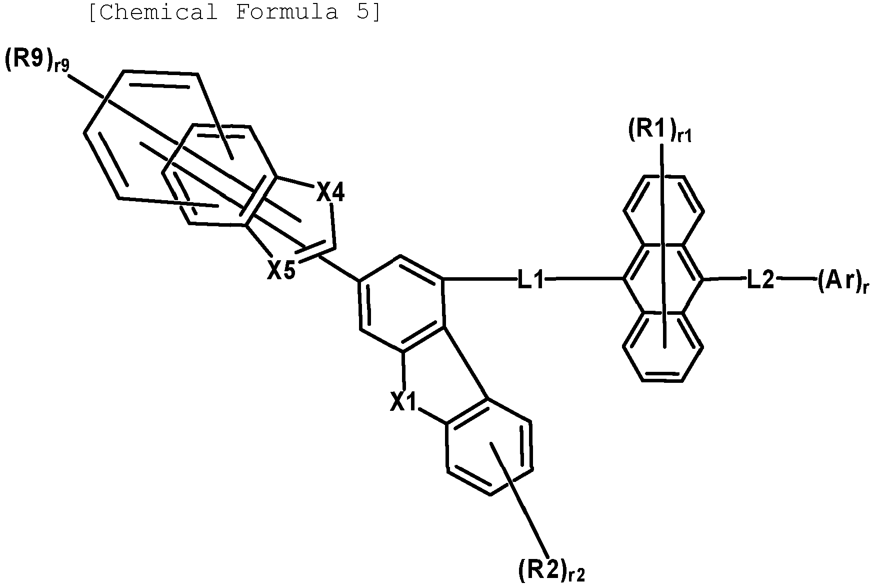 in Chemical Formulae 2 to 5,
X1, R1, R2, r1, L1, L2, Ar and r are the same as the definitions in Chemical Formula 1,
X2 is NR, O or S,
X3 and X4 are each independently O or S,
X5 is N or CR',
R, R' and R7 to R9 are the same as or different from each other, and are each independently hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S,
R6 is hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S, or adjacent groups are optionally bonded to each other to form a substituted or unsubstituted ring,
r2 is an integer from 1 to 4,
r6 is an integer from 1 to 7,
r7 is an integer from 1 to 3,
r8 is an integer from 1 to 4,
r9 is an integer from 1 to 6,
when r6 is 2 or higher, R6's are the same as or different from each other,
when r7 is 2 or higher, R7's are the same as or different from each other,
when r8 is 2 or higher, R8's are the same as or different from each other, and
when r9 is 2 or higher, R9's are the same as or different from each other.

4. The compound of claim 3, wherein Chemical Formula 2 is any one of the following Chemical Formulae 2-1 to 2-4: 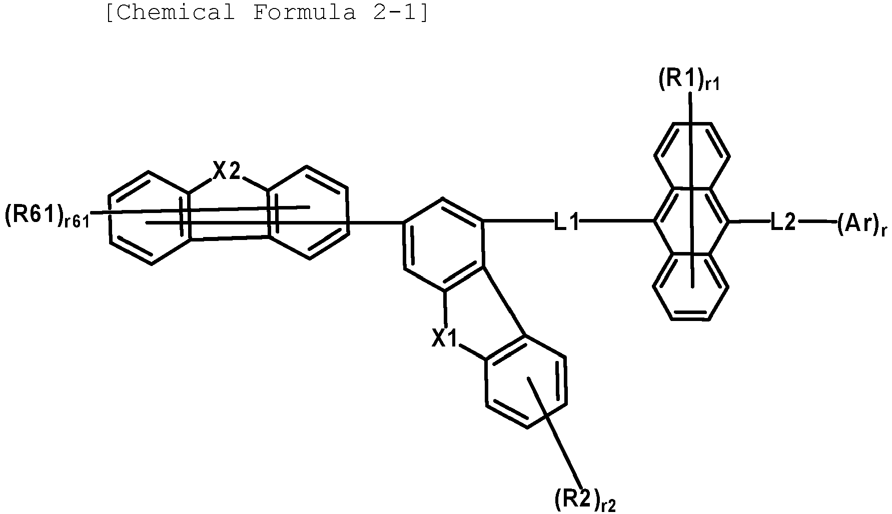 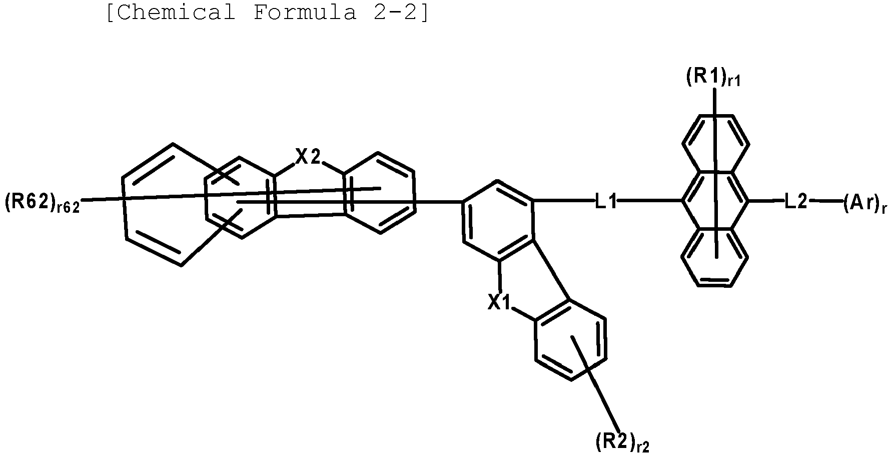 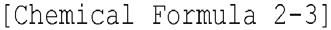 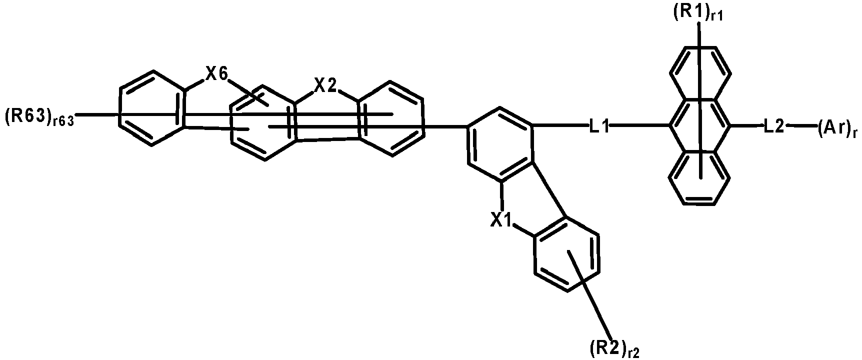 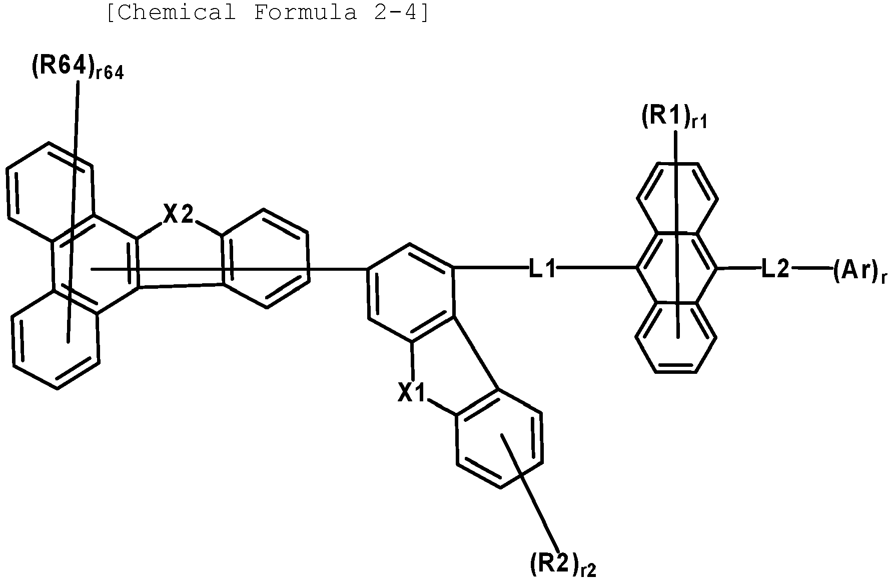 in Chemical Formulae 2-1 to 2-4,
X1, X2, R1, R2, r1, r2, L1, L2, Ar and r are the same as the definitions in Chemical Formula 2,
X6 is NR, O or S,
R and R61 to R64 are the same as or different from each other, and are each independently hydrogen, deuterium, a halogen group, a cyano group, a nitro group, a hydroxyl group, a substituted or unsubstituted alkyl group, a substituted or unsubstituted cycloalkyl group, a substituted or unsubstituted aryl group, or a substituted or unsubstituted heterocyclic group comprising at least one selected from N, O and S,
r61 is an integer from 1 to 7,
r62 and r63 are each an integer from 1 to 9,
r64 is an integer from 1 to 11,
when r61 is 2 or higher, R61's are the same as or different from each other,
when r62 is 2 or higher, R62's are the same as or different from each other,
when r63 is 2 or higher, R63's are the same as or different from each other, and
when r64 is 2 or higher, R64's are the same as or different from each other.

5. The compound of claim 1, wherein the compound of Chemical Formula 1 is any one of the following structures:

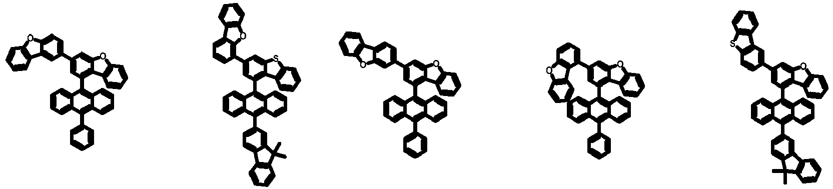

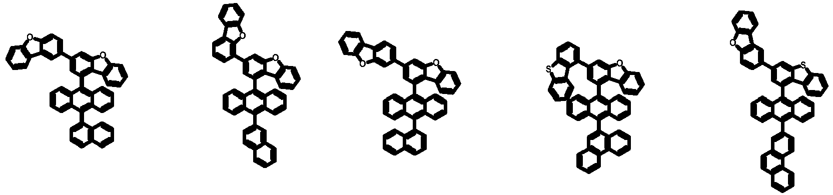

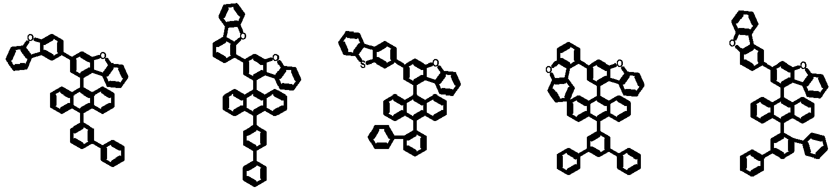

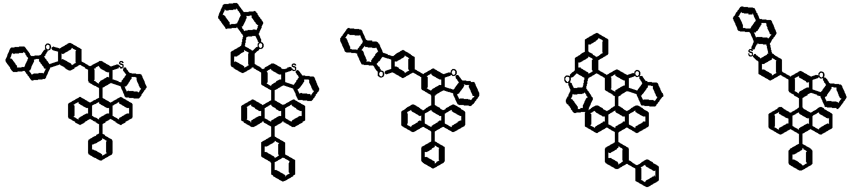

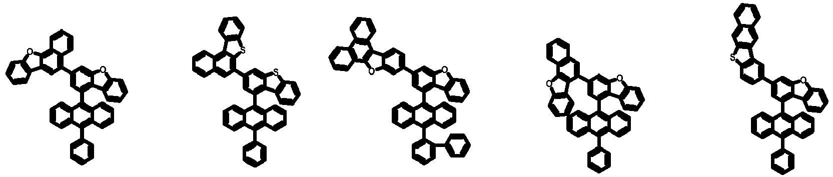

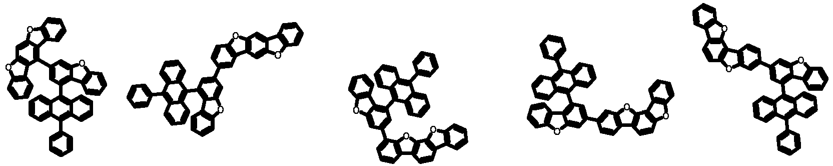

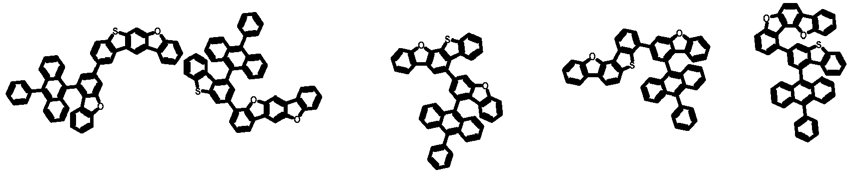

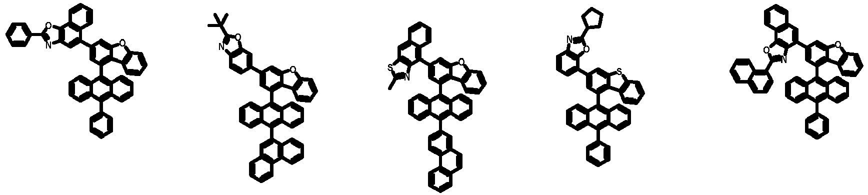

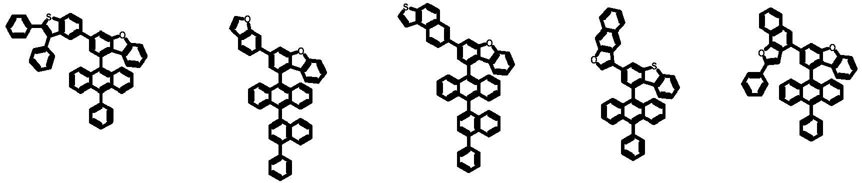

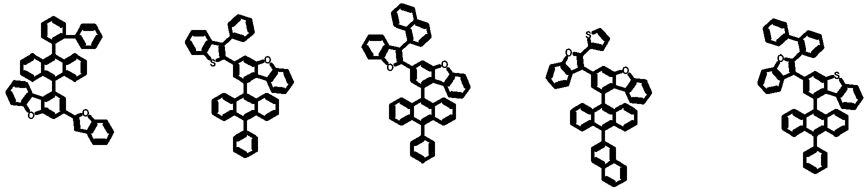

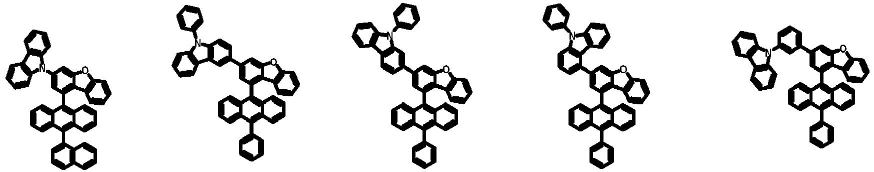

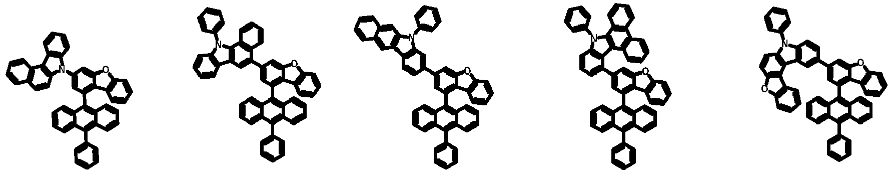

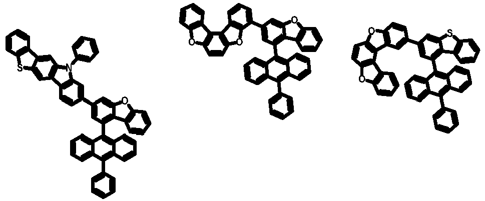

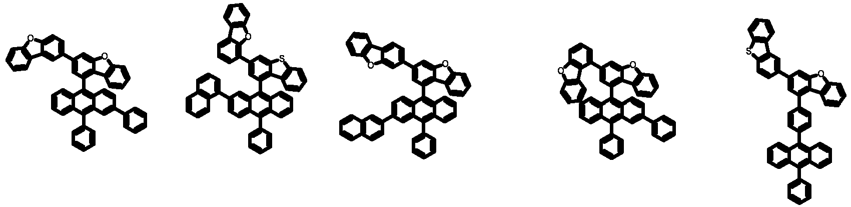

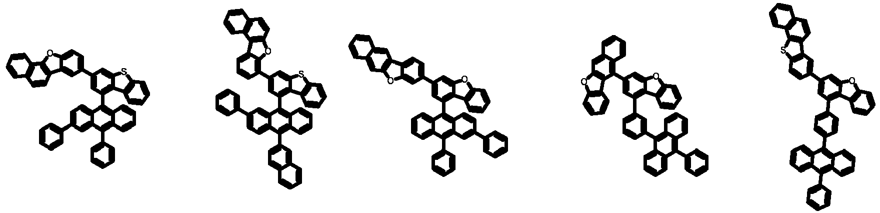

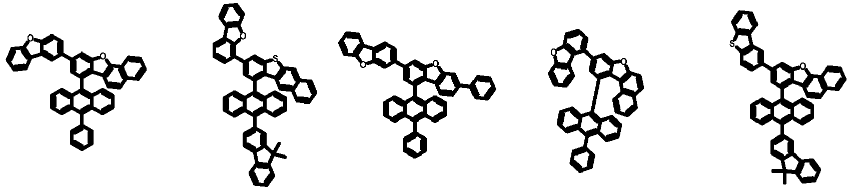

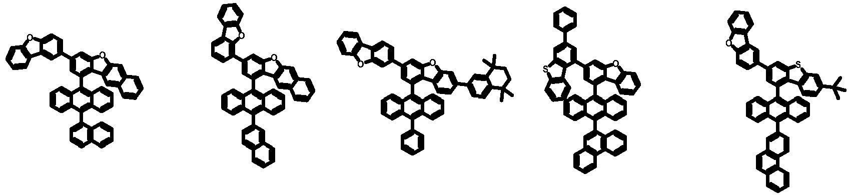

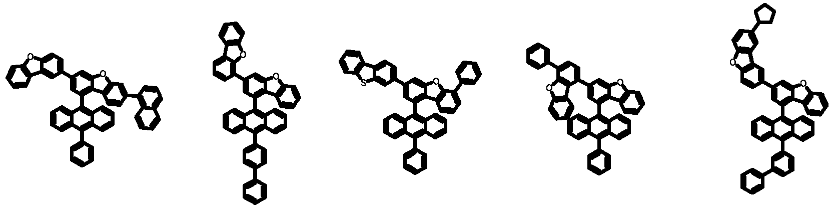

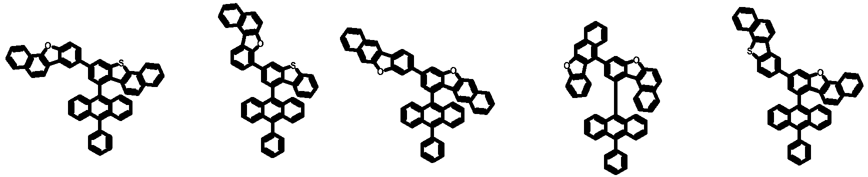

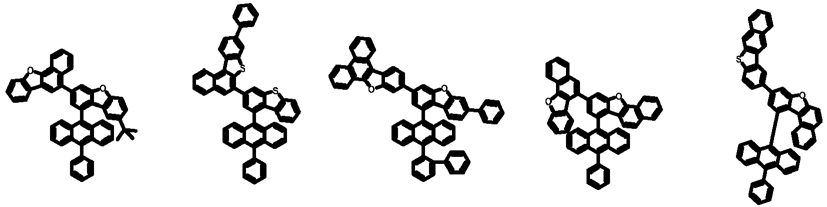

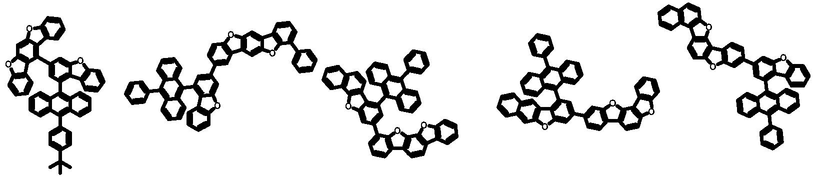

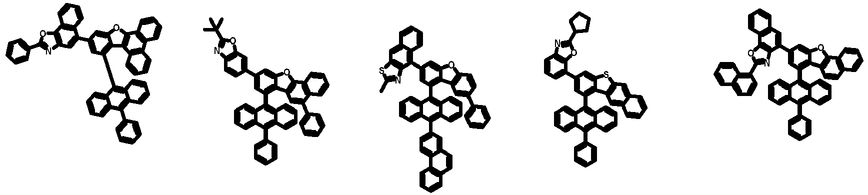

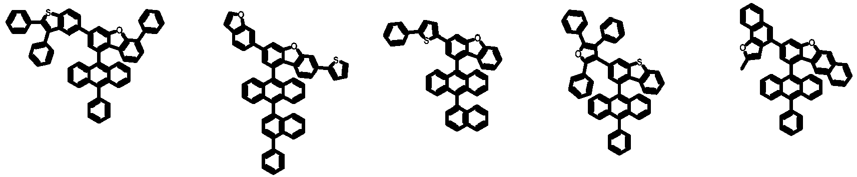

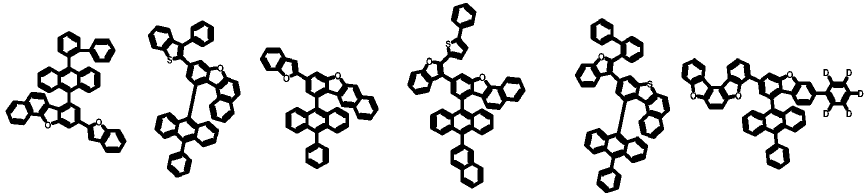

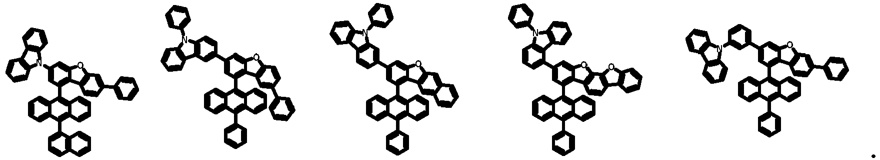

6. An organic light emitting device comprising:
a first electrode;
a second electrode; and
an organic material layer having one or more layers provided between the first electrode and the second electrode,
wherein one or more layers of the organic material layer comprise the compound according to any one of claims 1 to 5.

7. The organic light emitting device of claim 6, wherein the organic material layer comprises a light emitting layer comprising a host and a dopant, and the host comprises one or more of the compounds.

8. The organic light emitting device of claim 7, wherein the host comprises a first anthracene derivative substituted with deuterium and a second anthracene derivative unsubstituted with deuterium, and any one of the first anthracene derivative and the second anthracene derivative comprises the compound.

9. The organic light emitting device of claim 8, wherein the first anthracene derivative substituted with deuterium comprises the compound.

10. The organic light emitting device of claim 7, wherein the dopant is a fluorescent dopant.

11. The organic light emitting device of claim 7, wherein the dopant comprises a pyrene-based compound or a non-pyrene-based compound.

12. The organic light emitting device of claim 11, wherein the non-pyrene-based compound comprises a boron-based compound.

13. The organic light emitting device of claim 7, wherein the light emitting layer comprising the compound has a maximum emission peak (λₘₐₓ) of 400 nm to 470 nm.
